# EUROPEAN PATENT APPLICATION

(11) **EP 1 719 779 A2**
(43) Date of publication of application: **08.11.2006**
(21) Application number: 06012796.6
(22) Date of filing: 28.03.2001
(51) Int. Cl.: C07K 16/18, A61P 9/00, A61P 35/00, A61K 39/395, G01N 33/577

(54) **Antibodies against Matin**

(30) Priority: 29.03.2000 US 192875 P
(62) Divisional of application: 01918982.8
(71) Applicant: Beth Israel Deaconess Medical Center, Inc., Boston, MA 02215 (US)
(72) Inventor: Kalluri, Raghuram, Boston, MA 02216 (US)
(74) Representative: White, Martin Paul

(57) **Abstract**

Antibodies to a protein with anti-angiogenic properties are disclosed and can be used for various therapeutic, diagnostic and prognostic applications.

### Annex to the application documents - subsequently filed sequences listing

SEQUENCE LISTING

<110> Beth Israel Deaconess Medical Center, Inc.

<120> Anti-Angiogenic and Anti-Tumor Properties of Matin
      and Other Laminin Domains

<130> 1440.2004002

<140> PCT/US01/09921
<141> 2001-03-28

<150> US 60/192,875
<151> 2000-03-29

<160> 12

<170> FastSEQ for Windows Version 4.0

<210> 1
<211> 9503
<212> DNA
<213> Mus musculus

<220>
<221> CDS
<222> (49)...(9300)

<400> 1
agcacgggcg agaccttccc caggagcgca gggagcggcg gcgacaac atg cgc ggc   57
                                                     Met Arg Gly
                                                      1

agc ggc acg gga gcc gcg ctc ctg gtg ctc ctg gcc tcg gtg ctc tgg   105
Ser Gly Thr Gly Ala Ala Leu Leu Val Leu Leu Ala Ser Val Leu Trp
     5                  10                  15

gtc acc gtg cgg agc cag cag aga ggc ttg ttc cct gcc att ctc aac   153
Val Thr Val Arg Ser Gln Gln Arg Gly Leu Phe Pro Ala Ile Leu Asn
 20                  25                  30                  35

ctg gcc acc aat gcc cac atc agc gcc aat gct acc tgt gga gag aag   201
Leu Ala Thr Asn Ala His Ile Ser Ala Asn Ala Thr Cys Gly Glu Lys
                 40                  45                  50

ggg cct gag atg ttc tgc aaa ctc gtg gag cac gtg ccg ggc cgg cct   249
Gly Pro Glu Met Phe Cys Lys Leu Val Glu His Val Pro Gly Arg Pro
             55                  60                  65

gtt cga cac gcc caa tgc cgg gtc tgt gac ggt aac agt acg aat cct   297
Val Arg His Ala Gln Cys Arg Val Cys Asp Gly Asn Ser Thr Asn Pro
         70                  75                  80

aga gag cgc cat ccg ata tca cac gca atc gat ggc acc aac aac tgg 345
Arg Glu Arg His Pro Ile Ser His Ala Ile Asp Gly Thr Asn Asn Trp
    85                  90                  95

tgg cag agc ccc agt att cag aat ggg aga gag tat cac tgg gtc act 393
Trp Gln Ser Pro Ser Ile Gln Asn Gly Arg Glu Tyr His Trp Val Thr
100                 105                 110                 115

gtc acc ctg gac tta cgg cag gtc ttt caa gtt gca tac atc atc att 441
Val Thr Leu Asp Leu Arg Gln Val Phe Gln Val Ala Tyr Ile Ile Ile
                120                 125                 130

aaa gct gcc aat gcc cct cgg cct gga aac tgg att ttg gag cgc tcc 489
Lys Ala Ala Asn Ala Pro Arg Pro Gly Asn Trp Ile Leu Glu Arg Ser
            135                 140                 145

gtg gat ggc gtc aag ttc aaa ccc tgg cag tac tat gcc gtc agc gat 537
Val Asp Gly Val Lys Phe Lys Pro Trp Gln Tyr Tyr Ala Val Ser Asp
        150                 155                 160

aca gag tgt ttg acc cgc tac aaa ata act cca cgg cgg gga cct ccc 585
Thr Glu Cys Leu Thr Arg Tyr Lys Ile Thr Pro Arg Arg Gly Pro Pro
    165                 170                 175

act tac aga gca gac aac gaa gtc atc tgc acc tcg tat tat tca aag 633
Thr Tyr Arg Ala Asp Asn Glu Val Ile Cys Thr Ser Tyr Tyr Ser Lys
180                 185                 190                 195

ctg gtg cca ctt gaa cat gga gag att cac aca tca ctc atc aat ggc 681
Leu Val Pro Leu Glu His Gly Glu Ile His Thr Ser Leu Ile Asn Gly
                200                 205                 210

aga ccc agc gct gac gac ccc tca ccc cag ttg ctg gaa ttc acc tca 729
Arg Pro Ser Ala Asp Asp Pro Ser Pro Gln Leu Leu Glu Phe Thr Ser
            215                 220                 225

gca cgg tac att cgc ctt cgt ctt cag cgc atc aga aca ctc aac gca 777
Ala Arg Tyr Ile Arg Leu Arg Leu Gln Arg Ile Arg Thr Leu Asn Ala
        230                 235                 240

gac ctc atg acc ctt agc cat cgg gac ctc aga gac ctt gac ccc att 825
Asp Leu Met Thr Leu Ser His Arg Asp Leu Arg Asp Leu Asp Pro Ile
    245                 250                 255

gtc aca aga cgt tat tac tat tcg ata aaa gac att tcc gtt gga ggc 873
Val Thr Arg Arg Tyr Tyr Tyr Ser Ile Lys Asp Ile Ser Val Gly Gly
260                 265                 270                 275

atg tgc att tgc tac ggc cat gcc agc agc tgc ccg tgg gat gaa gaa 921
Met Cys Ile Cys Tyr Gly His Ala Ser Ser Cys Pro Trp Asp Glu Glu
                280                 285                 290

gca aag caa cta cag tgt cag tgt gaa cac aat acg tgt ggc gag agc 969

Ala Lys Gln Leu Gln Cys Gln Cys Glu His Asn Thr Cys Gly Glu Ser
            295                 300                 305

tgc gac agg tgc tgt cct ggc tac cat cag cag ccc tgg agg ccc gga      1017
Cys Asp Arg Cys Cys Pro Gly Tyr His Gln Gln Pro Trp Arg Pro Gly
        310                 315                 320

acc att tcc tcc ggc aac gag tgt gag gaa tgc aac tgt cac aac aaa      1065
Thr Ile Ser Ser Gly Asn Glu Cys Glu Glu Cys Asn Cys His Asn Lys
    325                 330                 335

gcc aaa gat tgt tac tat gac agc agt gtt gca aag gag agg aga agc      1113
Ala Lys Asp Cys Tyr Tyr Asp Ser Ser Val Ala Lys Glu Arg Arg Ser
340                 345                 350                 355

ctg aac act gcc ggg cag tac agt gga gga ggg gtt tgt gtc aac tgc      1161
Leu Asn Thr Ala Gly Gln Tyr Ser Gly Gly Gly Val Cys Val Asn Cys
                360                 365                 370

tcg cag aat acc aca ggg atc aac tgt gaa acc tgt atc gac cag tat      1209
Ser Gln Asn Thr Thr Gly Ile Asn Cys Glu Thr Cys Ile Asp Gln Tyr
            375                 380                 385

tac aga cct cac aag gta tct cct tat gat gac cac cct tgc cgt ccc      1257
Tyr Arg Pro His Lys Val Ser Pro Tyr Asp Asp His Pro Cys Arg Pro
        390                 395                 400

tgt aac tgt gac cct gtg ggg tct ctg agt tct gtc tgt atc aag gat      1305
Cys Asn Cys Asp Pro Val Gly Ser Leu Ser Ser Val Cys Ile Lys Asp
    405                 410                 415

gac cgc cat gcc gat tta gcc aat gga aag tgg cca ggt cag tgt cca      1353
Asp Arg His Ala Asp Leu Ala Asn Gly Lys Trp Pro Gly Gln Cys Pro
420                 425                 430                 435

tgt agg aaa ggt tat gct gga gat aaa tgt gac cgc tgc cag ttt ggc      1401
Cys Arg Lys Gly Tyr Ala Gly Asp Lys Cys Asp Arg Cys Gln Phe Gly
                440                 445                 450

tac cgg ggt ttc cca aat tgc atc ccc tgt gac tgc agg act gtc ggc      1449
Tyr Arg Gly Phe Pro Asn Cys Ile Pro Cys Asp Cys Arg Thr Val Gly
            455                 460                 465

agc ctg aat gag gat cca tgc ata gag ccg tgt ctt tgt aag aaa aat      1497
Ser Leu Asn Glu Asp Pro Cys Ile Glu Pro Cys Leu Cys Lys Lys Asn
        470                 475                 480

gtt gag ggt aag aac tgt gat cgc tgc aag cca gga ttc tac aac ttg      1545
Val Glu Gly Lys Asn Cys Asp Arg Cys Lys Pro Gly Phe Tyr Asn Leu
    485                 490                 495

aag gaa cga aac ccc gag ggc tgc tcc gag tgc ttc tgc ttc ggt gtc      1593
Lys Glu Arg Asn Pro Glu Gly Cys Ser Glu Cys Phe Cys Phe Gly Val
500                 505                 510                 515

tct ggt gtc tgt gac agc ctc acg tgg tcc att agt cag gtg acc aat 1641
Ser Gly Val Cys Asp Ser Leu Thr Trp Ser Ile Ser Gln Val Thr Asn
                520                 525                 530

atg tca ggg tgg ctg gtc act gac ttg atg agc aca aat aag atc cgg 1689
Met Ser Gly Trp Leu Val Thr Asp Leu Met Ser Thr Asn Lys Ile Arg
            535                 540                 545

tcc cag caa gat gtc ctg ggt ggg cac cgt cag atc agc atc aac aac 1737
Ser Gln Gln Asp Val Leu Gly Gly His Arg Gln Ile Ser Ile Asn Asn
        550                 555                 560

acg gcg gtc atg cag agg ctg act tcc act tac tac tgg gca gct cct 1785
Thr Ala Val Met Gln Arg Leu Thr Ser Thr Tyr Tyr Trp Ala Ala Pro
    565                 570                 575

gag gcc tac ctc gga aac aag ctg aca gca ttt ggc ggt ttc ctg aaa 1833
Glu Ala Tyr Leu Gly Asn Lys Leu Thr Ala Phe Gly Gly Phe Leu Lys
580                 585                 590                 595

tac act gtg tct tac gac att cca gtg gag acg gtg gac agt gac ctc 1881
Tyr Thr Val Ser Tyr Asp Ile Pro Val Glu Thr Val Asp Ser Asp Leu
                600                 605                 610

atg tct cat gct gac atc atc atc aag ggg aat ggg ctc acc ata agc 1929
Met Ser His Ala Asp Ile Ile Ile Lys Gly Asn Gly Leu Thr Ile Ser
            615                 620                 625

aca aga gct gag ggc ctg tcc ttg caa ccc tat gag gaa tac ttc aac 1977
Thr Arg Ala Glu Gly Leu Ser Leu Gln Pro Tyr Glu Glu Tyr Phe Asn
        630                 635                 640

gtg gtt aga ctt gtg cct gag aac ttc cgg gac ttt aat acc aga agg 2025
Val Val Arg Leu Val Pro Glu Asn Phe Arg Asp Phe Asn Thr Arg Arg
    645                 650                 655

gag att gac cgt gac cag ctg atg act gtc ctg gcc aat gtg aca cat 2073
Glu Ile Asp Arg Asp Gln Leu Met Thr Val Leu Ala Asn Val Thr His
660                 665                 670                 675

ctc ttg atc aga gcc aat tat aat tct gct aaa atg gcg ctc tat agg 2121
Leu Leu Ile Arg Ala Asn Tyr Asn Ser Ala Lys Met Ala Leu Tyr Arg
                680                 685                 690

ctg gat tct gtc tct ctg gac ata gca agc cct aat gct ata gac ttg 2169
Leu Asp Ser Val Ser Leu Asp Ile Ala Ser Pro Asn Ala Ile Asp Leu
            695                 700                 705

gca gtg gct gct gat gtg gaa cac tgt gaa tgt ccc caa ggc tac acg 2217
Ala Val Ala Ala Asp Val Glu His Cys Glu Cys Pro Gln Gly Tyr Thr
        710                 715                 720

ggg acc tcc tgt gag gcc tgc ctc cct ggc tat tat cga gtg gac ggg 2265

Gly Thr Ser Cys Glu Ala Cys Leu Pro Gly Tyr Tyr Arg Val Asp Gly
    725                 730                 735

ata ctc ttt gga gga atc tgt cag ccc tgc gag tgc cac ggg cat gca     2313
Ile Leu Phe Gly Gly Ile Cys Gln Pro Cys Glu Cys His Gly His Ala
740                 745                 750                 755

tcc gag tgt gac att cat gga att tgc tct gtg tgt aca cac aac acc     2361
Ser Glu Cys Asp Ile His Gly Ile Cys Ser Val Cys Thr His Asn Thr
                760                 765                 770

acg ggg gat cac tgt gag cag tgc ctg cct ggc ttc tat ggg aca cct     2409
Thr Gly Asp His Cys Glu Gln Cys Leu Pro Gly Phe Tyr Gly Thr Pro
            775                 780                 785

tca cgt ggg acc cca gga gac tgc cag cct tgt gcc tgc cct ctc tcc     2457
Ser Arg Gly Thr Pro Gly Asp Cys Gln Pro Cys Ala Cys Pro Leu Ser
        790                 795                 800

att gac tct aac aat ttc agc cct acc tgc cac ctc act gat gga gag     2505
Ile Asp Ser Asn Asn Phe Ser Pro Thr Cys His Leu Thr Asp Gly Glu
    805                 810                 815

gaa gtg gtt tgt gac caa tgt gcc ccg ggt tac tca gga tcc tgg tgt     2553
Glu Val Val Cys Asp Gln Cys Ala Pro Gly Tyr Ser Gly Ser Trp Cys
820                 825                 830                 835

gag aga tgt gca gat ggt tac tat gga aac cca acc gtg cca ggg gga     2601
Glu Arg Cys Ala Asp Gly Tyr Tyr Gly Asn Pro Thr Val Pro Gly Gly
                840                 845                 850

acc tgt gta cca tgc aac tgc agt ggc aat gtt gat ccc ttg gag gct     2649
Thr Cys Val Pro Cys Asn Cys Ser Gly Asn Val Asp Pro Leu Glu Ala
            855                 860                 865

ggc cac tgt gac tct gtc acg ggg gaa tgc ctg aag tgc tta tgg aac     2697
Gly His Cys Asp Ser Val Thr Gly Glu Cys Leu Lys Cys Leu Trp Asn
        870                 875                 880

aca gac ggt gcc cat tgt gag agg tgt gca gat ggc ttc tat gga gat     2745
Thr Asp Gly Ala His Cys Glu Arg Cys Ala Asp Gly Phe Tyr Gly Asp
    885                 890                 895

gcc gtg act gcc aaa aac tgc cga gcc tgt gac tgc cac gag aat ggc     2793
Ala Val Thr Ala Lys Asn Cys Arg Ala Cys Asp Cys His Glu Asn Gly
900                 905                 910                 915

tcc ctt tct ggc gtc tgc cat ctg gag act gga ctg tgt gac tgc aaa     2841
Ser Leu Ser Gly Val Cys His Leu Glu Thr Gly Leu Cys Asp Cys Lys
                920                 925                 930

cct cac gtg aca gga cag cag tgt gac cag tgc ctg tct ggc tac tac     2889
Pro His Val Thr Gly Gln Gln Cys Asp Gln Cys Leu Ser Gly Tyr Tyr
            935                 940                 945

ggg ttg gac acg ggg ctt ggc tgt gtg ccc tgt aac tgc agt gtg gaa 2937
Gly Leu Asp Thr Gly Leu Gly Cys Val Pro Cys Asn Cys Ser Val Glu
        950                 955                 960

ggc tct gta tct gac aac tgc acg gag gaa gga cag tgt cac tgt gga 2985
Gly Ser Val Ser Asp Asn Cys Thr Glu Glu Gly Gln Cys His Cys Gly
    965                 970                 975

cca ggt gtc tct ggg aaa cag tgt gac agg tgt tca cat ggt ttc tat 3033
Pro Gly Val Ser Gly Lys Gln Cys Asp Arg Cys Ser His Gly Phe Tyr
980                 985                 990                 995

gca ttc cag gat ggc ggc tgc aca ccc tgt gac tgt gct cat acc cag 3081
Ala Phe Gln Asp Gly Gly Cys Thr Pro Cys Asp Cys Ala His Thr Gln
                1000                1005                1010

aat aac tgt gac ccc gcc tct gga gag tgt ctc tgc ccg cct cac acg 3129
Asn Asn Cys Asp Pro Ala Ser Gly Glu Cys Leu Cys Pro Pro His Thr
            1015                1020                1025

cag ggg ctg aag tgt gag gag tgt gaa gag gca tac tgg ggt ctg gac 3177
Gln Gly Leu Lys Cys Glu Glu Cys Glu Glu Ala Tyr Trp Gly Leu Asp
        1030                1035                1040

ccg gag cag ggg tgc cag gct tgc aat tgc agt gct gtg ggc tcc acg 3225
Pro Glu Gln Gly Cys Gln Ala Cys Asn Cys Ser Ala Val Gly Ser Thr
    1045                1050                1055

agt gcc cag tgt gat gtt ctc tct ggc cac tgc ccc tgc aaa aaa ggg 3273
Ser Ala Gln Cys Asp Val Leu Ser Gly His Cys Pro Cys Lys Lys Gly
1060                1065                1070                1075

ttt ggt ggg cag agc tgc cat cag tgt tcc tta ggc tac aga agt ttt 3321
Phe Gly Gly Gln Ser Cys His Gln Cys Ser Leu Gly Tyr Arg Ser Phe
                1080                1085                1090

cct gac tgt gtc ccc tgt ggc tgt gac ctg agg ggg aca ctg cct gac 3369
Pro Asp Cys Val Pro Cys Gly Cys Asp Leu Arg Gly Thr Leu Pro Asp
            1095                1100                1105

acc tgt gac ctg gaa cag ggt ctc tgc agc tgc tca gag gac agt ggt 3417
Thr Cys Asp Leu Glu Gln Gly Leu Cys Ser Cys Ser Glu Asp Ser Gly
        1110                1115                1120

acc tgc tcc tgc aag gag aat gtc gtg ggc ccc cag tgc agt aag tgc 3465
Thr Cys Ser Cys Lys Glu Asn Val Val Gly Pro Gln Cys Ser Lys Cys
    1125                1130                1135

caa gcc ggc acc ttt gcc ttg cga ggg gac aac cct caa ggc tgc agc 3513
Gln Ala Gly Thr Phe Ala Leu Arg Gly Asp Asn Pro Gln Gly Cys Ser
1140                1145                1150                1155

ccc tgc ttc tgc ttc ggt ctg tcg cag ctc tgc tca gag ttg gag ggt 3561

Pro Cys Phe Cys Phe Gly Leu Ser Gln Leu Cys Ser Glu Leu Glu Gly
                1160                1165                1170

tac gtg agg act ctg ata act cta gcc tcc gat cag ccc ctc ctg cat 3609
Tyr Val Arg Thr Leu Ile Thr Leu Ala Ser Asp Gln Pro Leu Leu His
            1175                1180                1185

gtg gtt tca cag agc aac ctc aag ggc aca atc gaa ggc gtg cat ttc 3657
Val Val Ser Gln Ser Asn Leu Lys Gly Thr Ile Glu Gly Val His Phe
        1190                1195                1200

cag cct cct gac acc ttg ctg gac gca gag gct gtc cgc cag cat atc 3705
Gln Pro Pro Asp Thr Leu Leu Asp Ala Glu Ala Val Arg Gln His Ile
    1205                1210                1215

tat gca gag cca ttt tac tgg cgg cta cca aag cag ttc cag gga gac 3753
Tyr Ala Glu Pro Phe Tyr Trp Arg Leu Pro Lys Gln Phe Gln Gly Asp
1220                1225                1230                1235

cag ctc ttg gcc tat ggt ggg aaa ctc cag tac agt gtg gct ttc tac 3801
Gln Leu Leu Ala Tyr Gly Gly Lys Leu Gln Tyr Ser Val Ala Phe Tyr
                1240                1245                1250

tct aca ctt ggc acc gga aca tcc aat tat gag cct caa gtc ctc atc 3849
Ser Thr Leu Gly Thr Gly Thr Ser Asn Tyr Glu Pro Gln Val Leu Ile
            1255                1260                1265

aaa gga ggt cgg gcc agg aag cac gtc att tat atg gat gcc cca gcg 3897
Lys Gly Gly Arg Ala Arg Lys His Val Ile Tyr Met Asp Ala Pro Ala
        1270                1275                1280

cct gag aat gga gtg aga cag gat tac gaa gtg cag atg aaa gag gaa 3945
Pro Glu Asn Gly Val Arg Gln Asp Tyr Glu Val Gln Met Lys Glu Glu
    1285                1290                1295

ttc tgg aaa tat ttt aac tcc gtg tct gag aaa cac gtc aca cac tct 3993
Phe Trp Lys Tyr Phe Asn Ser Val Ser Glu Lys His Val Thr His Ser
1300                1305                1310                1315

gat ttt atg tct gtt ctc agc aat att gac tac atc ctc atc aaa gca 4041
Asp Phe Met Ser Val Leu Ser Asn Ile Asp Tyr Ile Leu Ile Lys Ala
                1320                1325                1330

tca tac ggc cag gga ctg cag cag agc aga att gcc aac att tcc atg 4089
Ser Tyr Gly Gln Gly Leu Gln Gln Ser Arg Ile Ala Asn Ile Ser Met
            1335                1340                1345

gag gtt ggc cgg aaa gct gtc gag ctg ccc gct gag ggc gag gcg gca 4137
Glu Val Gly Arg Lys Ala Val Glu Leu Pro Ala Glu Gly Glu Ala Ala
        1350                1355                1360

ttg ctg ttg gag ctc tgt gtc tgt cct cct ggc acc gca gga cac tcc 4185
Leu Leu Leu Glu Leu Cys Val Cys Pro Pro Gly Thr Ala Gly His Ser
    1365                1370                1375

tgt cag gac tgt gct cct ggg tac tac aga gaa aag ctc cca gaa agt    4233
Cys Gln Asp Cys Ala Pro Gly Tyr Tyr Arg Glu Lys Leu Pro Glu Ser
1380                1385                1390                1395

ggt ggc agg gga ccc cgc cct ctg ctg gct cct tgt gtg ccc tgc aat    4281
Gly Gly Arg Gly Pro Arg Pro Leu Leu Ala Pro Cys Val Pro Cys Asn
                1400                1405                1410

tgc aac aac cac agt gat gtc tgt gac ccc gaa act gga aag tgc ctg    4329
Cys Asn Asn His Ser Asp Val Cys Asp Pro Glu Thr Gly Lys Cys Leu
            1415                1420                1425

agc tgc agg gac cac aca tcc ggg gac cac tgt gag ctg tgt gct tct    4377
Ser Cys Arg Asp His Thr Ser Gly Asp His Cys Glu Leu Cys Ala Ser
        1430                1435                1440

ggc tac tat ggg aag gtg act gga ctg cct gga gac tgt acc ccg tgc    4425
Gly Tyr Tyr Gly Lys Val Thr Gly Leu Pro Gly Asp Cys Thr Pro Cys
    1445                1450                1455

acc tgt cct cat cac cct cct ttc agt ttc agc ccc act tgt gtc gtg    4473
Thr Cys Pro His His Pro Pro Phe Ser Phe Ser Pro Thr Cys Val Val
1460                1465                1470                1475

gaa ggt gac agt gat ttc cgg tgc aat gcc tgc ctc ccc ggc tat gaa    4521
Glu Gly Asp Ser Asp Phe Arg Cys Asn Ala Cys Leu Pro Gly Tyr Glu
                1480                1485                1490

gga cag tac tgt gaa agg tgc tct gca ggc tat cac ggc aac cct cga    4569
Gly Gln Tyr Cys Glu Arg Cys Ser Ala Gly Tyr His Gly Asn Pro Arg
            1495                1500                1505

gca gca ggt ggt agc tgc caa acg tgt gat tgc aac ccc caa ggc tct    4617
Ala Ala Gly Gly Ser Cys Gln Thr Cys Asp Cys Asn Pro Gln Gly Ser
        1510                1515                1520

gtc cac agt gac tgt gac cgt gca tcc ggg cag tgt gtc tgc aag cca    4665
Val His Ser Asp Cys Asp Arg Ala Ser Gly Gln Cys Val Cys Lys Pro
    1525                1530                1535

gga gct aca ggg ctc cac tgt gag aaa tgc ctg ccg aga cac atc ctg    4713
Gly Ala Thr Gly Leu His Cys Glu Lys Cys Leu Pro Arg His Ile Leu
1540                1545                1550                1555

atg gag agc gac tgt gtt tcc tgt gat gat gac tgt gtg ggt cct ttg    4761
Met Glu Ser Asp Cys Val Ser Cys Asp Asp Asp Cys Val Gly Pro Leu
                1560                1565                1570

ctg aac gac ctg gat tct gtt ggt gat gcc gtt ctg tct ctg aac ctc    4809
Leu Asn Asp Leu Asp Ser Val Gly Asp Ala Val Leu Ser Leu Asn Leu
            1575                1580                1585

acg ggc gtt tcc cct gct ccc tat gga atc ctg gaa aat ctg gaa aat    4857

Thr Gly Val Ser Pro Ala Pro Tyr Gly Ile Leu Glu Asn Leu Glu Asn
      1590                1595                1600

aca act aaa tat ttc cag agg tat tta ata aag gaa aat gcc aag aag      4905
Thr Thr Lys Tyr Phe Gln Arg Tyr Leu Ile Lys Glu Asn Ala Lys Lys
    1605                1610                1615

att cga gca gag atc cag ctc gaa ggg att gca gag caa aca gaa aat      4953
Ile Arg Ala Glu Ile Gln Leu Glu Gly Ile Ala Glu Gln Thr Glu Asn
1620                1625                1630                1635

ctg caa aag gag ctc acc aga gtg tta gca cgc cat cag aag gtg aac      5001
Leu Gln Lys Glu Leu Thr Arg Val Leu Ala Arg His Gln Lys Val Asn
                1640                1645                1650

gct gaa atg gaa aga act tcc aat ggg act caa gcc ctg gcc acg ttc      5049
Ala Glu Met Glu Arg Thr Ser Asn Gly Thr Gln Ala Leu Ala Thr Phe
            1655                1660                1665

att gag cag cta cat gca aac atc aaa gaa atc aca gaa aag gtg gca      5097
Ile Glu Gln Leu His Ala Asn Ile Lys Glu Ile Thr Glu Lys Val Ala
        1670                1675                1680

acg ttg aac cag acg gcg cgt aaa gat ttc cag cca ccc gtg tct gcc      5145
Thr Leu Asn Gln Thr Ala Arg Lys Asp Phe Gln Pro Pro Val Ser Ala
    1685                1690                1695

ctt cag agc atg cac cag aac att tcg tct ctg ctg gga ctc atc aag      5193
Leu Gln Ser Met His Gln Asn Ile Ser Ser Leu Leu Gly Leu Ile Lys
1700                1705                1710                1715

gaa agg aat ttc aca gag atg cag cag aat gct acc ctt gag ctc aag      5241
Glu Arg Asn Phe Thr Glu Met Gln Gln Asn Ala Thr Leu Glu Leu Lys
                1720                1725                1730

gct gct aaa gac tta ttg tca cgg att cag aaa agg ttt cag aag cct      5289
Ala Ala Lys Asp Leu Leu Ser Arg Ile Gln Lys Arg Phe Gln Lys Pro
            1735                1740                1745

cag gaa aag ttg aag gca ttg aag gag gcc aac agc ctc ctt tcc aac      5337
Gln Glu Lys Leu Lys Ala Leu Lys Glu Ala Asn Ser Leu Leu Ser Asn
        1750                1755                1760

cac agt gaa aaa ctg cag gct gct gag gag ctc ctt aag gaa gct gga      5385
His Ser Glu Lys Leu Gln Ala Ala Glu Glu Leu Leu Lys Glu Ala Gly
    1765                1770                1775

agc aag acc cag gag agc aac ctc ctg ctg ctc ctt gtc aag gcc aac      5433
Ser Lys Thr Gln Glu Ser Asn Leu Leu Leu Leu Leu Val Lys Ala Asn
1780                1785                1790                1795

ctg aaa gag gaa ttc cag gag aaa aag ctg cgt gtt caa gaa gaa caa      5481
Leu Lys Glu Glu Phe Gln Glu Lys Lys Leu Arg Val Gln Glu Glu Gln
                1800                1805                1810

aat gtg acc tca gag ctc att gcc aag ggt aga gaa tgg gtg gat gct 5529
Asn Val Thr Ser Glu Leu Ile Ala Lys Gly Arg Glu Trp Val Asp Ala
            1815                1820                1825

gcc ggg act cac aca gct gct gca caa gac acc cta aca cag ctg gag 5577
Ala Gly Thr His Thr Ala Ala Ala Gln Asp Thr Leu Thr Gln Leu Glu
        1830                1835                1840

cat cac cga gat gaa ctc ctt ctg tgg gcc aga aaa atc agg agc cac 5625
His His Arg Asp Glu Leu Leu Leu Trp Ala Arg Lys Ile Arg Ser His
    1845                1850                1855

gta gat gac ctc gtc atg cag atg tcc aaa cga aga gcc cgt gac ctg 5673
Val Asp Asp Leu Val Met Gln Met Ser Lys Arg Arg Ala Arg Asp Leu
1860                1865                1870                1875

gtc cac agg gca gag cag cat gcc tct gag ctg cag agc agg gca gga 5721
Val His Arg Ala Glu Gln His Ala Ser Glu Leu Gln Ser Arg Ala Gly
                1880                1885                1890

gct ttg gac aga gac ctt gaa aat gtt aga aac gtg tct ttg aat gcc 5769
Ala Leu Asp Arg Asp Leu Glu Asn Val Arg Asn Val Ser Leu Asn Ala
            1895                1900                1905

acc agt gcg gca cac gtc cac agc aac atc cag aca ctg aca gag gaa 5817
Thr Ser Ala Ala His Val His Ser Asn Ile Gln Thr Leu Thr Glu Glu
        1910                1915                1920

gct gag atg ctg gct gct gat gct cac aag acg gcg aat aag aca gac 5865
Ala Glu Met Leu Ala Ala Asp Ala His Lys Thr Ala Asn Lys Thr Asp
    1925                1930                1935

ttg atc tcc gaa tcc ctg gct tct cgg ggg aaa gca gtc ctt cag cgc 5913
Leu Ile Ser Glu Ser Leu Ala Ser Arg Gly Lys Ala Val Leu Gln Arg
1940                1945                1950                1955

tcg tcc cgg ttt cta aag gaa agt gtc ggt acc agg agg aag cag caa 5961
Ser Ser Arg Phe Leu Lys Glu Ser Val Gly Thr Arg Arg Lys Gln Gln
                1960                1965                1970

ggc att acg atg aag ctg gat gag ttg aaa aac tta acg agt caa ttt 6009
Gly Ile Thr Met Lys Leu Asp Glu Leu Lys Asn Leu Thr Ser Gln Phe
            1975                1980                1985

cag gag agc gtg gat aac att acg aag cag gcc aac gac tcc ctt gcg 6057
Gln Glu Ser Val Asp Asn Ile Thr Lys Gln Ala Asn Asp Ser Leu Ala
        1990                1995                2000

atg ctt aga gaa agc cct gga ggt atg aga gag aag ggc agg aaa gcc 6105
Met Leu Arg Glu Ser Pro Gly Gly Met Arg Glu Lys Gly Arg Lys Ala
    2005                2010                2015

aga gag ctg gcg gca gca gcc aac gag agt gcg gtg aag aca ctg gag 6153

Arg Glu Leu Ala Ala Ala Ala Asn Glu Ser Ala Val Lys Thr Leu Glu
2020                2025                2030                2035

gat gtg ctg gct ttg agc ctg agg gtc ttc aat aca tca gag gac ctg     6201
Asp Val Leu Ala Leu Ser Leu Arg Val Phe Asn Thr Ser Glu Asp Leu
                2040                2045                2050

tcc aga gtg aat gcc aca gtc cag gag aca aac gac ctt ctg cat aac     6249
Ser Arg Val Asn Ala Thr Val Gln Glu Thr Asn Asp Leu Leu His Asn
            2055                2060                2065

tcc acg atg acc act ctg ttg gct gga aga aaa atg aaa gac atg gaa     6297
Ser Thr Met Thr Thr Leu Leu Ala Gly Arg Lys Met Lys Asp Met Glu
        2070                2075                2080

atg caa gcc aac ctt tta ttg gat cga ttg aaa cct ttg aaa acc ctg     6345
Met Gln Ala Asn Leu Leu Leu Asp Arg Leu Lys Pro Leu Lys Thr Leu
    2085                2090                2095

gag gag aac ctg agc aga aac ctg tcg gag atc aag ctg ctg atc agc     6393
Glu Glu Asn Leu Ser Arg Asn Leu Ser Glu Ile Lys Leu Leu Ile Ser
2100                2105                2110                2115

cgg gcc cgg aaa caa gcg gcg tcg atc aaa gtc gcc gtg tct gca gac     6441
Arg Ala Arg Lys Gln Ala Ala Ser Ile Lys Val Ala Val Ser Ala Asp
                2120                2125                2130

aga gac tgc atc cgc gcc tat cag cct cag act tca tct acc aat tac     6489
Arg Asp Cys Ile Arg Ala Tyr Gln Pro Gln Thr Ser Ser Thr Asn Tyr
            2135                2140                2145

aac acc ttg atc ctg aac gtg aag acg cag gag ccc gac aac ctc ctc     6537
Asn Thr Leu Ile Leu Asn Val Lys Thr Gln Glu Pro Asp Asn Leu Leu
        2150                2155                2160

ttc tac ctc ggc agc agc agc agt tct gac ttt ctc gca gtg gag atg     6585
Phe Tyr Leu Gly Ser Ser Ser Ser Ser Asp Phe Leu Ala Val Glu Met
    2165                2170                2175

cgg cgg ggg aag gtc gcc ttt ctc tgg gac ctg ggc tcc ggg tcc aca     6633
Arg Arg Gly Lys Val Ala Phe Leu Trp Asp Leu Gly Ser Gly Ser Thr
2180                2185                2190                2195

agg ttg gaa ttc cca gag gtc tcc atc aat aac aac aga tgg cac agc     6681
Arg Leu Glu Phe Pro Glu Val Ser Ile Asn Asn Asn Arg Trp His Ser
                2200                2205                2210

atc tac ata acc agg ttt gga aac atg ggg tcc ctg agt gta aag gaa     6729
Ile Tyr Ile Thr Arg Phe Gly Asn Met Gly Ser Leu Ser Val Lys Glu
            2215                2220                2225

gca agc gct gcc gag aac cca ccg gtc agg aca agc aaa tct cct gga     6777
Ala Ser Ala Ala Glu Asn Pro Pro Val Arg Thr Ser Lys Ser Pro Gly
        2230                2235                2240

ccg tcg aag gtt ctg gac ata aac aat tca acg ctg atg ttt gtt gga 6825
Pro Ser Lys Val Leu Asp Ile Asn Asn Ser Thr Leu Met Phe Val Gly
    2245                2250                2255

ggg ctc gga ggt cag atc aag aaa tcc ccg gct gtg aag gtt act cat 6873
Gly Leu Gly Gly Gln Ile Lys Lys Ser Pro Ala Val Lys Val Thr His
2260                2265                2270                2275

ttt aag ggc tgc atg gga gag gcc ttc ttg aat ggc aaa tcg att ggc 6921
Phe Lys Gly Cys Met Gly Glu Ala Phe Leu Asn Gly Lys Ser Ile Gly
                2280                2285                2290

ctg tgg aat tac atc gag aga gag ggg aag tgc aat ggc tgc ttt gga 6969
Leu Trp Asn Tyr Ile Glu Arg Glu Gly Lys Cys Asn Gly Cys Phe Gly
            2295                2300                2305

agc tcc cag aac gaa gat tcc tcc ttc cat ttc gat gga agc ggg tac 7017
Ser Ser Gln Asn Glu Asp Ser Ser Phe His Phe Asp Gly Ser Gly Tyr
        2310                2315                2320

gcc atg gtg gag aag acg ctc cgg ccc acc gtg acg cag ata gta att 7065
Ala Met Val Glu Lys Thr Leu Arg Pro Thr Val Thr Gln Ile Val Ile
    2325                2330                2335

ctc ttc agc acc ttc tcc ccg aat ggc ctt ctt ttc tac ctg gct tca 7113
Leu Phe Ser Thr Phe Ser Pro Asn Gly Leu Leu Phe Tyr Leu Ala Ser
2340                2345                2350                2355

aac ggc acc aag gac ttc cta tcc atc gag ctg gtc cgt ggc agg gtc 7161
Asn Gly Thr Lys Asp Phe Leu Ser Ile Glu Leu Val Arg Gly Arg Val
                2360                2365                2370

aaa gtg atg gtg gac cta ggg tca gga ccc ctc act ctt atg aca gac 7209
Lys Val Met Val Asp Leu Gly Ser Gly Pro Leu Thr Leu Met Thr Asp
            2375                2380                2385

agg cgg tat aac aac gga acc tgg tat aaa atc gcc ttc cag cgg aac 7257
Arg Arg Tyr Asn Asn Gly Thr Trp Tyr Lys Ile Ala Phe Gln Arg Asn
        2390                2395                2400

cgg aag caa gga ctg cta gct gtc ttc gat gca tat gac acc agt gac 7305
Arg Lys Gln Gly Leu Leu Ala Val Phe Asp Ala Tyr Asp Thr Ser Asp
    2405                2410                2415

aag gag aca aag caa gga gag act cca gga gcc gct tcc gac ctc aat 7353
Lys Glu Thr Lys Gln Gly Glu Thr Pro Gly Ala Ala Ser Asp Leu Asn
2420                2425                2430                2435

cgg ctg gag aaa gac ctg att tac gtg ggt gga ttg cct cat tct aag 7401
Arg Leu Glu Lys Asp Leu Ile Tyr Val Gly Gly Leu Pro His Ser Lys
                2440                2445                2450

gct gtg agg aaa ggg gtc agc agc aga agc tat gtg ggc tgt atc aag 7449

Ala Val Arg Lys Gly Val Ser Ser Arg Ser Tyr Val Gly Cys Ile Lys
            2455                2460                2465

aac cta gag ata tcc agg tcc acc ttt gat ttg ctg aga aat tcc tac     7497
Asn Leu Glu Ile Ser Arg Ser Thr Phe Asp Leu Leu Arg Asn Ser Tyr
        2470                2475                2480

gga gtg aga aaa ggc tgc gca ctg gag cct atc cag agt gtg agt ttc     7545
Gly Val Arg Lys Gly Cys Ala Leu Glu Pro Ile Gln Ser Val Ser Phe
    2485                2490                2495

ctg aga ggc ggc tat gtg gag atg cca ccc aag tct ctc tca cct gag     7593
Leu Arg Gly Gly Tyr Val Glu Met Pro Pro Lys Ser Leu Ser Pro Glu
2500                2505                2510                2515

tca tcc ctg ctg gcc aca ttc gcc acc aag aac agc agc gga atc ctc     7641
Ser Ser Leu Leu Ala Thr Phe Ala Thr Lys Asn Ser Ser Gly Ile Leu
                2520                2525                2530

ctg gtt gcc ctg ggc aag gat gcg gag gag gct ggt ggg gct cag gca     7689
Leu Val Ala Leu Gly Lys Asp Ala Glu Glu Ala Gly Gly Ala Gln Ala
            2535                2540                2545

cat gtg ccc ttc ttt tcc atc atg ctg ctt gag gga cga att gaa gtg     7737
His Val Pro Phe Phe Ser Ile Met Leu Leu Glu Gly Arg Ile Glu Val
        2550                2555                2560

cat gtc aac tct ggg gac ggg acc agt ctg agg aag gcc ctc ctg cat     7785
His Val Asn Ser Gly Asp Gly Thr Ser Leu Arg Lys Ala Leu Leu His
    2565                2570                2575

gcc ccc acc ggc tcc tac agt gat gga cag gaa cac tcc atc tcc ctg     7833
Ala Pro Thr Gly Ser Tyr Ser Asp Gly Gln Glu His Ser Ile Ser Leu
2580                2585                2590                2595

gtt agg aat cgg aga gtt atc acc ata caa gtg gat gag aac agt ccc     7881
Val Arg Asn Arg Arg Val Ile Thr Ile Gln Val Asp Glu Asn Ser Pro
                2600                2605                2610

gta gaa atg aag ttg ggt cca tta aca gaa gga aag acg atc gac ata     7929
Val Glu Met Lys Leu Gly Pro Leu Thr Glu Gly Lys Thr Ile Asp Ile
            2615                2620                2625

tcc aac ctg tac ata ggg gga ctt ccg gag gac aag gcg acc ccg atg     7977
Ser Asn Leu Tyr Ile Gly Gly Leu Pro Glu Asp Lys Ala Thr Pro Met
        2630                2635                2640

ctc aag atg cgg act tcg ttc cat ggg tgt att aaa aac gtg gtc ctt     8025
Leu Lys Met Arg Thr Ser Phe His Gly Cys Ile Lys Asn Val Val Leu
    2645                2650                2655

gac gct caa ctt ttg gac ttc acc cat gcg act ggc tct gag caa gta     8073
Asp Ala Gln Leu Leu Asp Phe Thr His Ala Thr Gly Ser Glu Gln Val
2660                2665                2670                2675

gag ctg gac aca tgc ttg ctg gca gaa gag ccc atg cag agt ctg cac 8121
Glu Leu Asp Thr Cys Leu Leu Ala Glu Glu Pro Met Gln Ser Leu His
                2680                2685                2690

aga gaa cac ggg gaa ctc cct ccg gag ccc cca act cta cca cag cct 8169
Arg Glu His Gly Glu Leu Pro Pro Glu Pro Pro Thr Leu Pro Gln Pro
            2695                2700                2705

gaa ctg tgc gca gta gac acg gct ccg ggg tat gtg gca ggt gct cac 8217
Glu Leu Cys Ala Val Asp Thr Ala Pro Gly Tyr Val Ala Gly Ala His
        2710                2715                2720

cag ttt ggc ctc tcg cag aac agc cac ttg gtg ctc cct ctg aat cag 8265
Gln Phe Gly Leu Ser Gln Asn Ser His Leu Val Leu Pro Leu Asn Gln
    2725                2730                2735

tct gat gtc cgg aag agg ctc cag gtg cag ctg agc att cgg aca ttt 8313
Ser Asp Val Arg Lys Arg Leu Gln Val Gln Leu Ser Ile Arg Thr Phe
2740                2745                2750                2755

gcc tcc agt ggc ctc att tac tat gtg gct cat cag aac caa atg gac 8361
Ala Ser Ser Gly Leu Ile Tyr Tyr Val Ala His Gln Asn Gln Met Asp
                2760                2765                2770

tac gcc acg ctc cag ctc caa gag ggc cgc ctg cac ttc atg ttt gat 8409
Tyr Ala Thr Leu Gln Leu Gln Glu Gly Arg Leu His Phe Met Phe Asp
            2775                2780                2785

ctc ggc aag ggc cgg acc aag gtc tcc cac cct gcc ctg ctc agt gat 8457
Leu Gly Lys Gly Arg Thr Lys Val Ser His Pro Ala Leu Leu Ser Asp
        2790                2795                2800

ggc aag tgg cac aca gtc aag aca gaa tac att aaa agg aag gcg ttc 8505
Gly Lys Trp His Thr Val Lys Thr Glu Tyr Ile Lys Arg Lys Ala Phe
    2805                2810                2815

atg act gtt gac ggc caa gag tcc ccc agt gtg act gtg gtg ggc aat 8553
Met Thr Val Asp Gly Gln Glu Ser Pro Ser Val Thr Val Val Gly Asn
2820                2825                2830                2835

gca acc acg ctg gat gtg gaa agg aaa ctg tac ctc gga ggc ctt ccc 8601
Ala Thr Thr Leu Asp Val Glu Arg Lys Leu Tyr Leu Gly Gly Leu Pro
                2840                2845                2850

agc cac tac agg gcc agg aac atc ggg act atc acc cac agc atc cct 8649
Ser His Tyr Arg Ala Arg Asn Ile Gly Thr Ile Thr His Ser Ile Pro
            2855                2860                2865

gcc tgc att ggg gaa atc atg gtt aat ggc caa cag ctg gat aaa gac 8697
Ala Cys Ile Gly Glu Ile Met Val Asn Gly Gln Gln Leu Asp Lys Asp
        2870                2875                2880

agg ccc ttg tct gcc tct gct gtg gac agg tgc tat gtc gtg gct cag 8745

Arg Pro Leu Ser Ala Ser Ala Val Asp Arg Cys Tyr Val Val Ala Gln
    2885                2890                2895

gaa gga act ttc ttt gaa gga agc gga tat gca gct ctt gtc aag gaa 8793
Glu Gly Thr Phe Phe Glu Gly Ser Gly Tyr Ala Ala Leu Val Lys Glu
2900                2905                2910                2915

ggt tac aaa gtt cga ttg gat tta aat atc aca ctg gag ttc cgt act 8841
Gly Tyr Lys Val Arg Leu Asp Leu Asn Ile Thr Leu Glu Phe Arg Thr
                2920                2925                2930

acc tct aag aat ggc gtc ctc ctg gga atc agc agt gcc aaa gtg gat 8889
Thr Ser Lys Asn Gly Val Leu Leu Gly Ile Ser Ser Ala Lys Val Asp
            2935                2940                2945

gcc att ggc cta gag att gta gat ggc aag gtc tta ttt cac gtc aac 8937
Ala Ile Gly Leu Glu Ile Val Asp Gly Lys Val Leu Phe His Val Asn
        2950                2955                2960

aac ggt gcc gga agg ata aca gcc acc tac cag ccc aga gcc gcc aga 8985
Asn Gly Ala Gly Arg Ile Thr Ala Thr Tyr Gln Pro Arg Ala Ala Arg
    2965                2970                2975

gct ctc tgt gat ggc aag tgg cac aca ctc caa gcc cac aaa agc aag 9033
Ala Leu Cys Asp Gly Lys Trp His Thr Leu Gln Ala His Lys Ser Lys
2980                2985                2990                2995

cac cgc atc gtc ctg act gtg gac ggg aat tcc gtt agg gct gaa agc 9081
His Arg Ile Val Leu Thr Val Asp Gly Asn Ser Val Arg Ala Glu Ser
                3000                3005                3010

ccc cac acc cat tcc acc tcg gca gac acc aac gat ccc att tat gtg 9129
Pro His Thr His Ser Thr Ser Ala Asp Thr Asn Asp Pro Ile Tyr Val
            3015                3020                3025

ggt ggc tat cct gcc cac atc aaa cag aac tgc ctg agc agc cgg gcc 9177
Gly Gly Tyr Pro Ala His Ile Lys Gln Asn Cys Leu Ser Ser Arg Ala
        3030                3035                3040

tca ttc cgg ggc tgt gtg agg aac ctc agg ctg agc agg ggc tca caa 9225
Ser Phe Arg Gly Cys Val Arg Asn Leu Arg Leu Ser Arg Gly Ser Gln
    3045                3050                3055

gtg cag tcc ttg gac ctg agc cga gcc ttt gac cta caa gga gtc ttc 9273
Val Gln Ser Leu Asp Leu Ser Arg Ala Phe Asp Leu Gln Gly Val Phe
3060                3065                3070                3075

cct cat tcc tgc ccc ggg cct gag ccc taaactgtcg ccagcctctg 9320
Pro His Ser Cys Pro Gly Pro Glu Pro
                3080

cccttggaat catcgccaac gcatggaaga gagcagtttg tgaactcaag cagctcagct 9380
cccattccca tcccattgcc atctcaggtt atgtttccag aggaaaatgc tgtatttatg 9440
ttgaactaaa gccacacgga caacagatac ctctattaaa tggtttaaaa cgtcagtgga 9500

att                                                                9503

<210> 2
<211> 3084
<212> PRT
<213> Mus musculus

<400> 2
Met Arg Gly Ser Gly Thr Gly Ala Ala Leu Leu Val Leu Leu Ala Ser
1               5                   10                  15
Val Leu Trp Val Thr Val Arg Ser Gln Gln Arg Gly Leu Phe Pro Ala
            20                  25                  30
Ile Leu Asn Leu Ala Thr Asn Ala His Ile Ser Ala Asn Ala Thr Cys
        35                  40                  45
Gly Glu Lys Gly Pro Glu Met Phe Cys Lys Leu Val Glu His Val Pro
    50                  55                  60
Gly Arg Pro Val Arg His Ala Gln Cys Arg Val Cys Asp Gly Asn Ser
65                  70                  75                  80
Thr Asn Pro Arg Glu Arg His Pro Ile Ser His Ala Ile Asp Gly Thr
                85                  90                  95
Asn Asn Trp Trp Gln Ser Pro Ser Ile Gln Asn Gly Arg Glu Tyr His
            100                 105                 110
Trp Val Thr Val Thr Leu Asp Leu Arg Gln Val Phe Gln Val Ala Tyr
        115                 120                 125
Ile Ile Ile Lys Ala Ala Asn Ala Pro Arg Pro Gly Asn Trp Ile Leu
    130                 135                 140
Glu Arg Ser Val Asp Gly Val Lys Phe Lys Pro Trp Gln Tyr Tyr Ala
145                 150                 155                 160
Val Ser Asp Thr Glu Cys Leu Thr Arg Tyr Lys Ile Thr Pro Arg Arg
                165                 170                 175
Gly Pro Pro Thr Tyr Arg Ala Asp Asn Glu Val Ile Cys Thr Ser Tyr
            180                 185                 190
Tyr Ser Lys Leu Val Pro Leu Glu His Gly Glu Ile His Thr Ser Leu
        195                 200                 205
Ile Asn Gly Arg Pro Ser Ala Asp Asp Pro Ser Pro Gln Leu Leu Glu
    210                 215                 220
Phe Thr Ser Ala Arg Tyr Ile Arg Leu Arg Leu Gln Arg Ile Arg Thr
225                 230                 235                 240
Leu Asn Ala Asp Leu Met Thr Leu Ser His Arg Asp Leu Arg Asp Leu
                245                 250                 255
Asp Pro Ile Val Thr Arg Arg Tyr Tyr Tyr Ser Ile Lys Asp Ile Ser
            260                 265                 270
Val Gly Gly Met Cys Ile Cys Tyr Gly His Ala Ser Ser Cys Pro Trp
        275                 280                 285
Asp Glu Glu Ala Lys Gln Leu Gln Cys Gln Cys Glu His Asn Thr Cys
    290                 295                 300
Gly Glu Ser Cys Asp Arg Cys Cys Pro Gly Tyr His Gln Gln Pro Trp
305                 310                 315                 320
Arg Pro Gly Thr Ile Ser Ser Gly Asn Glu Cys Glu Glu Cys Asn Cys
                325                 330                 335
His Asn Lys Ala Lys Asp Cys Tyr Tyr Asp Ser Ser Val Ala Lys Glu
            340                 345                 350
Arg Arg Ser Leu Asn Thr Ala Gly Gln Tyr Ser Gly Gly Gly Val Cys
        355                 360                 365

Val Asn Cys Ser Gln Asn Thr Thr Gly Ile Asn Cys Glu Thr Cys Ile
    370                 375                 380
Asp Gln Tyr Tyr Arg Pro His Lys Val Ser Pro Tyr Asp Asp His Pro
385                 390                 395                 400
Cys Arg Pro Cys Asn Cys Asp Pro Val Gly Ser Leu Ser Ser Val Cys
                405                 410                 415
Ile Lys Asp Asp Arg His Ala Asp Leu Ala Asn Gly Lys Trp Pro Gly
            420                 425                 430
Gln Cys Pro Cys Arg Lys Gly Tyr Ala Gly Asp Lys Cys Asp Arg Cys
        435                 440                 445
Gln Phe Gly Tyr Arg Gly Phe Pro Asn Cys Ile Pro Cys Asp Cys Arg
    450                 455                 460
Thr Val Gly Ser Leu Asn Glu Asp Pro Cys Ile Glu Pro Cys Leu Cys
465                 470                 475                 480
Lys Lys Asn Val Glu Gly Lys Asn Cys Asp Arg Cys Lys Pro Gly Phe
                485                 490                 495
Tyr Asn Leu Lys Glu Arg Asn Pro Glu Gly Cys Ser Glu Cys Phe Cys
            500                 505                 510
Phe Gly Val Ser Gly Val Cys Asp Ser Leu Thr Trp Ser Ile Ser Gln
        515                 520                 525
Val Thr Asn Met Ser Gly Trp Leu Val Thr Asp Leu Met Ser Thr Asn
    530                 535                 540
Lys Ile Arg Ser Gln Gln Asp Val Leu Gly Gly His Arg Gln Ile Ser
545                 550                 555                 560
Ile Asn Asn Thr Ala Val Met Gln Arg Leu Thr Ser Thr Tyr Tyr Trp
                565                 570                 575
Ala Ala Pro Glu Ala Tyr Leu Gly Asn Lys Leu Thr Ala Phe Gly Gly
            580                 585                 590
Phe Leu Lys Tyr Thr Val Ser Tyr Asp Ile Pro Val Glu Thr Val Asp
        595                 600                 605
Ser Asp Leu Met Ser His Ala Asp Ile Ile Ile Lys Gly Asn Gly Leu
    610                 615                 620
Thr Ile Ser Thr Arg Ala Glu Gly Leu Ser Leu Gln Pro Tyr Glu Glu
625                 630                 635                 640
Tyr Phe Asn Val Val Arg Leu Val Pro Glu Asn Phe Arg Asp Phe Asn
                645                 650                 655
Thr Arg Arg Glu Ile Asp Arg Asp Gln Leu Met Thr Val Leu Ala Asn
            660                 665                 670
Val Thr His Leu Leu Ile Arg Ala Asn Tyr Asn Ser Ala Lys Met Ala
        675                 680                 685
Leu Tyr Arg Leu Asp Ser Val Ser Leu Asp Ile Ala Ser Pro Asn Ala
    690                 695                 700
Ile Asp Leu Ala Val Ala Ala Asp Val Glu His Cys Glu Cys Pro Gln
705                 710                 715                 720
Gly Tyr Thr Gly Thr Ser Cys Glu Ala Cys Leu Pro Gly Tyr Tyr Arg
                725                 730                 735
Val Asp Gly Ile Leu Phe Gly Gly Ile Cys Gln Pro Cys Glu Cys His
            740                 745                 750
Gly His Ala Ser Glu Cys Asp Ile His Gly Ile Cys Ser Val Cys Thr
        755                 760                 765
His Asn Thr Thr Gly Asp His Cys Glu Gln Cys Leu Pro Gly Phe Tyr
    770                 775                 780
Gly Thr Pro Ser Arg Gly Thr Pro Gly Asp Cys Gln Pro Cys Ala Cys
785                 790                 795                 800

Pro Leu Ser Ile Asp Ser Asn Asn Phe Ser Pro Thr Cys His Leu Thr
                805                 810                 815
Asp Gly Glu Glu Val Val Cys Asp Gln Cys Ala Pro Gly Tyr Ser Gly
            820                 825                 830
Ser Trp Cys Glu Arg Cys Ala Asp Gly Tyr Tyr Gly Asn Pro Thr Val
        835                 840                 845
Pro Gly Gly Thr Cys Val Pro Cys Asn Cys Ser Gly Asn Val Asp Pro
    850                 855                 860
Leu Glu Ala Gly His Cys Asp Ser Val Thr Gly Glu Cys Leu Lys Cys
865                 870                 875                 880
Leu Trp Asn Thr Asp Gly Ala His Cys Glu Arg Cys Ala Asp Gly Phe
                885                 890                 895
Tyr Gly Asp Ala Val Thr Ala Lys Asn Cys Arg Ala Cys Asp Cys His
            900                 905                 910
Glu Asn Gly Ser Leu Ser Gly Val Cys His Leu Glu Thr Gly Leu Cys
        915                 920                 925
Asp Cys Lys Pro His Val Thr Gly Gln Gln Cys Asp Gln Cys Leu Ser
    930                 935                 940
Gly Tyr Tyr Gly Leu Asp Thr Gly Leu Gly Cys Val Pro Cys Asn Cys
945                 950                 955                 960
Ser Val Glu Gly Ser Val Ser Asp Asn Cys Thr Glu Glu Gly Gln Cys
                965                 970                 975
His Cys Gly Pro Gly Val Ser Gly Lys Gln Cys Asp Arg Cys Ser His
            980                 985                 990
Gly Phe Tyr Ala Phe Gln Asp Gly Gly Cys Thr Pro Cys Asp Cys Ala
        995                 1000                1005
His Thr Gln Asn Asn Cys Asp Pro Ala Ser Gly Glu Cys Leu Cys Pro
    1010                1015                1020
Pro His Thr Gln Gly Leu Lys Cys Glu Glu Cys Glu Glu Ala Tyr Trp
1025                1030                1035                1040
Gly Leu Asp Pro Glu Gln Gly Cys Gln Ala Cys Asn Cys Ser Ala Val
                1045                1050                1055
Gly Ser Thr Ser Ala Gln Cys Asp Val Leu Ser Gly His Cys Pro Cys
            1060                1065                1070
Lys Lys Gly Phe Gly Gly Gln Ser Cys His Gln Cys Ser Leu Gly Tyr
        1075                1080                1085
Arg Ser Phe Pro Asp Cys Val Pro Cys Gly Cys Asp Leu Arg Gly Thr
    1090                1095                1100
Leu Pro Asp Thr Cys Asp Leu Glu Gln Gly Leu Cys Ser Cys Ser Glu
1105                1110                1115                1120
Asp Ser Gly Thr Cys Ser Cys Lys Glu Asn Val Val Gly Pro Gln Cys
                1125                1130                1135
Ser Lys Cys Gln Ala Gly Thr Phe Ala Leu Arg Gly Asp Asn Pro Gln
            1140                1145                1150
Gly Cys Ser Pro Cys Phe Cys Phe Gly Leu Ser Gln Leu Cys Ser Glu
        1155                1160                1165
Leu Glu Gly Tyr Val Arg Thr Leu Ile Thr Leu Ala Ser Asp Gln Pro
    1170                1175                1180
Leu Leu His Val Val Ser Gln Ser Asn Leu Lys Gly Thr Ile Glu Gly
1185                1190                1195                1200
Val His Phe Gln Pro Pro Asp Thr Leu Leu Asp Ala Glu Ala Val Arg
                1205                1210                1215
Gln His Ile Tyr Ala Glu Pro Phe Tyr Trp Arg Leu Pro Lys Gln Phe
            1220                1225                1230

Gln Gly Asp Gln Leu Leu Ala Tyr Gly Gly Lys Leu Gln Tyr Ser Val
        1235                1240                1245
Ala Phe Tyr Ser Thr Leu Gly Thr Gly Thr Ser Asn Tyr Glu Pro Gln
    1250                1255                1260
Val Leu Ile Lys Gly Gly Arg Ala Arg Lys His Val Ile Tyr Met Asp
1265                1270                1275                1280
Ala Pro Ala Pro Glu Asn Gly Val Arg Gln Asp Tyr Glu Val Gln Met
                1285                1290                1295
Lys Glu Glu Phe Trp Lys Tyr Phe Asn Ser Val Ser Glu Lys His Val
            1300                1305                1310
Thr His Ser Asp Phe Met Ser Val Leu Ser Asn Ile Asp Tyr Ile Leu
        1315                1320                1325
Ile Lys Ala Ser Tyr Gly Gln Gly Leu Gln Gln Ser Arg Ile Ala Asn
    1330                1335                1340
Ile Ser Met Glu Val Gly Arg Lys Ala Val Glu Leu Pro Ala Glu Gly
1345                1350                1355                1360
Glu Ala Ala Leu Leu Leu Glu Leu Cys Val Cys Pro Pro Gly Thr Ala
                1365                1370                1375
Gly His Ser Cys Gln Asp Cys Ala Pro Gly Tyr Tyr Arg Glu Lys Leu
            1380                1385                1390
Pro Glu Ser Gly Gly Arg Gly Pro Arg Pro Leu Leu Ala Pro Cys Val
        1395                1400                1405
Pro Cys Asn Cys Asn Asn His Ser Asp Val Cys Asp Pro Glu Thr Gly
    1410                1415                1420
Lys Cys Leu Ser Cys Arg Asp His Thr Ser Gly Asp His Cys Glu Leu
1425                1430                1435                1440
Cys Ala Ser Gly Tyr Tyr Gly Lys Val Thr Gly Leu Pro Gly Asp Cys
                1445                1450                1455
Thr Pro Cys Thr Cys Pro His His Pro Pro Phe Ser Phe Ser Pro Thr
            1460                1465                1470
Cys Val Val Glu Gly Asp Ser Asp Phe Arg Cys Asn Ala Cys Leu Pro
        1475                1480                1485
Gly Tyr Glu Gly Gln Tyr Cys Glu Arg Cys Ser Ala Gly Tyr His Gly
    1490                1495                1500
Asn Pro Arg Ala Ala Gly Gly Ser Cys Gln Thr Cys Asp Cys Asn Pro
1505                1510                1515                1520
Gln Gly Ser Val His Ser Asp Cys Asp Arg Ala Ser Gly Gln Cys Val
                1525                1530                1535
Cys Lys Pro Gly Ala Thr Gly Leu His Cys Glu Lys Cys Leu Pro Arg
            1540                1545                1550
His Ile Leu Met Glu Ser Asp Cys Val Ser Cys Asp Asp Asp Cys Val
        1555                1560                1565
Gly Pro Leu Leu Asn Asp Leu Asp Ser Val Gly Asp Ala Val Leu Ser
    1570                1575                1580
Leu Asn Leu Thr Gly Val Ser Pro Ala Pro Tyr Gly Ile Leu Glu Asn
1585                1590                1595                1600
Leu Glu Asn Thr Thr Lys Tyr Phe Gln Arg Tyr Leu Ile Lys Glu Asn
                1605                1610                1615
Ala Lys Lys Ile Arg Ala Glu Ile Gln Leu Glu Gly Ile Ala Glu Gln
            1620                1625                1630
Thr Glu Asn Leu Gln Lys Glu Leu Thr Arg Val Leu Ala Arg His Gln
        1635                1640                1645
Lys Val Asn Ala Glu Met Glu Arg Thr Ser Asn Gly Thr Gln Ala Leu
    1650                1655                1660

Ala Thr Phe Ile Glu Gln Leu His Ala Asn Ile Lys Glu Ile Thr Glu
1665                1670                1675                1680
Lys Val Ala Thr Leu Asn Gln Thr Ala Arg Lys Asp Phe Gln Pro Pro
                1685                1690                1695
Val Ser Ala Leu Gln Ser Met His Gln Asn Ile Ser Ser Leu Leu Gly
            1700                1705                1710
Leu Ile Lys Glu Arg Asn Phe Thr Glu Met Gln Gln Asn Ala Thr Leu
        1715                1720                1725
Glu Leu Lys Ala Ala Lys Asp Leu Leu Ser Arg Ile Gln Lys Arg Phe
    1730                1735                1740
Gln Lys Pro Gln Glu Lys Leu Lys Ala Leu Lys Glu Ala Asn Ser Leu
1745                1750                1755                1760
Leu Ser Asn His Ser Glu Lys Leu Gln Ala Ala Glu Glu Leu Leu Lys
                1765                1770                1775
Glu Ala Gly Ser Lys Thr Gln Glu Ser Asn Leu Leu Leu Leu Leu Val
            1780                1785                1790
Lys Ala Asn Leu Lys Glu Glu Phe Gln Glu Lys Lys Leu Arg Val Gln
        1795                1800                1805
Glu Glu Gln Asn Val Thr Ser Glu Leu Ile Ala Lys Gly Arg Glu Trp
    1810                1815                1820
Val Asp Ala Ala Gly Thr His Thr Ala Ala Ala Gln Asp Thr Leu Thr
1825                1830                1835                1840
Gln Leu Glu His His Arg Asp Glu Leu Leu Leu Trp Ala Arg Lys Ile
                1845                1850                1855
Arg Ser His Val Asp Asp Leu Val Met Gln Met Ser Lys Arg Arg Ala
            1860                1865                1870
Arg Asp Leu Val His Arg Ala Glu Gln His Ala Ser Glu Leu Gln Ser
        1875                1880                1885
Arg Ala Gly Ala Leu Asp Arg Asp Leu Glu Asn Val Arg Asn Val Ser
    1890                1895                1900
Leu Asn Ala Thr Ser Ala Ala His Val His Ser Asn Ile Gln Thr Leu
1905                1910                1915                1920
Thr Glu Glu Ala Glu Met Leu Ala Ala Asp Ala His Lys Thr Ala Asn
                1925                1930                1935
Lys Thr Asp Leu Ile Ser Glu Ser Leu Ala Ser Arg Gly Lys Ala Val
            1940                1945                1950
Leu Gln Arg Ser Ser Arg Phe Leu Lys Glu Ser Val Gly Thr Arg Arg
        1955                1960                1965
Lys Gln Gln Gly Ile Thr Met Lys Leu Asp Glu Leu Lys Asn Leu Thr
    1970                1975                1980
Ser Gln Phe Gln Glu Ser Val Asp Asn Ile Thr Lys Gln Ala Asn Asp
1985                1990                1995                2000
Ser Leu Ala Met Leu Arg Glu Ser Pro Gly Gly Met Arg Glu Lys Gly
                2005                2010                2015
Arg Lys Ala Arg Glu Leu Ala Ala Ala Ala Asn Glu Ser Ala Val Lys
            2020                2025                2030
Thr Leu Glu Asp Val Leu Ala Leu Ser Leu Arg Val Phe Asn Thr Ser
        2035                2040                2045
Glu Asp Leu Ser Arg Val Asn Ala Thr Val Gln Glu Thr Asn Asp Leu
    2050                2055                2060
Leu His Asn Ser Thr Met Thr Thr Leu Leu Ala Gly Arg Lys Met Lys
2065                2070                2075                2080
Asp Met Glu Met Gln Ala Asn Leu Leu Leu Asp Arg Leu Lys Pro Leu
                2085                2090                2095

Lys Thr Leu Glu Glu Asn Leu Ser Arg Asn Leu Ser Glu Ile Lys Leu
            2100                2105                2110
Leu Ile Ser Arg Ala Arg Lys Gln Ala Ala Ser Ile Lys Val Ala Val
        2115                2120                2125
Ser Ala Asp Arg Asp Cys Ile Arg Ala Tyr Gln Pro Gln Thr Ser Ser
    2130                2135                2140
Thr Asn Tyr Asn Thr Leu Ile Leu Asn Val Lys Thr Gln Glu Pro Asp
2145                2150                2155                2160
Asn Leu Leu Phe Tyr Leu Gly Ser Ser Ser Ser Ser Asp Phe Leu Ala
                2165                2170                2175
Val Glu Met Arg Arg Gly Lys Val Ala Phe Leu Trp Asp Leu Gly Ser
            2180                2185                2190
Gly Ser Thr Arg Leu Glu Phe Pro Glu Val Ser Ile Asn Asn Asn Arg
        2195                2200                2205
Trp His Ser Ile Tyr Ile Thr Arg Phe Gly Asn Met Gly Ser Leu Ser
    2210                2215                2220
Val Lys Glu Ala Ser Ala Ala Glu Asn Pro Pro Val Arg Thr Ser Lys
2225                2230                2235                2240
Ser Pro Gly Pro Ser Lys Val Leu Asp Ile Asn Asn Ser Thr Leu Met
                2245                2250                2255
Phe Val Gly Gly Leu Gly Gly Gln Ile Lys Lys Ser Pro Ala Val Lys
            2260                2265                2270
Val Thr His Phe Lys Gly Cys Met Gly Glu Ala Phe Leu Asn Gly Lys
        2275                2280                2285
Ser Ile Gly Leu Trp Asn Tyr Ile Glu Arg Glu Gly Lys Cys Asn Gly
    2290                2295                2300
Cys Phe Gly Ser Ser Gln Asn Glu Asp Ser Ser Phe His Phe Asp Gly
2305                2310                2315                2320
Ser Gly Tyr Ala Met Val Glu Lys Thr Leu Arg Pro Thr Val Thr Gln
                2325                2330                2335
Ile Val Ile Leu Phe Ser Thr Phe Ser Pro Asn Gly Leu Leu Phe Tyr
            2340                2345                2350
Leu Ala Ser Asn Gly Thr Lys Asp Phe Leu Ser Ile Glu Leu Val Arg
        2355                2360                2365
Gly Arg Val Lys Val Met Val Asp Leu Gly Ser Gly Pro Leu Thr Leu
    2370                2375                2380
Met Thr Asp Arg Arg Tyr Asn Asn Gly Thr Trp Tyr Lys Ile Ala Phe
2385                2390                2395                2400
Gln Arg Asn Arg Lys Gln Gly Leu Leu Ala Val Phe Asp Ala Tyr Asp
                2405                2410                2415
Thr Ser Asp Lys Glu Thr Lys Gln Gly Glu Thr Pro Gly Ala Ala Ser
            2420                2425                2430
Asp Leu Asn Arg Leu Glu Lys Asp Leu Ile Tyr Val Gly Gly Leu Pro
        2435                2440                2445
His Ser Lys Ala Val Arg Lys Gly Val Ser Ser Arg Ser Tyr Val Gly
    2450                2455                2460
Cys Ile Lys Asn Leu Glu Ile Ser Arg Ser Thr Phe Asp Leu Leu Arg
2465                2470                2475                2480
Asn Ser Tyr Gly Val Arg Lys Gly Cys Ala Leu Glu Pro Ile Gln Ser
                2485                2490                2495
Val Ser Phe Leu Arg Gly Gly Tyr Val Glu Met Pro Pro Lys Ser Leu
            2500                2505                2510
Ser Pro Glu Ser Ser Leu Leu Ala Thr Phe Ala Thr Lys Asn Ser Ser
        2515                2520                2525

Gly Ile Leu Leu Val Ala Leu Gly Lys Asp Ala Glu Glu Ala Gly Gly
    2530                2535                2540
Ala Gln Ala His Val Pro Phe Phe Ser Ile Met Leu Leu Glu Gly Arg
2545                2550                2555                2560
Ile Glu Val His Val Asn Ser Gly Asp Gly Thr Ser Leu Arg Lys Ala
                2565                2570                2575
Leu Leu His Ala Pro Thr Gly Ser Tyr Ser Asp Gly Gln Glu His Ser
            2580                2585                2590
Ile Ser Leu Val Arg Asn Arg Arg Val Ile Thr Ile Gln Val Asp Glu
        2595                2600                2605
Asn Ser Pro Val Glu Met Lys Leu Gly Pro Leu Thr Glu Gly Lys Thr
    2610                2615                2620
Ile Asp Ile Ser Asn Leu Tyr Ile Gly Gly Leu Pro Glu Asp Lys Ala
2625                2630                2635                2640
Thr Pro Met Leu Lys Met Arg Thr Ser Phe His Gly Cys Ile Lys Asn
                2645                2650                2655
Val Val Leu Asp Ala Gln Leu Leu Asp Phe Thr His Ala Thr Gly Ser
            2660                2665                2670
Glu Gln Val Glu Leu Asp Thr Cys Leu Leu Ala Glu Glu Pro Met Gln
        2675                2680                2685
Ser Leu His Arg Glu His Gly Glu Leu Pro Pro Glu Pro Pro Thr Leu
    2690                2695                2700
Pro Gln Pro Glu Leu Cys Ala Val Asp Thr Ala Pro Gly Tyr Val Ala
2705                2710                2715                2720
Gly Ala His Gln Phe Gly Leu Ser Gln Asn Ser His Leu Val Leu Pro
                2725                2730                2735
Leu Asn Gln Ser Asp Val Arg Lys Arg Leu Gln Val Gln Leu Ser Ile
            2740                2745                2750
Arg Thr Phe Ala Ser Ser Gly Leu Ile Tyr Tyr Val Ala His Gln Asn
        2755                2760                2765
Gln Met Asp Tyr Ala Thr Leu Gln Leu Gln Glu Gly Arg Leu His Phe
    2770                2775                2780
Met Phe Asp Leu Gly Lys Gly Arg Thr Lys Val Ser His Pro Ala Leu
2785                2790                2795                2800
Leu Ser Asp Gly Lys Trp His Thr Val Lys Thr Glu Tyr Ile Lys Arg
                2805                2810                2815
Lys Ala Phe Met Thr Val Asp Gly Gln Glu Ser Pro Ser Val Thr Val
            2820                2825                2830
Val Gly Asn Ala Thr Thr Leu Asp Val Glu Arg Lys Leu Tyr Leu Gly
        2835                2840                2845
Gly Leu Pro Ser His Tyr Arg Ala Arg Asn Ile Gly Thr Ile Thr His
    2850                2855                2860
Ser Ile Pro Ala Cys Ile Gly Glu Ile Met Val Asn Gly Gln Gln Leu
2865                2870                2875                2880
Asp Lys Asp Arg Pro Leu Ser Ala Ser Ala Val Asp Arg Cys Tyr Val
                2885                2890                2895
Val Ala Gln Glu Gly Thr Phe Phe Glu Gly Ser Gly Tyr Ala Ala Leu
            2900                2905                2910
Val Lys Glu Gly Tyr Lys Val Arg Leu Asp Leu Asn Ile Thr Leu Glu
        2915                2920                2925
Phe Arg Thr Thr Ser Lys Asn Gly Val Leu Leu Gly Ile Ser Ser Ala
    2930                2935                2940
Lys Val Asp Ala Ile Gly Leu Glu Ile Val Asp Gly Lys Val Leu Phe
2945                2950                2955                2960

His Val Asn Asn Gly Ala Gly Arg Ile Thr Ala Thr Tyr Gln Pro Arg
                2965                2970                2975
Ala Ala Arg Ala Leu Cys Asp Gly Lys Trp His Thr Leu Gln Ala His
            2980                2985                2990
Lys Ser Lys His Arg Ile Val Leu Thr Val Asp Gly Asn Ser Val Arg
        2995                3000                3005
Ala Glu Ser Pro His Thr His Ser Thr Ser Ala Asp Thr Asn Asp Pro
    3010                3015                3020
Ile Tyr Val Gly Gly Tyr Pro Ala His Ile Lys Gln Asn Cys Leu Ser
3025                3030                3035                3040
Ser Arg Ala Ser Phe Arg Gly Cys Val Arg Asn Leu Arg Leu Ser Arg
                3045                3050                3055
Gly Ser Gln Val Gln Ser Leu Asp Leu Ser Arg Ala Phe Asp Leu Gln
            3060                3065                3070
Gly Val Phe Pro His Ser Cys Pro Gly Pro Glu Pro
        3075                3080

<210> 3
<211> 30
<212> DNA
<213> Artificial Sequence

<220>
<223> Olgionucleotide Primer

<400> 3
cgggatccta gagactgcat ccgcgcctat                                        30

<210> 4
<211> 30
<212> DNA
<213> Artificial Sequence

<220>
<223> Olgionucleotide Primer

<400> 4
cccaagcttt actatctgcg tcacggtggg                                        30

<210> 5
<211> 33
<212> DNA
<213> Artificial Sequence

<220>
<223> Olgionucleotide Primer

<400> 5
cgggatcctc agatagtaat tctcttcagc acc                                    33

<210> 6
<211> 30
<212> DNA

<213> Artificial Sequence

<220>
<223> Olgionucleotide Primer

<400> 6
cccaagcttg gatgactcag gtgagagaga                                        30

<210> 7
<211> 28
<212> DNA
<213> Artificial Sequence

<220>
<223> Olgionucleotide Primer

<400> 7
cgggatcctc tgctggccac attcgcca                                          28

<210> 8
<211> 28
<212> DNA
<213> Artificial Sequence

<220>
<223> Olgionucleotide Primer

<400> 8
cccaagcttc ctcttccgga catcagac                                          28

<210> 9
<211> 30
<212> DNA
<213> Artificial Sequence

<220>
<223> Olgionucleotide Primer

<400> 9
cgggatcctc tccaggtgca gctgagcatt                                        30

<210> 10
<211> 30
<212> DNA
<213> Artificial Sequence

<220>
<223> Olgionucleotide Primer

<400> 10
cccaagcttc tgttggccat taaccatgat                                        30

<210> 11
<211> 30

<212> DNA
<213> Artificial Sequence

<220>
<223> Olgionucleotide Primer

<400> 11
cgggatcctc tggataaaga caggcccttg                                        30

<210> 12
<211> 32
<212> DNA
<213> Artificial Sequence

<220>
<223> Olgionucleotide Primer

<400> 12
cccaagcttg ggctcaggcc cggggcagga at                                     32

## Description

### RELATED APPLICATION

This application claims the benefit of U.S. Provisional Application No. 60/192875, filed on March 29, 2000, the entire teachings of the which are incorporated herein by reference.

### BACKGROUND OF THE INVENTION

Basement membranes are thin layers of specialized extracellular matrix that provide supporting structure on which epithelial and endothelial cells grow, and that surround muscle or fat (Paulsson, M., 1992, Crit. Rev. Biochem. Mol. Biol. 27:93-127). Basement membranes are always associated with cells, and it has been well documented that basement membranes not only provide mechanical support, but also influence cellular behavior such as differentiation and proliferation. Vascular basement membranes are composed of macromolecules such as collagen, laminin, heparan sulfate proteoglycans, fibronectin and nidogen (also called entactin) (Timpl, R., 1996, Curr. Opin. Cell. Biol. 8:618-624).

Angiogenesis is the process of formation of new blood vessels from pre-existing ones (Madri, J.A. et al., 1986, J. Histochem. Cytochem. 34:85-91; Folkman, J., 1972, Ann. Surg. 175:409-416). Angiogenesis is a complex process, and requires sprouting and migration of endothelial cells, proliferation of those cells, and their differentiation into tube-like structures and the production of a basement membrane matrix around the developing blood vessel. Additionally angiogenesis is a process critical for normal physiological events such as wound repair and endometrium remodeling (Folkman, J. et al., 1995, J. Biol. Chem. 267:10931-10934). It is now well documented that angiogenesis is required for metastasis and growth of solid tumors beyond a few mm³ in size (Folkman, J., 1972, Ann. Surg. 175:409-416; Folkman, J., 1995, Nat. Med. 1:27-31). Expansion of tumor mass occurs not only by perfusion of blood through the tumor, but also by paracrine stimulation of tumor cells by several growth factors and matrix proteins produced by the new capillary endothelium (Folkman, J., 1995, Nat. Med. 1:27-31). Recently, a number of angiogenesis inhibitors have been identified, namely angiostatin (O'Reilly, M.S. et al., 1994, Cell 79:315-28), endostatin (O'Reilly, M.S. et al., 1997, Cell 88:277-85), restin (Ramchandran, R. et al., 1999, Biochem. Biophys. Res. Commun. 255:735-9), Arresten (Colorado, P.C. et al., 2000, Cancer Res. 60:2520-6), Canstatin (Kamphaus, G.D. et al., 2000, J. Biol. Chem. 275:1209-15) and Tumstatin (Maeshima, Y. et al., 2000, J. Biol. Chem. 275:21340-8; Maeshima, Y. et al., 2000, J. Biol. Chem. 275:23745-50) and pigment epithelium-derived factor (PEDF) (Dawson, D.W. et al., 1999, Science 285:245-8).

### SUMMARY OF THE INVENTION

The.present invention relates to proteins comprising the globular domains of the α1 chain of laminin having anti-angiogenic properties. In particular, the present invention relates to the novel protein designated herein as "Matin," which comprises the G1 domain of laminin, and to biologically active (*e.g*., anti-angiogenic) fragments, mutants, analogs, homologs and derivatives thereof, as well as multimers (*e.g*., dimers, trimers, etc.) and fusion proteins (also referred to herein as chimeric proteins) thereof. In particular, Matin is a monomeric protein, and arrests endothelial cell proliferation *in vivo.*

In particular, the invention features isolated Matin, where Matin is an isolated protein of the G1 domain of the α1 chain of laminin, or a fragment, analog, derivative or mutant thereof, where the protein, fragment, analog, derivative or mutant thereof has anti-angiogenic activity. The Matin can be the G1 domain of the α1 chain of another laminin, other globular domains, globular domains from other α chains, other laminins, laminins from other mammals, and fragments, mutants, homologs, analogs and allelic variants of the Matin amino acid sequence. The Matin, or a fragment, analog, derivative or mutant thereof, can be a monomer, a multimer, or a chimeric protein, having anti-angiogenic or anti-tumor activity. The Matin can be about 28 to about 32 kDa in size, or about 30 kDa in size.

The invention also features the isolated protein of about residue 2148 to about residue 2354 of SEQ ID NO:2, about residue 2352 to about residue 2553 of SEQ ID NO:2, about residue 2534 to about residue 2761 of SEQ ID NO:2, about residue 2762 to about residue 2895 of SEQ ID NO:2, about residue 2896 to about residue 3100 of SEQ ID NO:2, or a fragment, analog, derivative or mutant thereof, where the protein, fragment, analog, derivative or mutant has anti-angiogenic activity. The protein, or a fragment, analog, derivative or mutant thereof, can be a monomer, dimer, trimer, multimer, or a chimeric protein, having anti-angiogenic or anti-tumor activity. The proteins, fragments, etc., from the other G domains of laminin are alternative sources of Matin.

The invention also features an isolated protein or peptide having 90% or greater sequence identity with SEQ ID NO:2, where the protein or peptide has anti-angiogenic activity, and an isolated protein or peptide having 85% or greater sequence identity with SEQ ID NO :2, where the protein or peptide has anti-angiogenic activity.

The invention also features an isolated polynucleotide having 90% or greater sequence identity with SEQ ID NO:1, where the polynucleotide encodes a protein having anti-angiogenic activity.

The chimeric protein described above can further comprise at least one protein molecule selected from the group consisting of: Vascostatin or fragments thereof, arresten or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins or fragments thereof. Vascostatin comprises the C-terminal globular domain of nidogen, and has anti-angiogenic properties. Vascostatin is described in International application PCT/US01/_, "Anti-Angiogenic and Anti-tumor Properties of Vascostatin and Other Nidogen Domains", by Raghuram Kalluri, filed March 28, 2001.

The invention further features a composition comprising, as a biologically active ingredient, one or more of the proteins, fragments, analogs, derivatives, mutants, monomers, multimers or chimeric proteins described above. The composition may also include a pharmaceutically-compatible carrier. The composition may further comprise at least one protein molecule selected from the group consisting of: Vascostatin or fragments thereof, arresten or fragments thereof, canstatin or fragments thereof, tumstatin or fragments thereof, endostatin or fragments thereof, angiostatin or fragments thereof, restin or fragments thereof, apomigren or fragments thereof, or other anti-angiogenic proteins, or fragments thereof. The composition may be used in a method of inhibiting a disease characterized by angiogenic activity, where the method comprises administering to a patient with the disease, the composition in conjunction with radiation therapy, chemotherapy, or immunotherapy.

In another aspect, the invention features a process for producing a protein encoded by the polynucleotide described above, where the process comprises: (a) growing a culture of a host cell transformed with the polynucleotide described above, where the host cell is selected from the group comprising bacterial, yeast, mammalian, insect or plant cells; and (b) purifying the protein from the culture, so that the protein encoded by the polynucleotide described above is produced.

The invention also features an isolated polynucleotide produced according to the process of: (a) preparing one or more polynucleotide probes that hybridize under conditions under moderate stringency to the polynucleotide described above; (b) hybridizing the probe(s) to mammalian DNA; and (c) isolating the DNA polynucleotide detected with the probe(s); so that the nucleotide sequence of the isolated polynucleotide corresponds to the nucleotide sequence of the polynucleotide described above.

The invention further features a process for providing a mammal with an anti-angiogenic protein, where the process comprises introducing mammalian cells into a mammal, the mammalian cells having been treated *in vitro* to insert within them the polynucleotide described above, and where the mammalian cell express *in vivo* within the mammal a therapeutically effective amount of the anti-angiogenic protein in an amount sufficient to inhibit angiogenic activity in the mammal. The expression of the anti-angiogenic protein may be transient or permanent expression. The mammalian cells may be chosen from the group consisting of: blood cells, TIL cells, bone marrow cells, vascular cells, tumor cells, liver cells, muscle cells, fibroblast cells. The polynucleotide may be inserted into the cells by a viral vector.

The invention additionally features antibodies that specifically bind to the isolated Matin protein, fragment, analog, derivative or mutant, or the Matin monomers, dimer, trimers, multimers or chimeric proteins described above.

In another aspect, the invention features a method for inhibiting angiogenic activity in mammalian tissue, where the method comprises contacting the tissue with a composition comprising one or more of the following: the Matin protein, fragment, analog, derivative or mutant described above, or the Matin monomers, multimers or chimeric proteins as described above. The angiogenic activity may be characteristic of a disease selected from the group comprising angiogenesis-dependent cancers, benign tumors, rheumatoid arthritis, diabetic retinopathy, psoriasis, ocular angiogenesis diseases, Osler-Webber Syndrome, myocardial angiogenesis, plaque neovascularization, telangiectasia, hemopheliac joints, angiofibroma, wound granulation, intestinal adhesions, atherosclerosis, scleroderma, hypertrophic scars, cat scratch disease, *Heliobacter pylori* ulcers, dialysis graft vascular access stenosis, contraception and obesity. The disease may be cancer.

The invention further features an isolated polynucleotide encoding an anti-angiogenic protein, where the isolated polynucleotide is produced by the process of: (a) preparing one or more polynucleotide probes that hybridize under conditions under moderate to high stringency to nucleotide 6442 to nucleotide 7062 of SEQ ID NO:1; (b) hybridizing the probe(s) to mammalian DNA; and (c) isolating the polynucleotide detected with the probe(s); so that the nucleotide sequence of the isolated polynucleotide has anti-angiogenic activity and corresponds to the nucleotide sequence of nucleotide 6442 to nucleotide 7062 of SEQ ID NO:1. The probes may be SEQ ID NO:3 and SEQ ID NO:4. The isolated polynucleotide may also be a subsequence of SEQ ID NO:1. Alternatively, the polynucleotide can also correspond to about nucleotide 7054 to about nucleotide 7599, nucleotide 7600 to about nucleotide 78283, nucleotide 8284 to about nucleotide 8685, or nucleotide 8686 to about nucleotide 9300.

The invention also features a method for producing an anti-angiogenic polypeptide, where the method comprises: (a) growing a culture of a host cell transformed with the polynucleotide of nucleotide 6442 to nucleotide 7062 of SEQ ID NO:1, where the host cell is selected from the group comprising bacterial, yeast, mammalian, insect or plant cells; and (b) purifying the protein from the culture; so that an anti-angiogenic polypeptide is produced. Alternatively, the polynucleotide can also correspond to about nucleotide 7054 to about nucleotide 7599, nucleotide 7600 to about nucleotide 78283, nucleotide 8284 to about nucleotide 8685, or nucleotide 8686 to about nucleotide 9300.

### BRIEF DESCRIPTION OF THE DRAWINGS

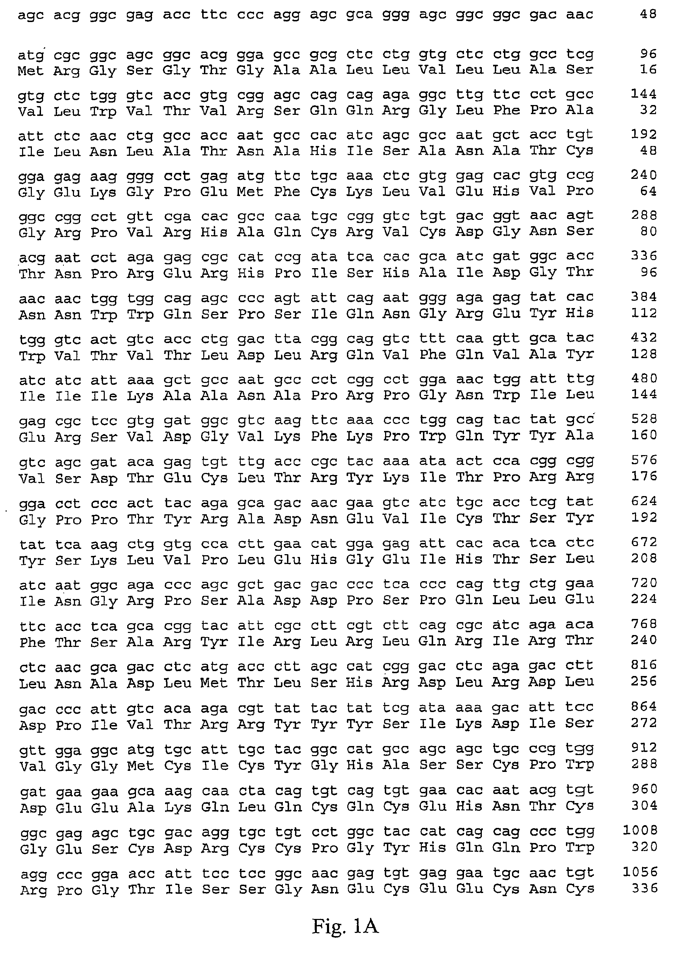

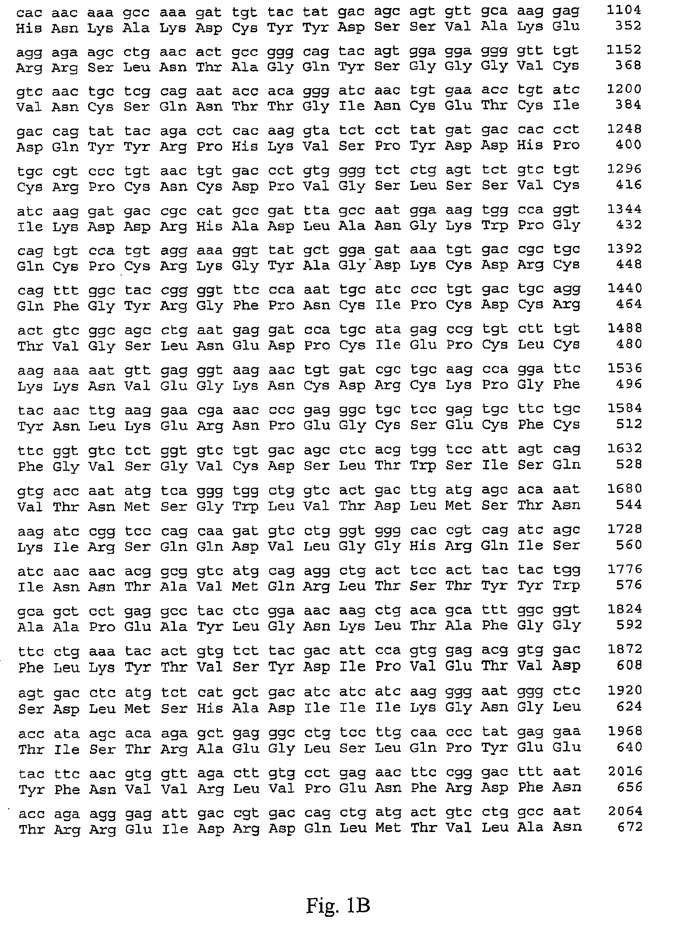

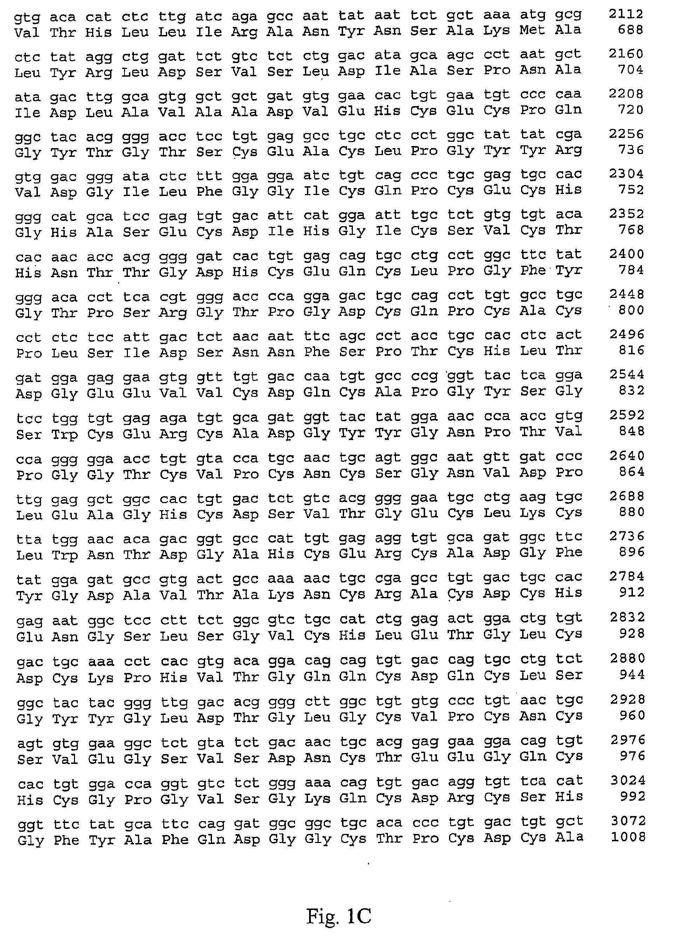

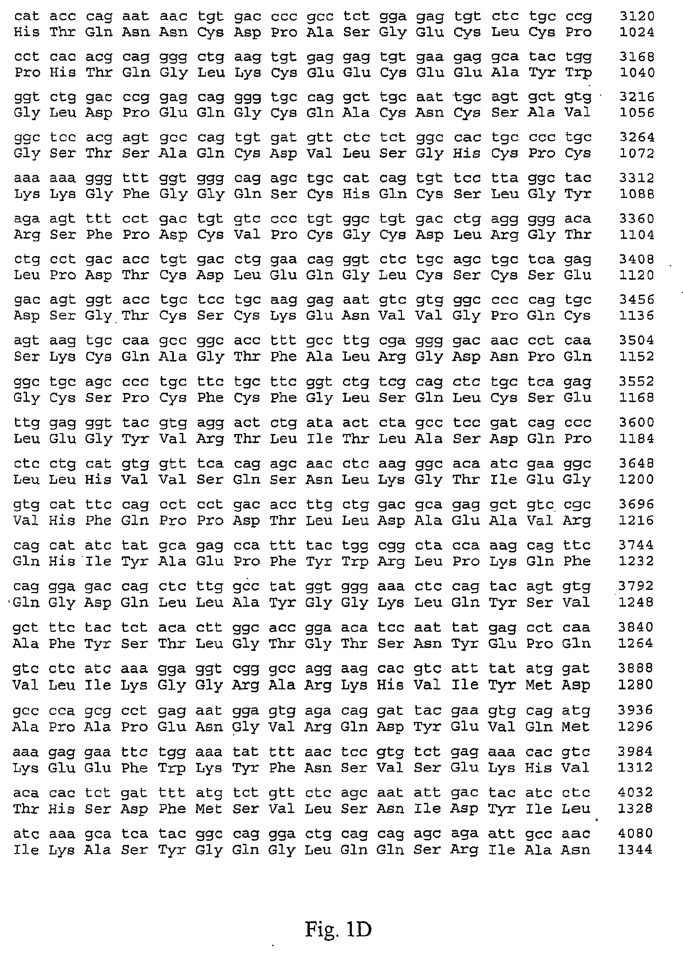

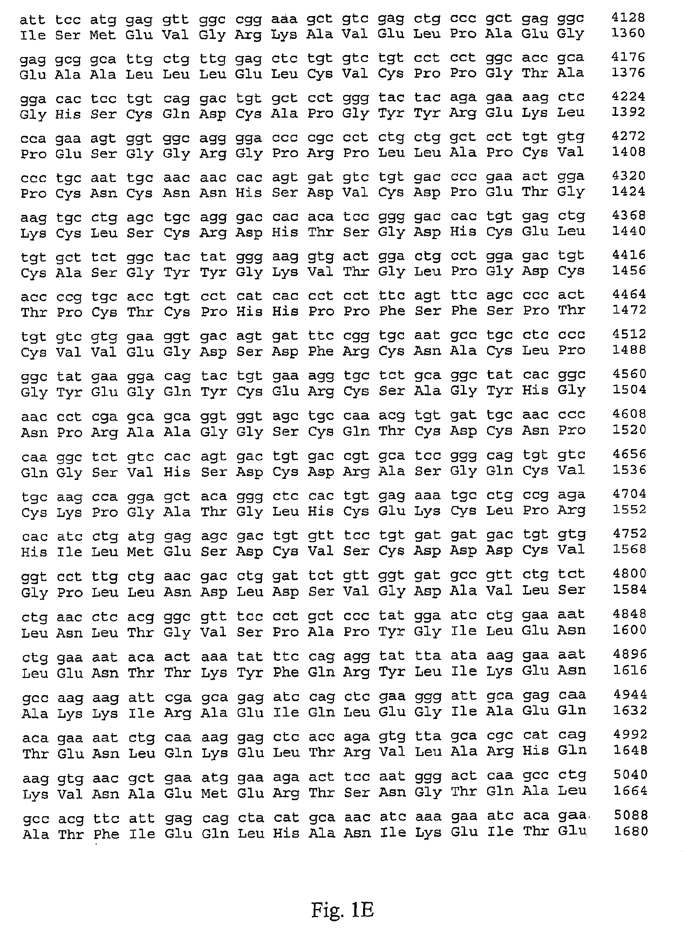

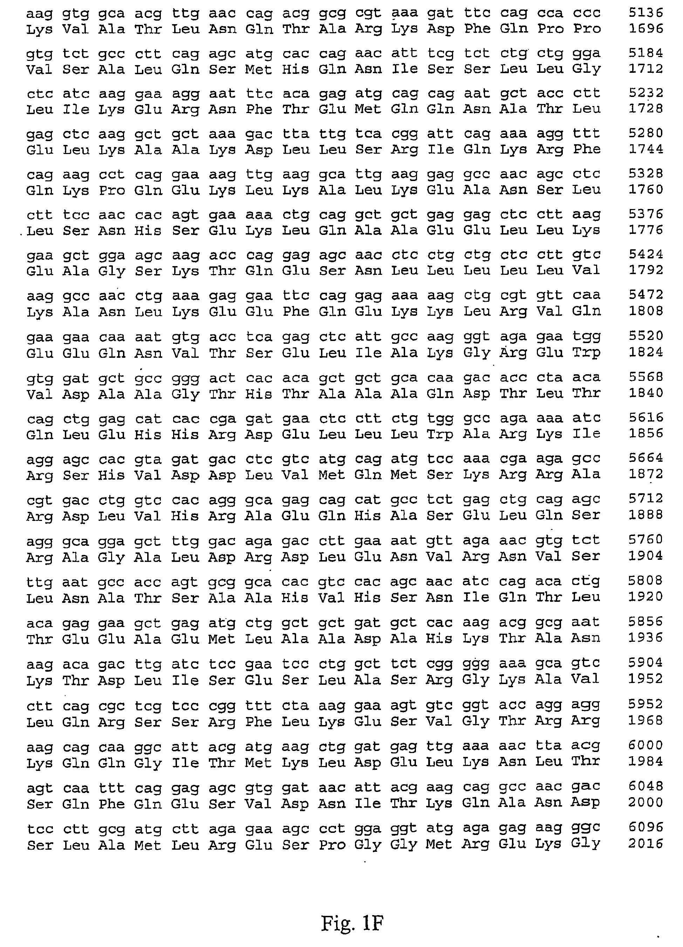

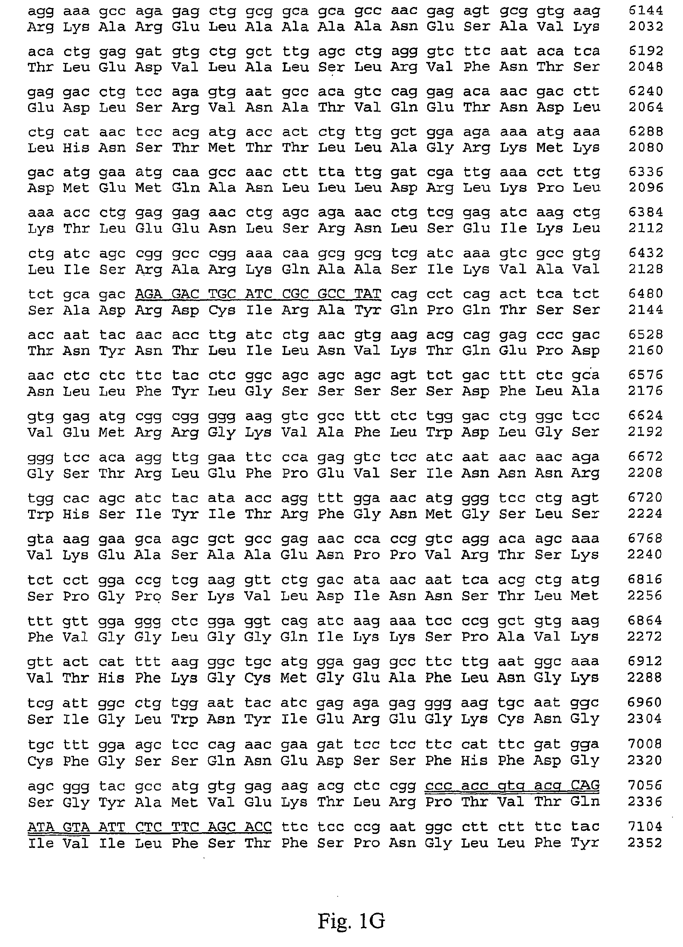

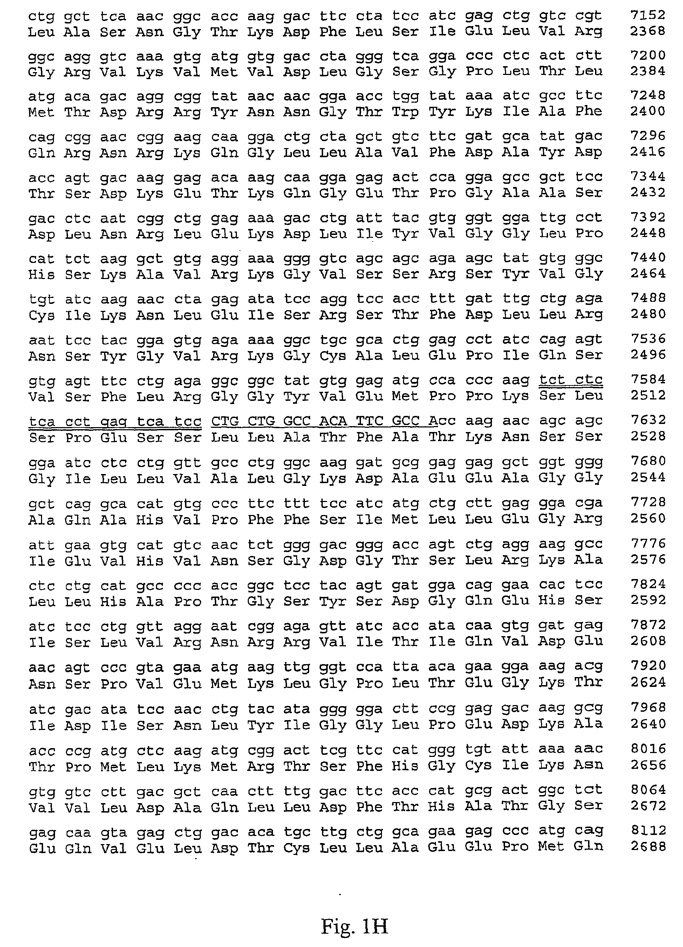

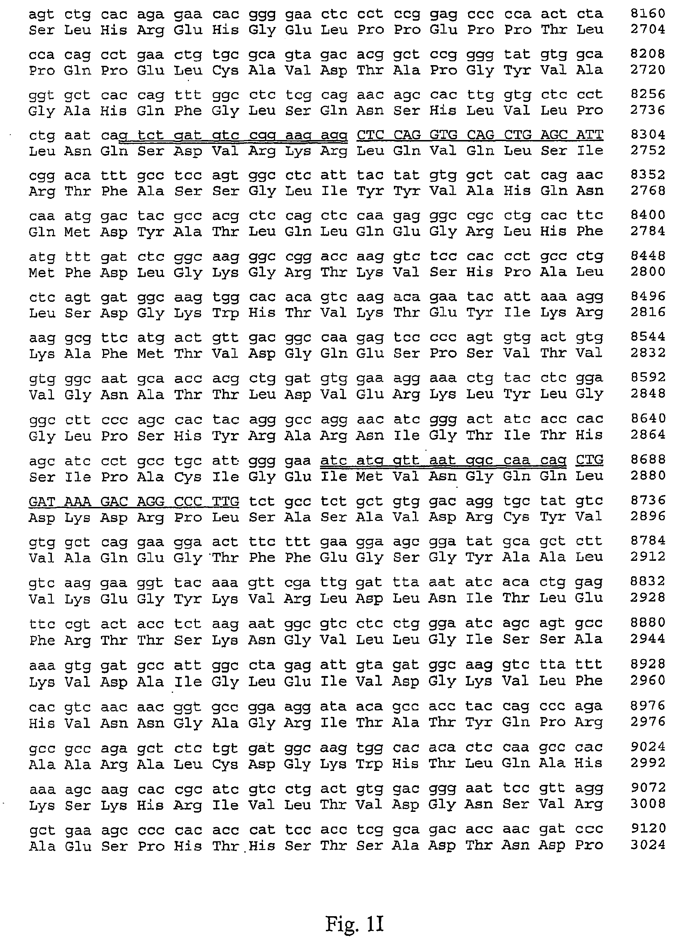

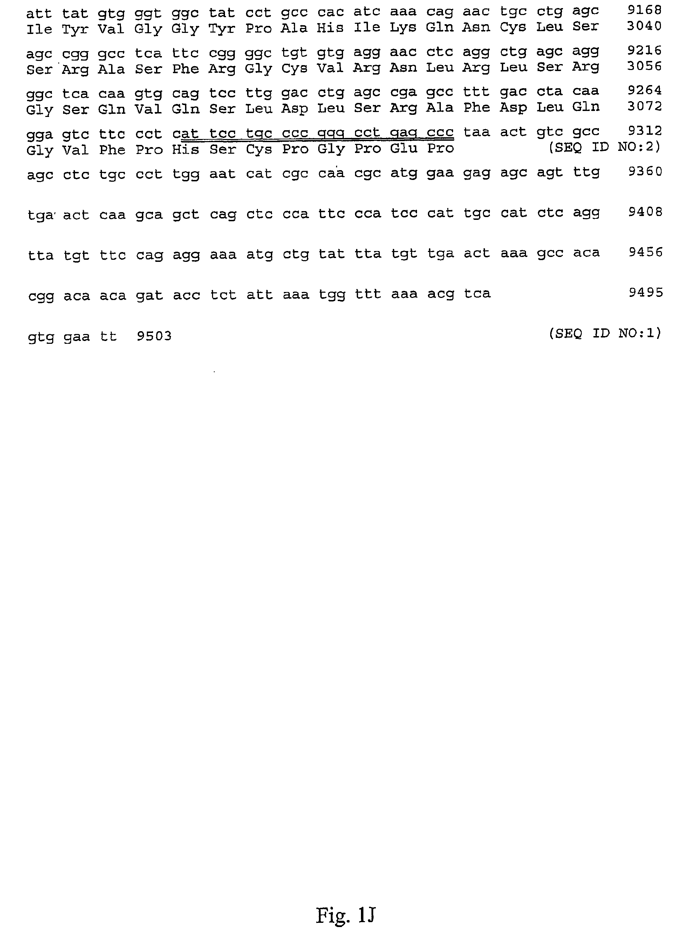

Figs. 1A, 1B, 1C, 1D, 1E, 1F, 1G, 1H, 1I and 1J are a diagram depicting the nucleotide (SEQ ID NO:1) and amino acid (SEQ ID NO:2) sequences of the five globular domains (G1, G2, G3, G4 and G5) of the α1 chain of laminin-1 (GenBank Acc.No. NM_008480). Forward primers are depicted in bold face type with single underlining, and reverse primers are shown with double underlining. Globular domain 1 (G1) extends from about nucleotide 6442 to about nucleotide 7062, and about amino acid 2132 to about amino acid 2338. The forward primer used for the G1 domain was 5'-CGG-GAT-CCT-AGA-GAC-TGC-ATC-CGC-GCC-TAT-3' (SEQ ID NO:3), and the reverse primer was 5'-CCC-AAG-CTT-TAC-TAT-CTG-CGT-CAC-GGT-GGG-3' (SEQ ID NO:4). Globular domain 2 (G2) extends from about nucleotide 7054 to about nucleotide 7599, and about amino acid 2336 to about amino acid 2517. The forward primer used for the G2 domain was 5'-CGG-GAT-CCT-CAG-ATA-GTA-ATT-CTC-TTC-AGC-ACC-3' (SEQ ID NO:5), and the reverse primer was 5'-CCC-AAG-CTT-GGA-TGA-CTC-AGG-TGA-GAG-AGA-3' (SEQ ID NO:6). Globular domain 3 (G3) extends from about nucleotide 7600 to about nucleotide 8283, and about amino acid 2518 to about amino acid 2745. The forward primer used for the G3 domain was 5'-CGG-GAT-CCT-CTG-CTG-GCC-ACA-TTC-GCC-A-3' (SEQ ID NO:7), and the reverse primer was S'-CCC-AAG-CTT-CCT-CTT-CCG-GAC-ATC-AGA-C-3' (SEQ ID NO:8). Globular domain 4 (G4) extends from about nucleotide 8284 to about nucleotide 8685, and about amino acid 2746 to about amino acid 2879. The forward primer used for the G4 domain was 5'-CGG-GAT-CCT-CTC-CAG-GTG-CAG-CTG-AGC-ATT-3' (SEQ ID NO:9), and the reverse primer was 5'-CCC-AAG-CTT-CTG-TTG-GCC-ATT-AAC-CAT-GAT-3' (SEQ ID NO:10). Globular domain 5 (G5) extends from about nucleotide 8686 to about nucleotide 9300, and about amino acid 2880 to about amino acid 3084. The forward primer used for the G5 domain was 5'-CGG-GAT-CCT-CTG GAT-AAA-GAC-AGG-CCC-TTG-3' (SEQ ID NO:11), and the reverse primer was 5'-CCC-AAG-CTT-GGG-CTC-AGG-CCC-GGG-GCA-GGA-AT-3' (SEQ ID NO:12). Underlined portions of the above primers correspond to the laminin sequence.
Fig. 2 is a schematic diagram representing the Matin cloning vector pET22b(+). Forward (SEQ ID NO:3) and reverse (SEQ ID NO:4) primers and site into which Matin was cloned are indicated.
Figs. 3A and 3B are histograms showing the effect of varying concentrations of Matin (x-axis) on proliferation of endothelial (C-PAE) cells (Fig. 3A) and non-endothelial (PC-3) cells (Fig. 3B). Proliferation was measured as a function of methylene blue staining.
Fig. 4 is a plot showing annexin V fluorescence for cells treated with Matin as compared to controls.
Figs. 5A and 5B are a pair of bar charts showing caspase-3 activity in Matin-treated CPAE cells (Fig. 5A) as compared to PC-3 cells (Fig. 5B).
Figs. 6A and 6B are a pair of histograms showing cell viability at increasing concentrations of Matin (x-axis) as a function of OD₅₉₀ (y-axis) in an MTT apoptosis assay for CPAE cells (Fig. 6A) as compared to PC-3 cells (Fig. 6B). Each point represents the mean +/- the standard error of the mean for triplicate wells.
Fig. 7 is a line graph showing the effect on tumor size (mm³, y-axis) against days of treatment (x-axis) with 20 mg/ml Matin (■) versus controls (20 mg/ml nephrin (○) and PBS (□).

### DETAILED DESCRIPTION OF THE INVENTION

A wide variety of diseases are the result of undesirable angiogenesis. Put another way, many diseases and undesirable conditions could be prevented or alleviated if it were possible to stop the growth and extension of capillary blood vessels under some conditions, at certain times, or in particular tissues.

The formation of new capillaries from pre-existing vessels, angiogenesis, is essential for the process of tumor growth and metastasis (Folkman, J. et al., 1992, J. Biol. Chem. 267:10931-10934; Folkman, J., 1995, Nat. Med. 1:27-31; Hanahan, D. et al., 1996, Cell 86:353-364). Human and animal tumors are not vascularized at the beginning, however for a tumor to grow beyond few mm³, it might vascularize (Folkman, J., 1995, Nat. Med. 1:27-31; Hanahan, D. et al., 1996, Cell 86;353-364). The switch to an angiogenic phenotype requires both upregulation of angiogenic stimulators and downregulation of angiogenesis inhibitors (Folkman, J., 1995, Nat. Med. 1:27-31). Vascular endothelial growth factor (VEGF) and basic fibroblast growth factor (bFGF) are the most commonly expressed angiogenic factors in tumors. Vascularized tumors may overexpress one or more of these angiogenic factors which can synergistically promote tumor growth. Inhibition of a single angiogenic factor such as VEGF with a receptor antagonist may not be enough to arrest tumor growth. A number of angiogenesis inhibitors have been recently identified, and certain factors such as IFN-α, platelet-factor-4 (Maione, T.E. et al., 1990, Science 247:77-79) and PEX (Brooks, P.C. et al., 1998, Cell 92:391-400) are not endogenously associated with tumor cells, whereas angiostatin (O'Reilly, M.S. et al., 1994, Cell 79:315-328) and endostatin (O'Reilly, M.S. et al., 1997, Cell 88:277-285) are tumor associated angiogenesis inhibitors generated by tumor tissue itself. Although treatment of tumor growth and metastasis with these endogenous angiogenesis inhibitors is very effective and an attractive idea, some potential problems associated with anti-angiogenic therapies must be considered. Delayed toxicity induced by chronic anti-angiogenic therapy as well as the possibility of impaired wound healing and reproductive angiogenesis occurring during treatment are to be considered seriously.

In the present invention, a protein, and fragments, analogs, derivatives, homologs and mutants thereof with anti-angiogenic properties are described, along with methods of use of this protein, analogs, derivatives, homologs and mutants to inhibit angiogenesis-mediated proliferative diseases. The protein comprises the G1 domain of the α1 chain of laminin, and is called "Matin." This protein is about 30 kDa, and inhibits endothelial cell proliferation.

Laminin is the most abundant noncollagenous protein found in basement membranes. It was initially purified from mouse EHS (Engelbreth-Holm-Swarm) tumors and the mouse embryonal carcinoma cell line M1536-B3. These oncogenic sources produce large amounts of easily extractable basement membrane-like substance, and most early research into components of the basement membrane used these tumor lines as sources, rather than naturally-occurring basement membranes. The patterns of gene expression are known to be different, however, between oncogenic and naturally-occurring tissues.

Laminin is a multidomain protein (Paulsson, M., 1992, Grit. Rev. Biochem. Mol. Biol. 27:93-127), with three distinct polypeptide chains, α, β1 and β2, connected into a cross shape by disulfide bonds. The N-terminal portion of each chain, containing domains III through VI, each forms one arm of the cross, and the C-terminal portions (containing domains I and II) of all three chains are joined together by disulfide bonds into the fourth arm. Put another way, the N-terminal half of the α chain makes up the vertical arms of the cross, while the N-terminal half of the β1 and β2 chains make up the left and right arms. The C-terminal halves of all three chains join together to form the lower vertical arm of the cross. The G domain only exists at the C-terminal end of the α chain, not on either of the β chains. The G domain is subdivided into five subdomains, G1 through G5. Merosin, an isoform of laminin, was found to share some amino acid identity with the C-terminus of the α chain of mouse laminin, and the general domain structure is conserved between the two.

Matin can be obtained from a variety of sources. Such sources include, but are not limited to, P19137 (LAMIN1N ALPHA-1 CHAIN PRECURSOR (LAMININ A CHAIN)), MMMSA (laminin alpha-1 chain precursor - mouse) and AAA39410 (laminin A chain [Mus musculus]). Human laminin has a slightly lower sequence identity with SEQ ID NO:2, *e.g.,* around 83%. Such sequences are also useful for obtaining Matin, and include, but are not limited to, P25391 (LAMININ ALPHA-1 CHAIN PRECURSOR (LAMININ A CHAIN)), S14458 (laminin alpha-1 chain precursor - human) and CAA41418 (laminin A chain [Homo sapiens]). Other sequences have a lower identity with SEQ ID NO:2, but may still be useful sources of anti-angiogenic Matin. These may include, but are not necessarily limited to, PX0082 (laminin, M chain - human (fragment)), MMHUMH (laminin alpha-2 chain - human (fragment)), AAB33989 (laminin M chain, merosin=basement membrane protein {G-domain} [human, placenta, Peptide Partial, 1751 aa] [Homo sapiens]), AAA63215 (merosin [Homo sapiens]), AAB18388 (laminin alpha 2 chain [Homo sapiens]), NP_000417 (laminin alpha 2 subunit precursor; Laminin, alpha-2 (merosin) [Homo sapiens]), P24043 (LAMININ ALPHA-2 CHAIN PRECURSOR (LAMININ M CHAIN) (MEROSIN HEAVY CHAIN)), CAA81394 (laminin M chain (merosin) [Homo sapiens]), XP_011387 (laminin alpha 2 subunit precursor [Homo sapiens]), Q60675 (LAMININ ALPHA-2 CHAIN PRECURSOR (LAMININ M CHAIN) (MEROSIN HEAVY CHAIN)), 553868 (laminin alpha-2 chain precursor - mouse), AAC52165 (laminin-2 alpha2 chain precursor [Mus musculus]).

Interestingly, the various globular domains themselves possess varying levels of sequence identity with each other. This is shown in Table 1, below.

**Table 1. Percent sequence identity of the globular domains of the mouse laminin α chain.**

| | G1 | G2 | G3 | G4 | G5 |
|---|---|---|---|---|---|
| G1 | | | | | |
| G2 | 31 | | | | |
| G3 | - | 27 | | | |
| G4 | 24 | 25 | - | | |
| G5 | 23 | 31 | 27 | 25 | |

Polynucleotides encoding Matin can also be obtained from a variety of sources. For instance, other mouse laminin α chain globular domains *(e.g., )* generally possess greater than 90% sequence identity with SEQ ID NO:1, and include, but are not limited to, J04064 (Mus musculus laminin A chain mRNA, complete cds) and X58531 (Human LAMA mRNA for laminin A chain, partial cds).

As disclosed herein, Matin can be can be produced in *E. coli* using a bacterial expression plasmid, such as pET22b, which is capable of periplasmic transport, thus resulting in soluble protein. Matin can also be produced in other cells, for instance, it can be produced as a secreted soluble protein in 293 kidney cells using the pcDNA 3.1 eukaryotic vector.

*E. coli*-produced Matin inhibits endothelial cell proliferation of endothelial cells in a dose-dependent manner.

Specific inhibition of endothelial cell proliferation and migration by Matin demonstrates its anti-angiogenic activity, and that it may function via a cell surface protein/receptor. Integrins are potential candidate molecules based on their extracellular matrix binding capacity and ability to modulate cell behavior such as migration and proliferation. In particular, aᵥb₃ integrin is a possible receptor, due to its induction during angiogenesis, and its promiscuous binding capacity. Angiogenesis also depends on specific endothelial cell adhesive events mediated by integrin aᵥb₃ (Brooks, P.C. et al., 1994, Cell 79:1157-1164). Matin may disrupt the interaction of proliferating endothelial cells to the matrix component, and thus drive endothelial cells to undergo apoptosis (Re, F. et al., 1994, J. Cell. Biol. 127:537-546). Matrix metalloproteinases (MMP's) have been implicated as key enzymes that regulate the formation of new blood vessels in tumors (Ray, J.M. et al., 1994, Eur. Respir. J. 7:2062-2072). Recently, it was demonstrated that an inhibitor of MMP-2 (PEX) can suppress tumor growth by inhibiting angiogenesis (Brooks, P.C. et al., 1998, Cell 92:391-400). Matin may function through inhibiting the activity of MMPs.

As used herein, the term "angiogenesis" means the generation of new blood vessels into a tissue or organ, and involves endothelial cell proliferation. Under normal physiological conditions, humans or animals undergo angiogenesis only in very specific restricted situations. For example, angiogenesis is normally observed in wound healing, fetal and embryonal development, and formation of the corpus luteum, endometrium and placenta. The term "endothelium" means a thin layer of flat epithelial cells that lines serous cavities, lymph vessels, and blood vessels. "Anti-angiogenic activity" therefore refers to the capability of a composition to inhibit the growth of blood vessels. The growth of blood vessels is a complex series of events, and includes localized breakdown of the basement membrane lying under the individual endothelial cells, proliferation of those cells, migration of the cells to the location of the future blood vessel, reorganization of the cells to form a new vessel membrane, cessation of endothelial cell proliferation, and, incorporation of pericytes and other cells that support the new blood vessel wall. "Anti-angiogenic activity" as used herein therefore includes interruption of any or all of these stages, with the end result that formation of new blood vessels is inhibited.

Anti-angiogenic activity may include endothelial inhibiting activity, which refers to the capability of a composition to inhibit angiogenesis in general and, for example, to inhibit the growth or migration of bovine capillary endothelial cells in culture in the presence of fibroblast growth factor, angiogenesis-associated factors, or other known growth factors. A "growth factor" is a composition that stimulates the growth, reproduction, or synthetic activity of cells. An "angiogenesis-associated factor" is a factor which either inhibits or promotes angiogenesis. An example of an angiogenesis-associated factor is an angiogenic growth factor, such as basic fibroblastic growth factor (bFGF), which is an angiogenesis promoter. Another example of an angiogenesis-associated factor is an angiogenesis inhibiting factor such as e.g., angiostatin (see, *e.g.,* U.S. Pat. No. 5,801,012, U.S. Pat. No. 5,837,682, U.S. Pat. No. 5,733,876, U.S. Pat. No. 5,776,704, U.S. Pat. No. 5,639,725, U.S. Pat. No. 5,792,845, WO 96/35774, WO 95/29242, WO 96/41194, WO 97/23500) or endostatin (see, *e.g.*, U.S. Pat. No. 5,854,205; U.S. Pat. No. 6,174,861; WO 97/15666).

By "substantially the same biological activity" or "substantially the same or superior biological activity" is meant that a composition has anti-angiogenic activity, and behaves similarly as does Matin, as determined in standard assays. "Standard assays" include, but are not limited to, those protocols used in the molecular biological arts to assess anti-angiogenic activity, cell cycle arrest, and apoptosis. Such assays include, but are not limited to, assays of endothelial cell proliferation, endothelial cell migration, cell cycle analysis, and endothelial cell tube formation, detection of apoptosis, *e.g.,* by apoptotic cell morphology or Annexin V-FITC assay, chorioallantoic membrane (CAM) assay, and inhibition of renal cancer tumor growth in nude mice. Such assays are provided in the Examples below, and also in U.S.S.N. 09/335,224,"Anti-Angiogenic Proteins and Methods of Use thereof," filed June 17, 1999, by Raghuram Kalluri, and in U.S.S.N. 09/479,118, "Anti-Angiogenic Proteins and Receptors and Methods of Use thereof," by Raghuram Kalluri, filed January 7, 2000, all of which are incorporated herein by reference in their entirety.

"Matin" is intended to include fragments, mutants, homologs, analogs, and allelic variants of the amino acid sequence of the Matin sequence, as well as Matin from other globular domains, globular domains from other α chains, other laminins, laminins from other mammals, and fragments, mutants, homologs, analogs and allelic variants of the Matin amino acid sequence.

It is to be understood that the present invention is contemplated to include any derivatives of Matin that have endothelial inhibitory activity (*e.g.*, the capability of a composition to inhibit angiogenesis in general and, for example, to inhibit the growth or migration of bovine capillary endothelial cells in culture in the presence of fibroblast growth factor, angiogenesis-associated factors, or other known growth factors). The present invention includes the entire Matin protein, derivatives of this protein and biologically-active fragments of this protein. This includes proteins with Matin activity that have amino acid substitutions or have sugars or other molecules attached to amino acid functional groups.

The invention also describes fragments, mutants, homologs and analogs of Matin. A "fragment" of a protein is defined herein as any amino acid sequence shorter than that protein, comprising at least 25 consecutive amino acids of the full polypeptide. Such a fragment may alternatively comprise 26, 27, 28, 29, 30, 31, 32, 33, 34, 35, 36, 37, 38, 39, 40, 41, 42, 43, 44, 45, 46, 47, 48, 49 or 50 consecutive amino acids of the full polypeptide. The fragment may comprise 51, 52, 53, 54, 55, 56, 57, 58, 59, 60, 61, 62, 63, 64, 65, 66, 67, 68, 69, 70, 71, 72, 73, 74 or 75 consecutive amino acids of the full polypeptide. Such molecules may or may not also comprise additional amino acids derived from the process of cloning, *e.g.,* amino acid residues or amino acid sequences corresponding to full or partial linker sequences. To be,encompassed by the present invention, such molecules, with or without such additional amino acid residues, must have substantially the same biological activity as the reference polypeptide.

Where the full-length molecule possesses more than one activity, *e.g.,* it may be possible to "split" the activities by splitting the full-length protein into several fragments, *e.g*., the full-length protein can be split into two fragments, one of which may possess one activity, while the other possesses another activity. The two fragments may or may not overlap, and the two activities may or may not be apparent in the full-length molecule. For instance, the full-length molecule may possess activity "A", and two fragments whereof may possess activities "A₁" and "A₂", respectively, or they may possess activities "B" and "C". Therefore, when it is stated that a fragment or mutant "must have substantially the same biological activity as the reference polypeptide", it is intended that in situations where one or more biological activities are split, the "reference polypeptide" is that subsequence of the overall molecule that corresponds to the fragment or mutant. That is, the fragment or mutant must have the substantially the same biological activity as that portion of the overall molecule to which they correspond.

By "mutant" of Matin is meant a polypeptide that includes any change in the amino acid sequence relative to the amino acid sequence of the equivalent reference Matin polypeptide. Such changes can arise either spontaneously or by manipulations by man, by chemical energy (*e.g*., X-ray), or by other forms of chemical mutagenesis, or by genetic engineering, or as a result of mating or other forms of exchange of genetic information. Mutations include, *e.g*., base changes, deletions, insertions, inversions, translocations, or duplications. Mutant forms of Matin may display either increased or decreased anti-angiogenic activity relative to the equivalent reference Matin polynucleotide, and such mutants may or may not also comprise additional amino acids derived from the process of cloning, *e.g*., amino acid residues or amino acid sequences corresponding to full or partial linker sequences. Mutants/fragments of the anti-angiogenic proteins of the present invention can also be generated,by PCR cloning, or by *Pseudomonas* elastase digestion, as described by Mariyama, M. et al. (1992, J. Biol. Chem. 267:1253-1258).

By "analog" of Matin is meant a non-natural molecule substantially similar to either the entire Matin molecule or a fragment or allelic variant thereof, and having substantially the same or superior biological activity. Such analogs are intended to include derivatives (*e.g.,* chemical derivatives, as defined above) of the biologically active Matin, as well as its fragments, mutants, homologs, and allelic variants, which derivatives exhibit a qualitatively similar agonist or antagonist effect to that of the unmodified Matin polypeptide, fragment, mutant, homolog, or allelic variant.

By "allele" of Matin is meant a polypeptide sequence containing a naturally-occurring sequence variation relative to the polypeptide sequence of the reference Matin polypeptide. By "allele" of a polynucleotide encoding the Matin polypeptide is meant a polynucleotide containing a sequence variation relative to the reference polynucleotide sequence encoding the reference Matin polypeptide, where the allele of the polynucleotide encoding the Matin polypeptide encodes an allelic form of the Matin polypeptide.

It is possible that a given polypeptide may be either a fragment, a mutant, an analog, or allelic variant of Matin, or it may be two or more of those things, e.g., a polypeptide may be both an analog and a mutant of the Matin polypeptide. For example, a shortened version of the Matin molecule (*e.g*., a fragment of Matin) may be created in the laboratory. If that fragment is then mutated through means known in the art, a molecule is created that is both a fragment and a mutant of Matin. In another example, a mutant may be created, which is later discovered to exist as an allelic form of Matin in some mammalian individuals. Such a mutant Matin molecule would therefore be both a mutant and an allelic variant. Such combinations of fragments, mutants, allelic variants, and analogs are intended to be encompassed in the present invention.

Encompassed by the present invention are proteins that have substantially the same amino acid sequence as Matin, or polynucleotides that have substantially the same nucleic acid sequence as the polynucleotides encoding Matin. "Substantially the same sequence" means a nucleic acid or polypeptide that exhibits at least about 70 % sequence identity with a reference sequence, *e.g.,* another nucleic acid or polypeptide, typically at least about 80% sequence identity with the reference sequence, preferably at least about 90% sequence identity, more preferably at least about 95% identity, and most preferably at least about 97% sequence identity with the reference sequence. The length of comparison for sequences will generally be at least 75 nucleotide bases or 25 amino acids, more preferably at least 150 nucleotide bases or 50 amino acids, and most preferably 243-264 nucleotide bases or 81-88 amino acids. "Polypeptide" as used herein indicates a molecular chain of amino acids and does not refer to a specific length of the product. Thus, peptides, oligopeptides and proteins are included within the definition of polypeptide. This term is also intended to include polypeptide that have been subjected to post-expression modifications such as, for example, glycosylations, acetylations, phosphorylations and the like.

"Sequence identity," as used herein, refers to the subunit sequence similarity between two polymeric molecules, *e.g*., two polynucleotides or two polypeptides. When a subunit position in both of the two molecules is occupied by the same monomeric subunit, *e.g*., if a position in each of two peptides is occupied by serine, then they are identical at that position. The identity between two sequences is a direct function of the number of matching or identical positions, *e.g*., if half *(e.g.,* 5 positions in a polymer 10 subunits in length) of the positions in two peptide or compound sequences are identical, then the two sequences are 50% identical; if 90% of the positions, *e.g*., 9 of 10 are matched, the two sequences share 90% sequence identity. By way of example, the amino acid sequences R₂R₅R₇R₁₀R₆R₃ and R₉R₈R₁R₁₀R₆R₃ have 3 of 6 positions in common, and therefore share 50% sequence identity, while the sequences R₂R₅R₇R₁₀R₆R₃ and R₈R₁R₁₀R₆R₃ have 3 of 5 positions in common, and therefore share 60% sequence identity. The identity between two sequences is a direct function of the number of matching or identical positions. Thus, if a portion of the reference sequence is deleted in a particular peptide, that deleted section is not counted for purposes of calculating sequence identity, e.g., R₂R₅R₇R₁₀R₆R₃ and R₂R₅R₇R₁₀R₃ have 5 out of 6 positions in common, and therefore share 83.3% sequence identity.

Identity is often measured using sequence analysis software *e.g*., BLASTN or BLASTP (available at http://www.ncbi.nlm.nih.gov/BLAST/). The default parameters for comparing two sequences *(e.g.,* "Blast"-ing two sequences against each other, http://www.ncbi.nlm.nih.gov/gorf/bl2.html) by BLASTN (for nucleotide sequences) are reward for match =1, penalty for mismatch = -2, open gap = 5, extension gap = 2. When using BLASTP for protein sequences, the default parameters are reward for match = 0, penalty for mismatch = 0, open gap = 11, and extension gap =1.

When two sequences share "sequence homology," it is meant that the two sequences differ from each other only by conservative substitutions. For polypeptide sequences, such conservative substitutions consist of substitution of one amino acid at a given position in the sequence for another amino acid of the same class (*e.g*., amino acids that share characteristics of hydrophobicity, charge, pK or other conformational or chemical properties, *e.g*., valine for leucine, arginine for lysine), or by one or more non-conservative amino acid substitutions, deletions, or insertions, located at positions of the sequence that do not alter the conformation or folding of the polypeptide to the extent that the biological activity of the polypeptide is destroyed. Examples of "conservative substitutions" include substitution of one non-polar (hydrophobic) residue such as isoleucine, valine, leucine or methionine for one another; the substitution of one polar (hydrophilic) residue for another such as between arginine and lysine, between glutamine and asparagine, between threonine and serine; the substitution of one basic residue such as lysine, arginine or histidine for one another; or the substitution of one acidic residue, such as aspartic acid or glutamic acid for one another; or the use of a chemically derivatized residue in place of a non-derivatized residue; provided that the polypeptide displays the requisite biological activity. Two sequences which share sequence homology may called "sequence homologs."

The invention contemplates mutants of the proteins and peptides disclosed herein, where the mutation(s) do not substantially alter the activity of the protein or peptide, that is the mutations are effectively "silent" mutations.

Homology, for polypeptides, is typically measured using sequence analysis software (*e.g*., Sequence Analysis Software Package of the Genetics Computer Group, University of Wisconsin Biotechnology Center, 1710 University Avenue, Madison, WI 53705). Protein analysis software matches similar sequences by assigning degrees of homology to various substitutions, deletions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

Also encompassed by the present invention are chemical derivatives of Matin. "Chemical derivative" refers to a subject polypeptide having one or more residues chemically derivatized by reaction of a functional side group. Such derivatized residues include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derlvatized to form salts, methyl and ethyl esters or other types of esters or hydrazides. Free hydroxyl groups may be derivatized to form O-acyl or O-alkyl derivatives. The imidazole nitrogen of histidine may be derivatized to form N-imbenzylhistidine. Also included as chemical derivatives are those peptides which contain one or more naturally occurring amino acid derivatives of the twenty standard amino acids. For examples: 4-hydroxyproline may be substituted for proline; 5-hydroxylysine may be substitute for lysine; 3-methylhistidine may be substituted for histidine; homoserine may be substituted for serine; and ornithine may be substituted for lysine.

The present invention also includes fusion proteins and chimeric proteins comprising the anti-angiogenic proteins, their fragments, mutants, homologs, analogs, and allelic variants. A fusion or chimeric protein can be produced as a result of recombinant expression and the cloning process, *e.g*., the protein may be produced comprising additional amino acids or amino acid sequences corresponding to full or partial linker sequences. A fusion or chimeric protein can consist of a multimer of a single protein, *e.g.,* repeats of the anti-angiogenic proteins, or the fusion and chimeric proteins can be made up of several proteins, *e.g*., several of the anti-angiogenic proteins. The fusion or chimeric protein can comprise a combination of two or more known anti-angiogenic proteins (*e.g*., angiostatin and endostatin, or biologically active fragments of angiostatin and endostatin), or an anti-angiogenic protein in combination with a targeting agent (*e.g*., endostatin with epidermal growth factor (EGF) or RGD peptides), or an anti-angiogenic protein in combination with an immunoglobulin molecule (*e.g*., endostatin and IgG, specifically with the Fc portion removed). The fusion and chimeric proteins can also include the anti-angiogenic proteins, their fragments, mutants, homologs, analogs, and allelic variants, and other anti-angiogenic proteins, *e.g.,* endostatin, or angiostatin. Other anti-angiogenic proteins can include Arresten, Canstatin or Tumstatin (PCT/US99/13737, the entire teachings of which are herein incorporated by reference), Vascostatin, restin and apomigren (PCT/LTS98/26058, the entire teachings of which are herein incorporated by reference) and fragments of endostatin (PCT/US98/26057, the entire teachings of which are herein incorporated by reference). The term "fusion protein" or "chimeric protein" as used herein can also encompass additional components for *e.g.,* delivering a chemotherapeutic agent, wherein a polynucleotide encoding the chemotherapeutic agent is linked to the polynucleotide encoding the anti-angiogenic protein. Fusion or chimeric proteins can also encompass multimers of an anti-angiogenic protein, *e.g*., a dimer or trimer. Such fusion or chimeric proteins can be linked together via post-translational modification (*e.g*., chemically linked), or the entire fusion protein may be made recombinantly.

Multimeric proteins comprising Matin, its fragments, mutants, homologs, analogs and allelic variants are also intended to be encompassed by the present invention. By "multimer" is meant a protein comprising two or more copies of a subunit protein. The subunit protein may be one of the proteins of the present invention, *e.g*., Matin repeated two or more times, or a fragment, mutant, homolog, analog or allelic variant, *e.g*., a Matin mutant or fragment, repeated two or more times. Such a multimer may also be a fusion or chimeric protein, *e.g*., a repeated tumstatin mutant may be combined with polylinker sequence, and/or one or more anti-angiogenic peptides, which may be present in a single copy, or may also be tandemly repeated, *e.g*., a protein may comprise two or more multimers within the overall protein.

The invention also encompasses a composition comprising one or more isolated polynucleotide(s) encoding Matin, as well as vectors and host cells containing such a polynucleotide, and processes for producing Matin and its fragments, mutants, homologs, analogs and allelic variants. The term "vector" as used herein means a carrier into which pieces of nucleic acid may be inserted or cloned, which carrier functions to transfer the pieces of nucleic acid into a host cell. Such a vector may also bring about the replication and/or expression of the transferred nucleic acid pieces. Examples of vectors include nucleic acid molecules derived, *e.g.*, from a plasmid, bacteriophage, or mammalian, plant or insect virus, or non-viral vectors such as ligand-nucleic acid conjugates, liposomes, or lipid-nucleic acid complexes. It may be desirable that the transferred nucleic molecule is operatively linked to an expression control sequence to form an expression vector capable of expressing the transferred nucleic acid. Such transfer of nucleic acids is generally called "transformation," and refers to the insertion of an exogenous polynucleotide into a host cell, irrespective of the method used for the insertion. For example, direct uptake, transduction or f-mating are included. The exogenous polynucleotide may be maintained as a non-integrated vector, for example, a plasmid, or alternatively, may be integrated into the host genome. "Operably linked" refers to a situation wherein the components described are in a relationship permitting them to function in their intended manner, *e.g*., a control sequence "operably linked" to a coding sequence is ligated in such a manner that expression of the coding sequence is achieved under conditions compatible with the control sequence. A "coding sequence" is a polynucleotide sequence which is transcribed into mRNA and translated into a polypeptide when placed under the control of (*e.g.*, operably linked to) appropriate regulatory sequences. The boundaries of the coding sequence are determined by a translation start codon at the 5'-terminus and a translation stop codon at the 3'-terminus. Such boundaries can be naturally-occurring, or can be introduced into or added the polynucleotide sequence by methods known in the art. A coding sequence can include, but is not limited to, mRNA, cDNA, and recombinant polynucleotide sequences.

The vector into which the cloned polynucleotide is cloned may be chosen because it functions in a prokaryotic, or alternatively, it is chosen because it functions in a eukaryotic organism. Two examples of vectors which allow for both the cloning of a polynucleotide encoding the Matin protein, and the expression of that protein from the polynucleotide, are the pET22b and pET28(a) vectors (Novagen, Madison, Wisconsin, USA) and a modified pPICZαA vector (InVitrogen, San Diego, California, USA), which allow expression of the protein in bacteria and yeast, respectively (see for example, WO 99/29878 and U.S.S.N. 09/589,483, the entire teachings which are hereby incorporated by reference).

Once a polynucleotide has been cloned into a suitable vector, it can be transformed into an appropriate host cell. By "host cell" is meant a cell which has been or can be used as the recipient of transferred nucleic acid by means of a vector. Host cells can prokaryotic or eukaryotic, mammalian, plant, or insect, and can exist as single cells, or as a collection, *e.g*., as a culture, or in a tissue culture, or in a tissue or an organism. Host cells can also be derived from normal or diseased tissue from a multicellular organism, *e.g.*, a mammal. Host cell, as used herein, is intended to include not only the original cell which was transformed with a nucleic acid, but also descendants of such a cell, which still contain the nucleic acid.

In one embodiment, the isolated polynucleotide encoding the anti-angiogenic protein additionally comprises a polynucleotide linker encoding a peptide. Such linkers are known to those of skill in the art and, for example the linker can comprise at least one additional codon encoding at least one additional amino acid. Typically the linker comprises one to about twenty or thirty amino acids. The polynucleotide linker is translated, as is the polynucleotide encoding the anti-angiogenic protein, resulting in the expression of an anti-angiogenic protein with at least one additional amino acid residue at the amino or carboxyl terminus of the anti-angiogenic protein. Importantly, the additional amino acid, or amino acids, do not compromise the activity of the anti-angiogenic protein.

After inserting the selected polynucleotide into the vector, the vector is transformed into an appropriate prokaryotic strain and the strain is cultured (*e.g.,* maintained) under suitable culture conditions for the production of the biologically active anti-angiogenic protein, thereby producing a biologically active anti-angiogenic protein, or mutant, derivative, fragment or fusion protein thereof. In one embodiment, the invention comprises cloning of a polynucleotide encoding an anti-angiogenic protein into the vectors pET22b, pET17b or pET28a, which are then transformed into bacteria. The bacterial host strain then expresses the anti-angiogenic protein. Typically the anti-angiogenic proteins are produced in quantities of about 10-20 milligrams, or more, per liter of culture fluid.

In another embodiment of the present invention, the eukaryotic vector comprises a modified yeast vector. One method is to use a pPICzα plasmid wherein the plasmid contains a multiple cloning site. The multiple cloning site has inserted into the multiple cloning site a His.Tag motif. Additionally the vector can be modified to add a *Nde*I site, or other suitable restriction sites. Such sites are well known to those of skill in the art. Anti-angiogenic proteins produced by this embodiment comprise a histidine tag motif (His.tag) comprising one, or more histidines, typically about 5-20 histidines. The tag must not interfere with the anti-angiogenic properties of the protein.

One method of producing Matin, for example, is to amplify the polynucleotide of SEQ ID NO:1 and clone it into an expression vector, *e.g*., pET22b, pET28(a), pPICZαA, or some other expression vector, transform the vector containing the polynucleotide into a host cell capable of expressing the polypeptide encoded by the polynucleotide, culturing the transformed host cell under culture conditions suitable for expressing the protein, and then extracting and purifying the protein from the culture. Exemplary methods of producing anti-angiogenic proteins in general, are provided in the Examples below and in U.S.S.N. 09/335, 224, "Anti-Angiogenic Proteins and Methods of Use Thereof," by Raghuram Kalluri, filed June 17, 1999. The Matin protein may also be expressed as a product of transgenic animals, *e.g.*, as a component of the milk of transgenic cows, goats, sheep or pigs, as is described in U.S. Pat. No. 5,962,648, or as a product of a transgenic plant, *e.g.,* combined or linked with starch molecules in maize, or as is described in U.S. Pat. No. 5,639,947 or 5,990,385.

Matin may also be produced by conventional, known methods of chemical synthesis. Methods for constructing the proteins of the present invention by synthetic means are known to those skilled in the art. The synthetically-constructed Matin protein sequence, by virtue of sharing primary, secondary or tertiary structural and/or conformational characteristics with e.g., recombinantly-produced Matin, may possess biological properties in common therewith, including biological activity. Thus, the synthetically-constructed Matin protein sequence may be employed as biologically active or immunological substitutes for *e.g.,* recombinantly-produced, purified Matin protein in screening of therapeutic compounds and in immunological processes for the development of antibodies.

The Matin protein is useful in inhibiting angiogenesis, as determined in standard assays, and provided in the Examples below.

Polynucleotides encoding Matin can be cloned out of isolated DNA or a cDNA library. Nucleic acids polypeptides, referred to herein as "isolated" are nucleic acids or polypeptides substantially free (*i.e.,* separated away from) the material of the biological source from which they were obtained (*e.g*., as exists in a mixture of nucleic acids or in cells), which may have undergone further processing. "Isolated" nucleic acids or polypeptides include nucleic acids or polypeptides obtained by methods described herein, similar methods, or other suitable methods, including essentially pure nucleic acids or polypeptides, nucleic acids or polypeptides produced by chemical synthesis, by combinations of chemical or biological methods, and recombinantly produced nucleic acids or polypeptides which are isolated. An isolated polypeptide therefore means one which is relatively free of other proteins, carbohydrates, lipids, and other cellular components with which it is normally associated. An isolated nucleic acid is not immediately contiguous with *(i.e.,* covalently linked to) both of the nucleic acids with which it is immediately contiguous in the naturally-occurring genome of the organism from which the nucleic acid is derived. The term, therefore, includes, for example, a nucleic acid which is incorporated into a vector (*e.g*., an autonomously replicating virus or plasmid), or a nucleic acid which exists as a separate molecule independent of other nucleic acids such as a nucleic acid fragment produced by chemical means or restriction endonuclease treatment.

The polynucleotides and proteins of the present invention can also be used to design probes to isolate other anti-angiogenic proteins. Exceptional methods are provided in U.S. Pat. No. 5,837,490, by Jacobs et al.*,* the entire teachings of which are herein incorporated by reference in its entirety. The design of the oligonucleotide probe should preferably follow these parameters: (a) it should be designed to an area of the sequence which has the fewest ambiguous bases ("N's"), if any, and (b) it should be designed to have a Tₘ of approx. 80°C (assuming 2°C for each A or T and 4 degrees for each G or C).

The oligonucleotide should preferably be labeled with g-³²P-ATP (specific activity 6000 Ci/mmole) and T4 polynucleotide kinase using commonly employed techniques for labeling oligonucleotides. Other labeling techniques can also be used. Unincorporated label should preferably be removed by gel filtration chromatography or other established methods. The amount of radioactivity incorporated into the probe should be quantitated by measurement in a scintillation counter. Preferably, specific activity of the resulting probe should be approximately 4 x 10⁶ dpm/pmole. The bacterial culture containing the pool of full-length clones should preferably be thawed and 100 µl of the stock used to inoculate a sterile culture flask containing 25 ml of sterile L-broth containing ampicillin at 100 µg/ml. The culture should preferably be grown to saturation at 37°C, and the saturated culture should preferably be diluted in fresh L-broth. Aliquots of these dilutions should preferably be plated to determine the dilution and volume which will yield approximately 5000 distinct and well-separated colonies on solid bacteriological media containing L-broth containing ampicillin at 100 µg/ml and agar at 1.5% in a 150 mm petri dish when grown overnight at 37°C. Other known methods of obtaining distinct, well-separated colonies can also be employed.

Standard colony hybridization procedures should then be used to transfer the colonies to nitrocellulose filters and lyse, denature and bake them. Highly stringent condition are those that are at least as stringent as, for example, 1x SSC at 65°C, or 1x SSC and 50% formamide at 42°C. Moderate stringency conditions are those that are at least as stringent as 4x SSC at 65°C, or 4x SSC and 50% formamide at 42°C. Reduced stringency conditions are those that are at least as stringent as 4x SSC at 50°C, or 6x SSC and 50% formamide at 40°C.

The filter is then preferably incubated at 65°C for 1 hour with gentle agitation in 6x SSC (20x stock is 175.3 g NaCl/liter, 88.2 g Na citrate/liter, adjusted to pH 7.0 with NaOH) containing 0.5% SDS, 100 µg/ml of yeast RNA, and 10 mM EDTA (approximately 10 mL per 150 min filter). Preferably, the probe is then added to the hybridization mix at a concentration greater than or equal to 1 x 10⁶ dpm/mL. The filter is then preferably incubated at 65°C with gentle agitation overnight. The filter is then preferably washed in 500 mL of 2x SSC/0.5% SDS at room temperature without agitation, preferably followed by 500 mL of 2x SSC/0.1% SDS at room temperature with gentle shaking for 15 minutes. A third wash with 0.1x SSC/0.5% SDS at 65°C for 30 minutes to 1 hour is optional. The filter is then preferably dried and subjected to autoradiography for sufficient time to visualize the positives on the X-ray film. Other known hybridization methods can also be employed. The positive colonies are then picked, grown in culture, and plasmid DNA isolated using standard procedures. The clones can then be verified by restriction analysis, hybridization analysis, or DNA sequencing.

Stringency conditions for hybridization refers to conditions of temperature and buffer composition which permit hybridization of a first nucleic acid sequence to a second nucleic acid sequence, wherein the conditions determine the degree of identity between those sequences which hybridize to each other. Therefore, "high stringency conditions" are those conditions wherein only nucleic acid sequences which are very similar to each other will hybridize. The sequences may be less similar to each other if they hybridize under moderate stringency conditions. Still less similarity is needed for two sequences to hybridize under low stringency conditions. By varying the hybridization conditions from a stringency level at which no hybridization occurs, to a level at which hybridization is first observed, conditions can be determined at which a given sequence will hybridize to those sequences that are most similar to it. The precise conditions determining the stringency of a particular hybridization include not only the ionic strength, temperature, and the concentration of destabilizing agents such as formamide, but also on factors such as the length of the nucleic acid sequences, their base composition, the percent of mismatched base pairs between the two sequences, and the frequency of occurrence of subsets of the sequences (*e.g.,* small stretches of repeats) within other non-identical sequences. Washing is the step in which conditions are set so as to determine a minimum level of similarity between the sequences hybridizing with each other. Generally, from the lowest temperature at which only homologous hybridization occurs, a 1% mismatch between two sequences results in a 1°C decrease in the melting temperature (Tₘ) for any chosen SSC concentration. Generally, a doubling of the concentration of SSC results in an increase in the T_{*m*} of about 17°C. Using these guidelines, the washing temperature can be determined empirically, depending on the level of mismatch sought. Hybridization and wash conditions are explained in Current Protocols in Molecular Biology (Ausubel, F.M. et al., eds., John Wiley & Sons, Inc., 1995, with supplemental updates) on pages 2.10.1 to 2.10.16, and 6.3.1 to 6.3.6.

High stringency conditions can employ hybridization at either (1) 1x SSC (10x SSC = 3 M NaCl, 0.3 M Na₃-citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 1x SSC, 50% formamide, 1% SDS, 0.1 - 2 mg/ml denatured salmon sperm DNA at 42°C, (3) 1% bovine serum albumen (fraction V), 1 mM Na₂·EDTA, 0.5 M NaHPO₄ (pH 7.2) (1 M NaHPO₄ = 134 g Na₂HPO₄·7H₂O, 4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with high stringency washes of either (1) 0.3 - O.lx SSC, 0.1% SDS at 65°C, or (2) 1 mM Na₂EDTA, 40 mM NaHPO₄ (pH 7.2), 1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations *(e.g.,* Na⁺), and "L" is the length of the hybrid in base pairs.

Moderate stringency conditions can employ hybridization at either (1) 4x SSC, (10x SSC = 3 M NaCl, 0.3 M Na₃citrate·2H₂O (88 g/liter), pH to 7.0 with 1 M HCl), 1% SDS (sodium dodecyl sulfate), 0.1 - 2 mg/ml denatured salmon sperm DNA at 65°C, (2) 4x SSC, 50% formamide, 1% SDS, 0.1 - 2 mg/ml denatured salmon sperm DNA at 42°C, (3) 1% bovine serum albumen (fraction V), 1 mM Na₂·EDTA, 0.5 M NaHPO₄ (pH 7.2) (1 M NaHPO₄ =134 g Na₂HPO₄·7H₂O,4 ml 85% H₃PO₄ per liter), 7% SDS, 0.1 - 2 mg/ml denatured salmon sperm DNA at 65°C, (4) 50% formamide, 5x SSC, 0.02 M Tris-HCl (pH 7.6), 1x Denhardt's solution (100x = 10 g Ficoll 400, 10 g polyvinylpyrrolidone, 10 g bovine serum albumin (fraction V), water to 500 ml), 10% dextran sulfate, 1% SDS, 0.1 - 2 mg/ml denatured salmon sperm DNA at 42°C, (5) 5x SSC, 5x Denhardt's solution, 1% SDS, 100 µg/ml denatured salmon sperm DNA at 65°C, or (6) 5x SSC, 5x Denhardt's solution, 50% formamide, 1% SDS, 100 µg/ml denatured salmon sperm DNA at 42°C, with moderate stringency washes of 1x SSC, 0.1% SDS at 65°C. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (*e.g.,* Na⁺), and "L" is the length of the hybrid in base pairs.

Low stringency conditions can employ hybridization at either (1) 4x SSC, (10x SSC = 3 M NaCl, 0.3 M Na₃-citxate·2H₂O (88 g/liter), pH to 7.0 with 1 M HCl), 1% SDS (sodium dodecyl sulfate), 0.1 - 2 mg/ml denatured salmon sperm DNA at 50°C, (2) 6x SSC, 50% formamide, 1% SDS, 0.1 - 2 mg/ml denatured salmon sperm DNA at 40°C, (3) 1% bovine serum albumen (fraction V), 1 mM Na₂·EDTA, 0.5 M NaHPO₄ (pH 7.2) (1 M NaHPO₄ = 134 g Na₂BPO₄·7H₂O, 4 ml 85% H₃PO₄ per liter), 7% SDS, 0.1 - 2 mg/ml denatured salmon sperm DNA at 50°C, (4) 50% formamide, 5x SSC, 0.02 M Tris-HCl (pH 7.6), 1x Denhardt's solution (100x =10 g Ficoll 400,10 g polyvinylpyrrolidone, 10 g bovine serum albumin (fraction V), water to 500 ml), 10% dextran sulfate, 1% SDS, 0.1 - 2 mg/ml denatured salmon sperm DNA at 40°C, (5) 5x SSC, 5x Denhardt's solution, 1% SDS, 100 µg/ml denatured salmon sperm DNA at 50°C, or (6) 5x SSC, 5x Denhardt's solution, 50% formamide, 1% SDS, 100 µg/ml denatured salmon sperm DNA at 40°C, with low stringency washes of either 2x SSC, 0.1 % SDS at 50°C, or (2) 0.5% bovine serum albumin (fraction V), 1 mM Na₂EDTA, 40 mM NaHPO₄ (pH 7.2), 5% SDS. The above conditions are intended to be used for DNA-DNA hybrids of 50 base pairs or longer. Where the hybrid is believed to be less than 18 base pairs in length, the hybridization and wash temperatures should be 5 - 10°C below that of the calculated Tₘ of the hybrid, where Tₘ in °C = (2 x the number of A and T bases) + (4 x the number of G and C bases). For hybrids believed to be about 18 to about 49 base pairs in length, the Tₘ in °C = (81.5°C + 16.6(log₁₀M) + 0.41(% G + C) - 0.61 (% formamide) - 500/L), where "M" is the molarity of monovalent cations (*e.g*., Na⁺), and "L" is the length of the hybrid in base pairs.

The present invention includes methods of inhibiting angiogenesis in mammalian tissue using Matin or its biologically-active fragments, analogs, homologs, derivatives or mutants. In particular, the present invention includes methods of treating an angiogenesis-mediated disease with an effective amount of one or more of the anti-angiogenic proteins, or one or more biologically active fragment thereof, or combinations of fragments that possess anti-angiogenic activity, or agonists and antagonists. An effective amount of anti-angiogenic protein is an amount sufficient to inhibit the angiogenesis which results in the disease or condition, thus completely, or partially, alleviating the disease or condition. Alleviation of the angiogenesis-mediated disease can be determined by observing an alleviation of symptoms of the disease, *e.g*., a reduction in the size of a tumor, or arrested tumor growth. As used herein, the term "effective amount" also means the total amount of each active component of the composition or method that is sufficient to show a meaningful patient benefit, *i.e.,* treatment, healing, prevention or amelioration of the relevant medical condition, or an increase in rate of treatment, healing, prevention or amelioration of such conditions. When applied to a combination, the term refers to combined amounts of the active ingredients that result in the therapeutic effect, whether administered in combination, serially or simultaneously. Angiogenesis-mediated diseases include, but are not limited to, cancers, solid tumors, blood-born tumors (*e.g.,* leukemias), tumor metastasis, benign tumors (*e.g*., hemangiomas, acoustic neuromas, neurofibromas, organ fibrosis, trachomas, and pyogenic granulomas), rheumatoid arthritis, psoriasis, ocular angiogenic diseases (*e.g.,* diabetic retinopathy, retinopathy of prematurity, macular degeneration, corneal graft rejection, neovascular glaucoma, retrolental fibroplasia, rubeosis), Osler-Webber Syndrome, myocardial angiogenesis, plaque neovascularization, telangiectasia, hemophiliac joints, angiofibroma, and wound granulation. The anti-angiogenic proteins are useful in the treatment of diseases of excessive or abnormal stimulation of endothelial cells. These diseases include, but are not limited to, intestinal adhesions, Crohn's disease, atherosclerosis, scleroderma, fibrosis and hypertrophic scars (*i.e.,* keloids). The anti-angiogenic proteins can be used as a birth control agent by preventing vascularization required for embryo implantation. The anti-angiogenic proteins are useful in the treatment of diseases that have angiogenesis as a pathologic consequence such as cat scratch disease (*Rochele minalia quintosa*) and ulcers (*Heliobacter pylori*). The anti-angiogenic proteins can also be used to prevent dialysis graft vascular access stenosis, and obesity, *e.g.,* by inhibiting capillary formation in adipose tissue, thereby preventing its expansion. The anti-angiogenic proteins can also be used to treat localized (*e.g*., nonmetastisized) diseases. "Cancer" means neoplastic growth, hyperplastic or proliferative growth or a pathological state of abnormal cellular development and includes solid tumors, non-solid tumors, and any abnormal cellular proliferation, such as that seen in leukemia. As used herein, "cancer" also means angiogenesis-dependent cancers and tumors, *i.e*., tumors that require for their growth (expansion in volume and/or mass) an increase in the number and density of the blood vessels supplying them with blood. "Regression" refers to the reduction of tumor mass and size as determined using methods well-known to those of skill in the art.

Alternatively, where an increase in angiogenesis is desired, e.g., in wound healing, or in post-infarct heart tissue, antibodies or antisera to the anti-angiogenic proteins can be used to block localized, native anti-angiogenic proteins and processes, and thereby increase formation of new blood vessels so as to inhibit atrophy of tissue.

The anti-angiogenic proteins may be used in combination with themselves, or other compositions and procedures for the treatment of diseases, *e.g.,* Matin and Vascostatin can be combined in a pharmaceutical composition, one or more of their fragments can be combined in a composition, or a tumor may be treated conventionally with surgery, radiation, chemotherapy, or immunotherapy, combined with the anti-angiogenic proteins and then the anti-angiogenic proteins may be subsequently administered to the patient to extend the dormancy of micrometastases and to stabilize and inhibit the growth of any residual primary tumor. The anti-angiogenic proteins, or fragments, antisera, receptor agonists, or receptor antagonists thereof, or combinations thereof can also be combined with other anti-angiogenic compounds, or proteins, fragments, antisera, receptor agonists, receptor antagonists of other anti-angiogenic proteins (*e.g.,* angiostatin, endostatin). Additionally, the auti-angiogenic proteins, or their fragments, antisera, receptor agonists, receptor antagonists, or combinations thereof, are combined with pharmaceutically acceptable excipients, and optionally sustained-release matrix, such as biodegradable polymers, to form therapeutic compositions. The compositions of the present invention mday also contain other anti-angiogenic proteins or chemical compounds, such as endostatin or angiostatin, and mutants, fragments, and analogs thereof. The compositions may further contain other agents which either enhance the activity of the protein or compliment its activity or use in treatment, such as chemotherapeutic or radioactive agents. Such additional factors and/or agents may be included in the composition to produce a synergistic effect with protein of the invention, or to minimize side effects. Additionally, administration of the composition of the present invention may be administered concurrently with other therapies, *e.g*., administered in conjunction with a chemotherapy or radiation therapy regimen.

The invention includes methods for inhibiting angiogenesis in mammalian (*e.g.,* human) tissues by contacting the tissue with a composition comprising the proteins of the invention. By "contacting" is meant not only topical application, but also those modes of delivery that introduce the composition into the tissues, or into the cells of the tissues.

Use of timed release or sustained release delivery systems are also included in the invention. Such systems are highly desirable in situations where surgery is difficult or impossible, *e.g*., patients debilitated by age or the disease course itself, or where the risk-benefit analysis dictates control over cure.

A sustained-release matrix, as used herein, is a matrix made of materials, usually polymers, which are degradable by enzymatic or acid/base hydrolysis or by dissolution. Once inserted into the body, the matrix is acted upon by enzymes and body fluids. The sustained-release matrix desirably is chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polyproteins, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

The angiogenesis-modulating composition of the present invention maybe a solid, liquid or aerosol and may be administered by any known route of administration. Examples of solid compositions include pills, creams, and implantable dosage units. The pills may be administered orally, the therapeutic creams may be administered topically. The implantable dosage unit may be administered locally, for example at a tumor site, or which may be implanted for systemic release of the angiogenesis-modulating composition, for example subcutaneously. Examples of liquid composition include formulations adapted for injection subcutaneously, intravenously, intraarterially, and formulations for topical and intraocular administration. Examples of aerosol formulation include inhaler formulation for administration to the lungs.

The proteins and protein fragments with the anti-angiogenic activity described above can be provided as isolated and substantially purified proteins and protein fragments in pharmaceutically acceptable formulations using formulation methods known to those of ordinary skill in the art. These formulations can be administered by standard routes. In general, the combinations may be administered by the topical, transdermal, intraperitoneal, intracranial, intracerebroventricular, intracerebral, intravaginal, intrauterine, oral, rectal or parenteral (*e.g*., intravenous, intraspinal, subcutaneous or intramuscular) route. In addition, the anti-angiogenic proteins may be incorporated into biodegradable polymers allowing for sustained release of the compound, the polymers being implanted in the vicinity of where drug delivery is desired, for example, at the site of a tumor or implanted so that the anti-angiogenic proteins are slowly released systemically. Osmotic minipumps may also be used to provide controlled delivery of high concentrations of the anti-angiogenic proteins through cannulae to the site of interest, such as directly into a metastatic growth or into the vascular supply to that tumor. The biodegradable polymers and their use are described, for example, in detail in Brem et al. (1991, J. Neurosurg. 74:441-6), which is hereby incorporated by reference in its entirety.

The compositions containing a polypeptide of this invention can be administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; *i.e.,* carrier or vehicle.

Modes of administration of the compositions of the present inventions include intravenous, intramuscular, intraperitoneal, intrasternal, subcutaneous and intraarticular injection and infusion. Pharmaceutical compositions for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyois (*e.g*., glycerol, propylene glycol, polyethylene glycol and the like), carboxymethylcellulose and suitable mixtures thereof, vegetable oils (*e.g*., olive oil) and injectable organic esters such as ethyl oleate. Proper fluidity may be maintained, for example, by the use of coating materials such as lecithin, by the maintenance of the required particle size in the case of dispersions and by the use of surfactants. These compositions may also contain adjuvants such as preservatives, wetting agents, emulsifying agents and dispersing agents. Prevention of the action of microorganisms may be ensured by the inclusion of various antibacterial and antifungal agents such as paraben, chlorobutanol, phenol sorbic acid and the like. It may also be desirable to include isotonic agents such as sugars, sodium chloride and the like. Prolonged absorption of the injectable pharmaceutical form may be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polmer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter or by incorporating sterilizing agents in the form of sterile solid compositions which can be dissolved or dispersed in sterile water or other sterile injectable media just prior to use.

The therapeutic compositions of the present invention can include pharmaceutically acceptable salts of the components therein, *e.g*., which may be derived from inorganic or organic acids. By "pharmaceutically acceptable salt" is meant those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge, et al. describe pharmaceutically acceptable salts in detail in J. Pharmaceutical Sciences (1977) 66:1 *et seq.,* which is incorporated herein by reference. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. The salts may be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsufonate, digluconate, glycerophosphate, hemisulfate, heptonoate, hexanoate, fumarate, hydrochloride, hydrobromide, hydroiodide, 2-hydroxymethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl, and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

As used herein, the terms "pharmaceutically acceptable," "physiologically tolerable" and grammatical variations thereof as they refer to compositions, carriers, diluents and reagents, are used interchangeably and represent that the materials are capable of administration to or upon a mammal with a minimum of undesirable physiological effects such as nausea, dizziness, gastric upset and the like. The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients include, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The anti-angiogenic proteins of the present invention can also be included in a composition comprising a prodrug. As used herein, the term "prodrug" refers to compounds which are rapidly transformed *in vivo* to yield the parent compound, for example, by enzymatic hydrolysis in blood. A thorough discussion is provided in T. Higuchi and V. Stella, Prodrugs as Novel Delivery Systems, Vol. 14 of the ACS Symposium Series and in Edward B. Roche, ed., Bioreversible Carriers in Drug Design, American Pharmaceutical Association and Permagon Press, 1987, both of which are incorporated herein by reference. As used herein, the term "pharmaceutically acceptable prodrug" refers to (1) those prodrugs of the compounds of the present invention which are, within the scope of sound medical judgement, suitable for use in contact with the tissues of humans and animals without undue toxicity, irritation, allergic response and the like, commensurate with a suitable benefit-to-risk ratio and effective for their intended use and (2) zwitterionic forms, where possible, of the parent compound.

The dosage of the anti-angiogenic proteins of the present invention will depend on the disease state or condition being treated and other clinical factors such as weight and condition of the human or animal and the route of administration of the compound. Depending upon the half-life of the anti-angiogenic proteins in the particular animal or human, the anti-angiogenic proteins can be administered between several times per day to once a week. It is to be understood that the present invention has application for both human and veterinary use. The methods of the present invention contemplate single as well as multiple administrations, given either simultaneously or over an extended period of time. In addition, the anti-angiogenic proteins can be administered in conjunction with other forms of therapy, e.g., chemotherapy, radiotherapy, or immunotherapy.

The anti-angiogenic protein formulations include those suitable for oral, rectal, ophthalmic (including intravitreal or intracameral), nasal, topical (including buccal and sublingual), intrauterine, vaginal or parenteral (including subcutaneous, intraperitoneal, intramuscular, intravenous, intradermal, intracranial, intratracheal, and epidural) administration. The anti-angiogenic protein formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into association the active ingredient with liquid carriers or fmely divided solid carriers or both, and then, if necessary, shaping the product.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

When an effective amount of protein of the present invention is administered orally, the anti-angiogenic proteins of the present invention will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% protein of the present invention, and preferably from about 25 to 90% protein of the present invention. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of protein of the present invention, and preferably from about 1 to 50% protein of the present invention.

When an effective amount of protein of the present invention is administered by intravenous, cutaneous or subcutaneous injection, protein of the present invention will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition to protein of the present invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art.

The amount of protein of the present invention in the pharmaceutical composition of the present invention will depend upon the nature and severity of the condition being treated, and on the nature of prior treatments which the patient has undergone. Ultimately, the attending physician will decide the amount of protein of the present invention with which to treat each individual patient. Initially, the attending physician will administer low doses of protein of the present invention and observe the patient's response. Larger doses of protein of the present invention may be administered until the optimal therapeutic effect is obtained for the patient, and at that point the dosage is not increased further.

The duration of intravenous therapy using the pharmaceutical composition of the present invention will vary, depending on the severity of the disease being treated and the condition and potential idiosyncratic response of each individual patient. It is contemplated that the duration of each application of the protein of the present invention will be in the range of 12 to 24 hours of continuous intravenous administration. Ultimately the attending physician will decide on the appropriate duration of intravenous therapy using the pharmaceutical composition of the present invention.

Preferred unit dosage formulations are those containing a daily dose or unit, daily sub-dose, or an appropriate fraction thereof, of the administered ingredient. It should be understood that in addition to the ingredients, particularly mentioned above, the formulations of the present invention may include other agents conventional in the art having regard to the type of formulation in question. Optionally, cytotoxic agents may be incorporated or otherwise combined with the anti-angiogenic proteins, or biologically functional protein fragements thereof, to provide dual therapy to the patient.

The therapeutic compositions are also presently valuable for veterinary applications. Particularly domestic animals and thoroughbred horses, in addition to humans, are desired patients for such treatment with proteins of the present invention.

Cytotoxic agents such as ricin, can be linked to the anti-angiogenic proteins, and fragments thereof, thereby providing a tool for destruction of cells that bind the anti-angiogenic proteins. These cells may be found in many locations, including but not limited to, micrometastases and primary tumors. Proteins linked to cytotoxic agents are infused in a manner designed to maximize delivery to the desired location. For example, ricin-linked high affinity fragments are delivered through a cannula into vessels supplying the target site or directly into the target. Such agents are also delivered in a controlled manner through osmotic pumps coupled to infusion cannulae. A combination of antagonists to the anti-angiogenic proteins may be co-applied with stimulators of angiogenesis to increase vascularization of tissue. This therapeutic regimen provides an effective means of destroying metastatic cancer.

Additional treatment methods include administration of the anti-angiogenic proteins, fragments, analogs, antisera, or receptor agonists and antagonists thereof, linked to cytotoxic agents. It is to be understood that the anti-angiogenic proteins can be human or animal in origin. The anti-angiogenic proteins can also be produced synthetically by chemical reaction or by recombinant techniques in conjunction with expression systems. The anti-angiogenic proteins can also be produced by enzymatically cleaving isolated laminin to generate proteins having auti-angiogenic activity. The anti-angiogenic proteins may also be produced by compounds that mimic the action of endogenous enzymes that cleave laminin to the anti-angiogenic proteins. Production of the anti-angiogenic proteins may also be modulated by compounds that affect the activity of cleavage enzymes.

The present invention also encompasses gene therapy whereby a polynucleotide encoding the anti-angiogenic proteins, integrins, integrin subunits, or a mutant, fragment, or fusion protein thereof, is introduced and regulated in a patient. Various methods of transferring or delivering DNA to cells for expression of the gene product protein, otherwise referred to as gene therapy, are disclosed in Gene Transfer into Mammalian Somatic Cells in vivo, N. Yang (1992) Crit. Rev. Biotechn. 12(4):335-56, which is hereby incorporated by reference. Gene therapy encompasses incorporation of DNA sequences into somatic cells or germ line cells for use in either ex vivo or in vivo therapy. Gene therapy functions to replace genes, augment normal or abnormal gene function, and to combat infectious diseases and other pathologies.

Strategies for treating these medical problems with gene therapy include therapeutic strategies such as identifying the defective gene and then adding a functional gene to either replace the function of the defective gene or to augment a slightly functional gene; or prophylactic strategies, such as adding a gene for the product protein that will treat the condition or that will make the tissue or organ more susceptible to a treatment regimen. As an example of a prophylactic strategy, a gene such as that encoding one or more of the anti-angiogenic proteins maybe placed in a patient and thus prevent occurrence of angiogenesis; or a gene that makes tumor cells more susceptible to radiation could be inserted and then radiation of the tumor would cause increased killing of the tumor cells.

Many protocols for transfer of the DNA or regulatory sequences of the anti-angiogenic proteins are envisioned in this invention. Transfection of promoter sequences, other than one normally found specifically associated with the anti-angiogenic proteins, or other sequences which would increase production of the anti-angiogenic proteins are also envisioned as methods of gene therapy. An example of this technology is found in Transkaryotic Therapies, Inc., of Cambridge, Mass., using homologous recombination to insert a "genetic switch" that turns on an erythropoietin gene in cells. See Genetic Engineering News, Apr. 15, 1994. Such "genetic switches" could be used to activate the anti-angiogenic proteins (or their receptors) in cells not normally expressing those proteins (or receptors).

Gene transfer methods for gene therapy fall into three broad categories: physical (*e.g*., electroporation, direct gene transfer and particle bombardment), chemical (*e.g.,* lipid-based carriers, or other non-viral vectors) and biological (*e.g.,* virus-derived vector and receptor uptake). For example, non-viral vectors may be used which include liposomes coated with DNA. Such liposome/DNA complexes may be directly injected intravenously into the patient. It is believed that the liposome/DNA complexes are concentrated in the liver where they deliver the DNA to macrophages and Kupffer cells. These cells are long lived and thus provide long term expression of the delivered DNA. Additionally, vectors or the "naked" DNA of the gene may be directly injected into the desired organ, tissue or tumor for targeted delivery of the therapeutic DNA.

Gene therapy methodologies can also be described by delivery site. Fundamental ways to deliver genes include *ex vivo* gene transfer, *in vivo* gene transfer, and *in vitro* gene transfer. In *ex vivo* gene transfer, cells are taken from the patient and grown in cell culture. The DNA is transfected into the cells, the transfected cells are expanded in number and then reimplanted in the patient. In *in vitro* gene transfer, the transformed cells are cells growing in culture, such as tissue culture cells, and not particular cells from a particular patient. These "laboratory cells" are transfected, the transfected cells are selected and expanded for either implantation into a patient or for other uses.

*In vivo* gene transfer involves introducing the DNA into the cells of the patient when the cells are within the patient. Methods include using virally mediated gene transfer using a noninfectious virus to deliver the gene in the patient or injecting naked DNA into a site in the patient and the DNA is taken up by a percentage of cells in which the gene product protein is expressed. Additionally, the other methods described herein, such as use of a "gene gun," may be used for in vitro insertion of the DNA or regulatory sequences controlling production of the anti-angiogenic proteins.

Chemical methods of gene therapy may involve a lipid based compound, not necessarily a liposome, to transfer the DNA across the cell membrane. Lipofectins or cytofectins, lipid-based positive ions that bind to negatively charged DNA, make a complex that can cross the cell membrane and provide the DNA into the interior of the cell. Another chemical method uses receptor-based endocytosis, which involves binding a specific ligand to a cell surface receptor and enveloping and transporting it across the cell membrane. The ligand binds to the DNA and the whole complex is transported into the cell. The ligand gene complex is injected into the blood stream and then target cells that have the receptor will specifically bind the ligand and transport the ligand-DNA complex into the cell.

Many gene therapy methodologies employ viral vectors to insert genes into cells. For example, altered retrovirus vectors have been used in *ex vivo* methods to introduce genes into peripheral and tumor-infiltrating lymphocytes, hepatocytes, epidermal cells, myocytes, or other somatic cells. These altered cells are then introduced into the patient to provide the gene product from the inserted DNA.

Viral vectors have also been used to insert genes into cells using in vivo protocols. To direct the tissue-specific expression of foreign genes, cis-acting regulatory elements or promoters that are known to be tissue-specific can be used. Alternatively, this can be achieved using *in situ* delivery of DNA or viral vectors to specific anatomical sites *in vivo.* For example, gene transfer to blood vessels *in vivo* was achieved by implanting *in vitro* transduced endothelial cells in chosen sites on arterial walls. The virus infected surrounding cells which also expressed the gene product. A viral vector can be delivered directly to the *in vivo* site, by a catheter for example, thus allowing only certain areas to be infected by the virus, and providing long-term, site specific gene expression. *In vivo* gene transfer using retrovirus vectors has also been demonstrated in mammary tissue and hepatic tissue by injection of the altered virus into blood vessels leading to the organs.

Viral vectors that have been used for gene therapy protocols include but are not limited to, retroviruses, other RNA viruses such as poliovirus or Sindbis virus, adenovirus, adeno-associated virus, herpes viruses, SV 40, vaccinia and other DNA viruses. Replication-defective murine retroviral vectors are the most widely utilized gene transfer vectors. Murine leukemia retroviruses are composed of a single strand RNA complexed with a nuclear core protein and polymerase (pol) enzymes, encased by a protein core (gag) and surrounded by a glycoprotein envelope (env) that determines host range. The genomic structure of retroviruses include the *gag, pol,* and *env* genes enclosed at by the 5' and 3' long terminal repeats (LTR). Retroviral vector systems exploit the fact that a minimal vector containing the 5' and 3' LTRs and the packaging signal are sufficient to allow vector packaging, infection and integration into target cells providing that the viral structural proteins are supplied in trans in the packaging cell line. Fundamental advantages of retroviral vectors for gene transfer include efficient infection and gene expression in most cell types, precise single copy vector integration into target cell chromosomal DNA, and ease of manipulation of the retroviral genome.

The adenovirus is composed of linear, double stranded DNA complexed with core proteins and surrounded with capsid proteins. Advances in molecular virology have led to the ability to exploit the biology of these organisms to create vectors capable of transducing novel genetic sequences into target cells *in vivo.* Adenoviral-based vectors will express gene product proteins at high levels. Adenoviral vectors have high efficiencies of infectivity, even with low titers of virus. Additionally, the virus is fully infective as a cell free virion so injection of producer cell lines is not necessary. Another potential advantage to adenoviral vectors is the ability to achieve long term expression of heterologous genes *in vivo.*

Mechanical methods of DNA delivery include fusogenic lipid vesicles such as liposomes or other vesicles for membrane fusion, lipid particles of DNA incorporating cationic lipid such as lipofectin, polylysine-mediated transfer of DNA, direct injection of DNA, such as microinjection of DNA into germ or somatic cells, pneumatically delivered DNA-coated particles, such as the gold particles used in a "gene gun," and inorganic chemical approaches such as calcium phosphate transfection. Particle-mediated gene transfer methods were first used in transforming plant tissue. With a particle bombardment device, or "gene gun," a motive force is generated to accelerate DNA-coated high density particles (such as gold or tungsten) to a high velocity that allows penetration of the target organs, tissues or cells. Particle bombardment can be used in *in vitro* systems, or with *ex vivo* or *in vivo* techniques to introduce DNA into cells, tissues or organs. Another method, ligand-mediated gene therapy, involves complexing the DNA with specific ligands to form ligand-DNA conjugates, to direct the DNA to a specific cell or tissue.

It has been found that injecting plasmid DNA into muscle cells yields high percentage of the cells which are transfected and have sustained expression of marker genes. The DNA of the plasmid may or may not integrate into the genome of the cells. Non-integration of the transfected DNA would allow the transfection and expression of gene product proteins in terminally differentiated, non-proliferative tissues for a prolonged period of time without fear of mutational insertions, deletions, or alterations in the cellular or mitochondrial genome. Long-term, but not necessarily permanent, transfer of therapeutic genes into specific cells may provide treatments for genetic diseases or for prophylactic use. The DNA could be reinjected periodically to maintain the gene product level without mutations occurring in the genomes of the recipient cells. Non-integration of exogenous DNAs may allow for the presence of several different exogenous DNA constructs within one cell with all of the constructs expressing various gene products.

Electroporation for gene transfer uses an electrical current to make cells or tissues susceptible to electroporation-mediated mediated gene transfer. A brief electric impulse with a given field strength is used to increase the permeability of a membrane in such a way that DNA molecules can penetrate into the cells. This technique can be used in *in vitro* systems, or with *ex vivo* or *in vivo* techniques to introduce DNA into cells, tissues or organs.

Carrier mediated gene transfer *in vivo* can be used to transfect foreign DNA into cells. The carner-DNA complex can be conveniently introduced into body fluids or the bloodstream and then site-specifically directed to the target organ or tissue in the body. Both liposomes and polycations, such as polylysine, lipofectins or cytofectins, can be used. Liposomes can be developed which are cell specific or organ specific and thus the foreign DNA carried by the liposome will be taken up by target cells. Injection of immunoliposomes that are targeted to a specific receptor on certain cells can be used as a convenient method of inserting the DNA into the cells bearing the receptor. Another carrier system that has been used is the asialoglycoportein/polylysine conjugate system for carrying DNA to hepatocytes for *in vivo* gene transfer.

The transfected DNA may also be complexed with other kinds of carriers so that the DNA is carried to the recipient cell and then resides in the cytoplasm or in the nucleoplasm. DNA can be coupled to carrier nuclear proteins in specifically engineered vesicle complexes and carried directly into the nucleus.

Gene regulation of the anti-angiogenic proteins may be accomplished by administering compounds that bind to the gene encoding one of the anti-angiogenic proteins, or control regions associated with the gene, or its corresponding RNA transcript to modify the rate of transcription or translation. Additionally, cells transfected with a DNA sequence encoding the anti-angiogenic proteins may be administered to a patient to provide an *in vivo* source of those proteins. For example, cells may be transfected with a vector containing a nucleic acid sequence encoding the anti-angiogenic proteins. The transfected cells may be cells derived from the patient's normal tissue, the patient's diseased tissue, or may be non-patient cells.

For example, tumor cells removed from a patient can be transfected with a vector capable of expressing the proteins of the present invention, and re-introduced into the patient. The transfected tumor cells produce levels of the protein in the patient that inhibit the growth of the tumor. Patients may be human or non-human animals. Cells may also be transfected by non-vector, or physical or chemical methods known in the art such as electroporation, ionoporation, or via a "gene gun." Additionally, the DNA may be directly injected, without the aid of a carrier, into a patient. In particular, the DNA may be injected into skin, muscle or blood.

The gene therapy protocol for transfecting the anti-angiogenic proteins into a patient may either be through integration of the anti-angiogenic protein DNA into the genome of the cells, into minichromosomes or as a separate replicating or non-replicating DNA construct in the cytoplasm or nucleoplasm of the cell. Expression of the anti-angiogenic proteins may continue for a long-period of time or may be reinjected periodically to maintain a desired level of the protein(s) in the cell, the tissue or organ or a determined blood level.

In addition, the invention encompasses antibodies and antisera, which can be used for testing of novel anti-angiogenic proteins, and can also be used in diagnosis, prognosis, or treatment of diseases and conditions characterized by, or associated with, angiogenic activity or lack thereof. Such antibodies and antisera can also be used to up-regulate angiogenesis where desired, *e.g*., in post-infarct heart tissue, antibodies or antisera to the proteins of the invention can be used to block localized, native anti-angiogenic proteins and processes, and increase formation of new blood vessels and inhibit atrophy of heart tissue.

Such antibodies and antisera can be combined with pharmaceutically-acceptable compositions and carriers to form diagnostic, prognostic or therapeutic compositions. The term "antibody" or "antibody molecule" refers to a population of immunoglobulin molecules and/or immunologically active portions of immunoglobulin molecules, *i.e.,* molecules that contain an antibody combining site or paratope.

Passive antibody therapy using antibodies that specifically bind the anti-angiogenic proteins can be employed to modulate angiogenic-dependent processes such as reproduction, development, and wound healing and tissue repair. In addition, antisera directed to the Fab regions of antibodies of the anti-angiogenic proteins can be administered to block the ability of endogenous antisera to the proteins to bind the proteins.

The the anti-angiogenic proteins of the present invention also can be used to generate antibodies that are specific for the inhibitor(s) and receptor(s). The antibodies can be either polyclonal antibodies or monoclonal antibodies. These antibodies that specifically bind to the anti-angiogenic proteins or their receptors can be used in diagnostic methods and kits that are well known to those of ordinary skill in the art to detect or quantify the anti-angiogenic proteins or their receptors in a body fluid or tissue. Results from these tests can be used to diagnose or predict the occurrence or recurrence of a cancer and other angiogenic mediated diseases.

The invention also includes use of the anti-angiogenic proteins, antibodies to those proteins, and compositions comprising those proteins and/or their antibodies in diagnosis or prognosis of diseases characterized by angiogenic activity. As used herein, the term "prognostic method" means a method that enables a prediction regarding the progression of a disease of a human or animal diagnosed with the disease, in particular, an angiogenesis dependent disease. The term "diagnostic method" as used herein means a method that enables a determination of the presence or type of angiogenesis-dependent disease in or on a human or animal.

The the anti-angiogenic proteins can be used in a diagnostic method and kit to detect and quantify antibodies capable of binding the proteins. These kits would permit detection of circulating antibodies to the anti-angiogenic proteins which indicates the spread of micrometastases in the presence of the anti-angiogenic proteins secreted by primary tumors *in situ.* Patients that have such circulating anti-protein antibodies may be more likely to develop multiple tumors and cancers, and may be more likely to have recurrences of cancer after treatments or periods of remission. The Fab fragments of these anti-protein antibodies may be used as antigens to generate anti-protein Fab-fragment antisera which can be used to neutralize anti-protein antibodies. Such a method would reduce the removal of circulating protein by anti-protein antibodies, thereby effectively elevating circulating levels of the anti-angiogenic proteins.

The present invention also includes isolation of receptors specific for the anti-angiogenic proteins. Protein fragments that possess high affinity binding to tissues can be used to isolate the receptor of the anti-angiogenic proteins on affinity columns. Isolation and purification of the receptor(s) is a fundamental step towards elucidating the mechanism of action of the anti-angiogenic proteins. Isolation of a receptor and identification of agonists and antagonists will facilitate development of drugs to modulate the activity of the receptor, the final pathway to biological activity. Isolation of the receptor enables the construction of nucleotide probes to monitor the location and synthesis of the receptor, using *in situ* and solution hybridization technology. Further, the gene for the receptor can be isolated, incorporated into an expression vector and transfected into cells, such as patient tumor cells to increase the ability of a cell type, tissue or tumor to bind the anti-angiogenic proteins and inhibit local angiogenesis.

The anti-angiogenic proteins are employed to develop affinity columns for isolation of the receptor(s) for the anti-angiogenic proteins from cultured tumor cells. Isolation and purification of the receptor is followed by amino acid sequencing. Using this information the gene or genes coding for the receptor can be identified and isolated. Next, cloned nucleic acid sequences are developed for insertion into vectors capable of expressing the receptor. These techniques are well known to those skilled in the art. Transfection of the nucleic acid sequence(s) coding for the receptor into tumor cells, and expression of the receptor by the transfected tumor cells enhances the responsiveness of these cells to endogenous or exogenous anti-angiogenic proteins and thereby decreasing the rate of metastatic growth.

Angiogenesis-inhibiting proteins of the present invention can be synthesized in a standard microchemical facility and purity checked with HPLC and mass spectrophotometry. Methods of protein synthesis, HPLC purification and mass spectrophotometry are commonly known to those skilled in these arts. The anti-angiogenic proteins and their receptors proteins are also produced in recombinant *E. coli* or yeast expression systems, and purified with column chromatography.

Different protein fragments of the intact the anti-angiogenic proteins can be synthesized for use in several applications including, but not limited to the following; as antigens for the development of specific antisera, as agonists and antagonists active at binding sites of the anti-angiogenic proteins, as proteins to be linked to, or used in combination with, cytotoxic agents for targeted killing of cells that bind the anti-angiogenic proteins.

The synthetic protein fragments of the anti-angiogenic proteins have a variety of uses. The protein that binds to the receptor(s) of the anti-angiogenic proteins with high specificity and avidity is radiolabeled and employed for visualization and quantitation of binding sites using autoradiographic and membrane binding techniques. This application provides important diagnostic and research tools. Knowledge of the binding properties of the receptor(s) facilitates investigation of the transduction mechanisms linked to the receptor(s).

The anti-angiogenic proteins and proteins derived from them can be coupled to other molecules using standard methods. The amino and carboxyl termini of the anti-angiogenic proteins both contain tyrosine and lysine residues and are isotopically and nonisotopically labeled with many techniques, for example radiolabeling using conventional techniques (tyrosine residues-chloramine T, iodogen, lactoperoxidase; lysine residues-Bolton-Hunter reagent). These coupling techniques are well known to those skilled in the art. Alternatively, tyrosine or lysine is added to fragments that do not have these residues to facilitate labeling of reactive amino and hydroxyl groups on the protein. The coupling technique is chosen on the basis of the functional groups available on the amino acids including, but not limited to amino, sulfhydral, carboxyl, amide, phenol, and imidazole. Various reagents used to effect these couplings include among others, glutaraldehyde, diazotized benzidine, carbodiimide, and p-benzoquinone.

The anti-angiogenic proteins are chemically coupled to isotopes, enzymes, carrier proteins, cytotoxic agents, fluorescent molecules, chemiluminescent, bioluminescent and other compounds for a variety of applications. The efficiency of the coupling reaction is determined using different techniques appropriate for the specific reaction. For example, radiolabeling of a protein of the present invention with ¹²⁵I is accomplished using chloramine T and Na¹²⁵I of high specific activity. The reaction is terminated with sodium metabisulfite and the mixture is desalted on disposable columns. The labeled protein is eluted from the column and fractions are collected. Aliquots are removed from each fraction and radioactivity measured in a gamma counter. In this manner, the unreacted Na¹²⁵I is separated from the labeled protein. The protein fractions with the highest specific radioactivity are stored for subsequent use such as analysis of the ability to bind to antisera of the anti-angiogenic proteins.

In addition, labeling the anti-angiogenic proteins with short lived isotopes enables visualization of receptor binding sites *in vivo* using positron emission tomography or other modem radiographic techniques to locate tumors with the proteins' binding sites.

Systematic substitution of amino acids within these synthesized proteins yields high affinity protein agonists and antagonists to the receptor(s) of the anti-angiogenic proteins that enhance or diminish binding to the receptor(s). Such agonists are used to suppress the growth ofmicrometastases, thereby limiting the spread of cancer. Antagonists to the anti-angiogenic proteins are applied in situations of inadequate vascularization, to block the inhibitory effects of the anti-angiogenic proteins and promote angiogenesis. For example, this treatment may have therapeutic effects to promote wound healing in diabetics.

The invention is further illustrated by the following examples, which are not meant to be construed in any way as imposing limitations upon the scope thereof. On the contrary, it is to be clearly understood that resort may be had to various other embodiments, modifications, and equivalents thereof, which, after reading the description herein, may suggest themselves to those skilled in the art without departing from the spirit of the present invention and/or the scope of the appended claims.

### EXAMPLES

### Example 1: Recombinant Production of Matin in E. coli.

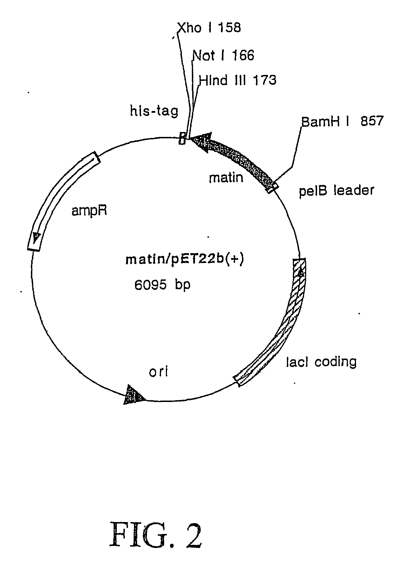

The nucleotide (SEQ ID NO:1) and amino acid (SEQ ID NO:2) sequences for the α1 chain of laminin (GenBank Acc. No. NM_008480) are shown in Fig. 1. The sequence encoding Matin (globular domain 1, or the G1 domain, extending approximately from nucleotide 6442 to nucleotide 7062) was amplified by PCR from the plasmid FBssrAi using the forward primer 5'-CGG-GAT-CCT-AGA-GAC-TGC-ATC-CGC-GCC-TAT-3' (SEQ ID NO:3), and the reverse primer was 5'-CCC-AAG-CTT-TAC-TAT-CTG-CGT-CAC-GGT-GGG-3' (SEQ ID NO:4) (underlined portions of the primer represent laminin sequence). The resulting cDNA fragment was digested with *Bam*HI and *Hin*dIII and ligated into predigested pET22b(+) (Novagen, Madison, Wisconsin, USA). The construct is shown in Fig. 2. The ligation placed Matin in-frame with the pelB leader sequence, allowing for periplasmic localization and expression of soluble protein. The 3' end of the sequence was ligated in-frame with the polyhistidine tag sequence.

Plasmid constructs encoding Matin were first transformed into *E. coli* HMS 174 (Novagen, Madison, Wisconsin, USA) and then transformed into BL21 for expression (Novagen, Madison, Wisconsin, USA). Overnight bacterial culture was used to inoculate a 500 ml culture in LB medium (Fisher Scientific, Pittsburgh, Pennsylvania, USA). This culture was grown for approximately 4 hours until the cells reached an OD₆₀₀ of 0.6. Protein expression was then induced by addition of IPTG to a final concentration of 1 mM. After a 2-hour induction, cells were harvested by centrifugation at 5,000 x g and lysed by resuspension in 6 M guanidine, 0.1 M NaH₂PO₄, 0.01 M Tris-HCl, pH 8.0. Resuspended cells were sonicated briefly, and centrifuged at 12,000 x g for 30 minutes. The supernatant fraction was passed over a 5 ml Ni-NTA agarose column (Qiagen, Hilden, Germany) 4-6 times at a speed of 2 ml per minute. Non-specifically bound protein was removed by washing with both 10 mM and 25 mM imidazole in 8 M urea, 0.1 M NaH₂PO₄, 0.01 M Tris-HCl, pH 8.0. Matin protein was eluted from the column with increasing concentrations of imidazole (50 mM, 125 mM, and 250 mM) in 8 M urea, 0.1 M NaH₂PO₄, 0.01 M Tris-HCl, pH 8.0. The eluted protein was dialyzed twice against PBS at 4°C. A portion of the total protein precipitated during dialysis. Dialyzed protein was collected and centrifuged at approximately 3,500 x g and separated into insoluble (pellet) and soluble (supernatant) fractions.

*E. coli*-expressed Matin was isolated predominantly as a soluble protein and SDS-PAGE analysis revealed a monomeric band at about 30 kDa. The eluted fractions containing this band were used in the following experiments. Protein concentration in each fraction was determined by the BCA assay (Pierce Chemical Co., Rockford, Illinois, USA) and quantitative SDS-PAGE analysis using scanning densitometry.

### Example 2: Matin Inhibits Endothelial Cell Proliferation.

The anti-proliferative effect of Matin on C-PAE cells was examined by the methylene blue staining assay using *E*. *coli* produced soluble protein.
*Cell lines and culture.* PC-3 (human prostate adenocarcinoma cell line) and C-PAE (bovine pulmonary arterial endothelial cell line) cells were obtained from American Type Culture Collection. The C-PAE cell lines were maintained in DMEM (Life Technologies/Gibco BRL, Gaithersburg, Maryland, USA) supplemented with 10% fetal calf serum (FCS), 100 units/ml of penicillin, and 100 mg/ml of streptomycin.
*Proliferation assay.* C-PAE cells were grown to confluence in DMEM with 10% FCS and kept contact-inhibited for 48 hours. C-PAE cells were used between the second and fourth passages. PC-3 cells were used as non-endothelial controls in this experiment. Cells were harvested by trypsinization (Life Technologies/Gibco BRL, Gaithersberg, Maryland, USA) at 37°C for 5 minutes. A suspension of 12,500 cells in DMEM with 0.1% FCS was added to each well of a 24-well plate coated with 10 µg/ml fibronectin. The cells were incubated for 24 hours at 37°C with 5% CO₂ and 95% humidity. Medium was removed and replaced with DMEM containing 20% FCS. Unstimulated control cells were incubated with 0.1% FCS.

Using the methylene-blue staining method, 7000 cells were plated into each well of a 96-well plate, and treated as described above. Cells were then counted using the method of Oliver *et al.* (Oliver, M.H. et al., 1989, J. Cell. Sci. 92:513-518). After 48 hours of treatment, all wells were washed with 100 µl of PBS, and the cells fixed with 10% formalin in neutral-buffered saline (Sigma Chemical Co., St. Louis, Missouri, USA). The cells were then stained with 1% methylene blue (Sigma) in 0.01M borate buffer, pH 8.5. Wells were washed with 0.01M borate buffer, and the methylene blue extracted from the cells with 0.1N HCl/ethanol, and the absorbance measured in a microplate reader (Bio-Rad, Hercules, California, USA) at 655 nm. Polymyxin B (Sigma) at a final concentration of 5 µg/ml was used to inactivate endotoxin (Liu, S. et al., 1997, Clin. Biochem. 30:455-463).

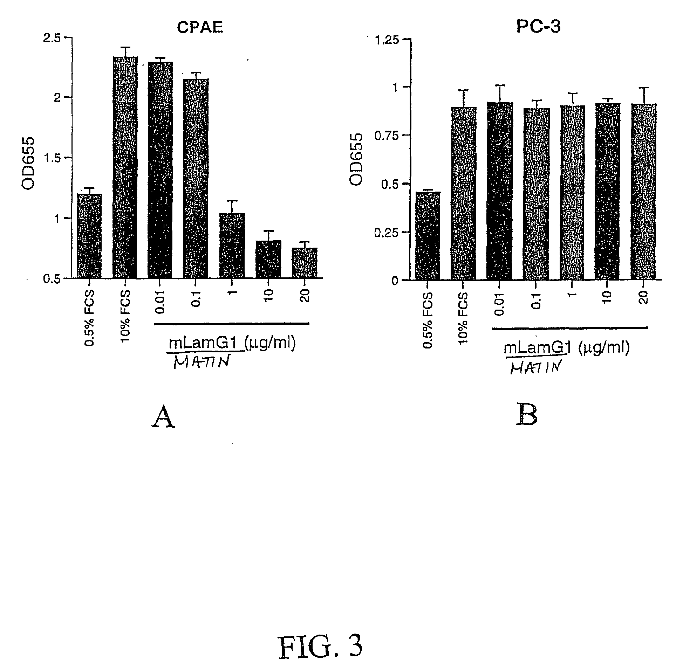

The results are shown in Figs. 3A and 3B, which are a pair of histograms showing the effect of increasing amounts of Matin on the uptake of dye by C-PAE cells relative to PC-3 cells. Absorbance at OD₆₅₅ is shown on the y-axis. "0.5% FCS" represents the 0.5% FCS-treated (unstimulated) control, and "10% FCS" is the 10% FCS-treated (stimulated) control. The remaining bars represent treatments with increasing concentrations of Matin. Matin inhibited FCS-stimulated proliferation of C-PAE cells in a dose-dependent manner. The difference between the mean value of the cell number in the Matin treatment versus the control was significant in the 0.1 - 10.0 µg/ml range, with p<0.05. When PC-3 cells were treated with Matin, no inhibitory effect was observed. In C-PAE cells, dye uptake dropped off to the level seen in unstimulated cells at a Matin treatment level of about 0.1 µg/ml. Each bars represents the mean of the relative absorbance units at 655 nm ± the standard error of the mean for triplicate wells. This endothelial cell specificity indicates that Matin is likely an effective anti-angiogenic agent.

### Example 3: Matin Induces Endothelial Cell Apoptosis.

*Annexin V-FITC assay.* In the early stage of apoptosis, translocation of the membrane phospholipid PS from the inner surface of plasma membrane to outside is observed (van Engeland, M. et al., 1998, Cytometry 31:1-9; Zhang, G. et al., 1997, Biotechniques 23:525-531; Koopman, G. et al. 1994, Blood 84:1415-1420). Externalized PS can be detected by staining with a FITC conjugate of Annexin V that has a naturally high binding affinity to PS (van Engeland, *supra*). Apoptosis of endothelial cells upon treatment with Matin was therefore evaluated using annexin V-FITC labeling.

C-PAE cells (0.5 x 10⁶ per well) were seeded onto a 6-well plate in 10% FCS supplemented DMEM. The next day, fresh medium containing 10% FCS was added with either 80 ng/ml of TNF-α (positive control) or Matin ranging from 0.02 to 20 µg/ml. Control cells received an equal volume of PBS. After 18 hours of treatment, medium containing floating cells was collected, and attached cells were trypsinized and centrifuged together with floating cells at 3,000 x g. The cells were then washed in PBS and resuspended in binding buffer (10 mM HEPES/NaOH, pH 7.4, 140 mM NaCl, 2.5 mM CaCl₂). Annexin V-FITC (Clontech, Palo Alto, California, USA) was added to a final concentration of 150 ng/ml, and the cells were incubated in the dark for 10 minutes. The cells were washed again in PBS and resuspended in binding buffer. Annexin V-FITC labeled cells were counted using a FACStar Plus flow cytometer (Becton-Dickinson, Waltham, Massachusetts, USA). For each treatment, 15,000 cells were counted and stored in listmode. This data was then analyzed using Cell Quest software (Becton-Dickinson, Waltham, Massachusetts, USA).

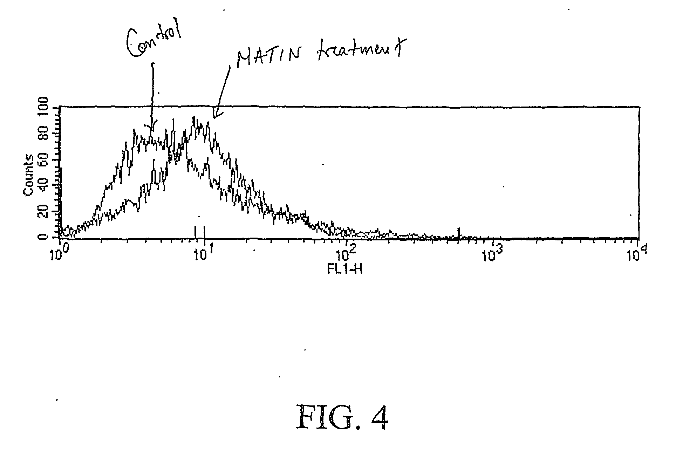

The results are shown in Fig. 4, which is a plot showing annexin fluorescence activity. After 18 hours of treatment with Matin at 20 µg/ml, a distinct shift of peak annexin fluorescence was observed. The shift in fluorescence intensity was similar for Matin at 20 µg/ml and the positive control TNF-α (80 ng/ml). Matin at 2 µg/ml also showed a mild shift in annexin fluorescence intensity, but concentrations below 0.2 µg/ml did not demonstrate any annexin V positivity. This shift of peak intensity was not observed when nonendothelial cells (PC-3) were used.
*Caspase-3 assay.* Caspase-3 (CPP32) is an intracellular protease activated at the early stage of apoptosis, and initiates cellular breakdown by degrading structural and DNA repair proteins (Casciola-Rosen, L. et al., 1996, J. Exp. Med. 183:1957-1964; Salvesen, G.S. et al., 1997, Cell 91:443-446). The protease activity of Caspase-3 was measured spectrophotometrically by detection of the chromophore (p-nitroanilide) cleaved from the labeled substrate (DEVD-pNA).

C-PAE cells or PC-3 cells (0.5 x 10⁶ per well) were plated onto a 6-well plate precoated with fibronectin (10 µg/ml) in DMEM supplemented with 10% FCS, and incubated overnight. The next day, the medium was replaced with DMEM containing 2% FCS and then incubated overnight at 37°C. Then cells were then stimulated with bFGF (3ng/ml) in DMEM supplemented with 2% FCS, and also containing either TNF-α (80 ng/ml, positive control) or Matin (10 µg/ml), and incubated for 24 hours. Controls received PBS buffer. After 24 hours, the supernatant cells were collected, and attached cells were trypsinized and combined with the supernatant cells. Cells were counted and resuspended in cell lysis buffer (Clontech, Palo Alto, California, USA) at a concentration of 4 x 10⁷ cells/ml. The rest of the protocol followed the manufacturer's instructions (Clontech, Palo Alto, California, USA). A specific inhibitor of Caspase-3, DEVD-fmk (Asp-Glu-Val-Asp-fluoromethyl ketone) was used to confirm the specificity of the assay. The absorbance was measured in a microplate reader (Bio-Rad, Hercules, California, USA) at 405 nm.

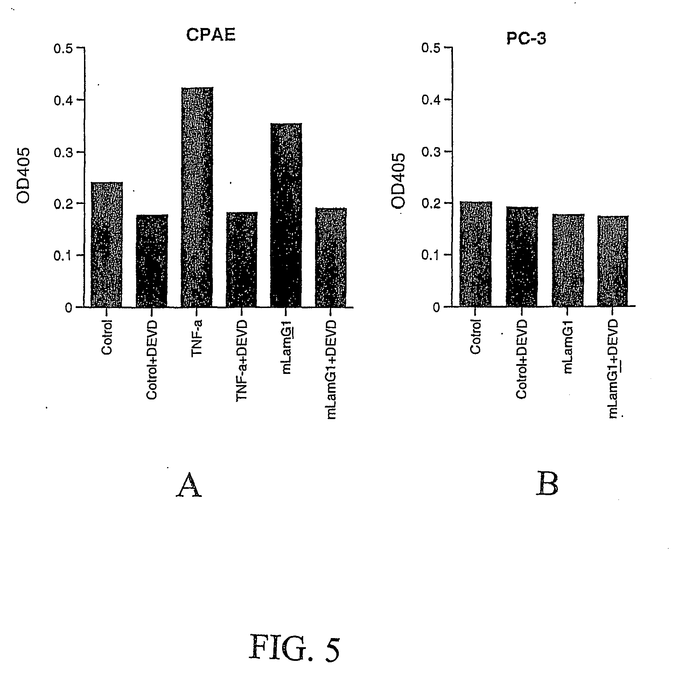

The results are shown in Figs. 5A and 5B, which are a pair of histograms showing the amount of Caspase-3 activity as a function of absorbance at OD₄₀₅ (y-axis) for C-PAE cells (Fig. 5A) and PC-3 cells (Fig. 5B) under various treatments (x-axis).

C-PAE cells treated with20 µg/ml Matin exhibited a 1.6-fold increase in Caspase-3 activity, whereas the positive control TNF-α gave a comparable (1.7-fold) increase compared with control. A specific inhibitor of Caspase-3, DEVD-fmk, decreased the protease activity to baseline indicating that the increase in the measured activity was specific for Caspase-3. In nonendothelial PC-3 cells, there was no difference in Caspase-3 activity between control and Matin-treated cells.
*MTT Assay.* The pro-apoptotic activity of Matin was examined in C-PAE cells. Cell viability was assessed by MTT (3-(4, 5-dimethylthiazol-2-yl)-2, 5-diphenyl tetrasolium bromide) assay (Sugiyama, H. et al., 1998, Kidney Int. 54:1188-1196). This assay is a quantitative colorimetric analysis for cell survival based on the ability of living cells to cleave the tetrasolium ring in active mitochondria. C-PAE cells (7,000 cells per well) were plated to a 96-well plate in 10% FCS containing DMEM. The next day, either TNF-α (positive control, 80 ng/ml), or varying concentrations of Matin were added to the wells and incubated for 24 hours. MTT solution (5 mg/ml; CHEMICON International, Temecula, California, USA) was then added to the wells at a rate of 10 µl/well and incubated at 37°C for 4 hours. Acid-isopropanol was added and mixed thoroughly. The absorbance was measured in a microplate reader (Bio-Rad, Hercules, California, USA) at 590 nm.

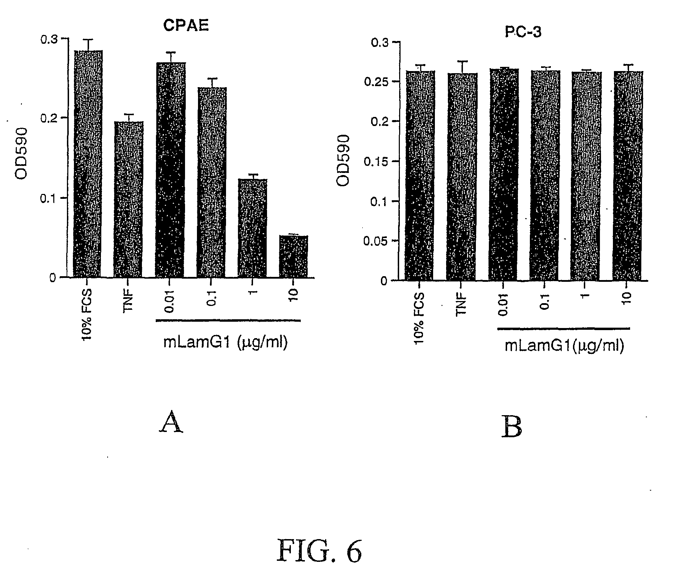

The results are shown in Figs. 6A and 6B, which are a pair of histograms showing cell viability (as a function of OD₅₉₀, y-axis) at increasing concentrations of Matin (x-axis). Each point represents the mean +/- the standard error of the mean for triplicate wells.

Matin decreased cell viability in a dose-dependent manner. At 10 µg/ml, Matin decreased the cell viability by about 80% compared to controls. No inhibitory effect was observed in Matin-treated PC-3 cells.

### Example 4: Matin Inhibits Tumor Growth in vivo.

Five million PC-3 cells were harvested and injected subcutaneously on the back of 7- to 9-week-old male athymic nude mice. The tumors were measured using Vernier calipers and the volume was calculated using the standard formula width² x length x 0.52. The tumors were allowed to grow to about 100 mm³, and animals were then divided into groups of 5 or 6 mice. Matin or nephrin was intraperitoneally injected daily (20 mg/kg) for 10 days in sterile PBS to their respective experimental group. The control group received vehicle injection (either BSA or PBS). Tumor volume was calculated every 2 or 3 days over 10 days. Nephrin is a non-collagen-derived protein which was used as a control. The nephrin was expressed in pET22b, as for Matin.

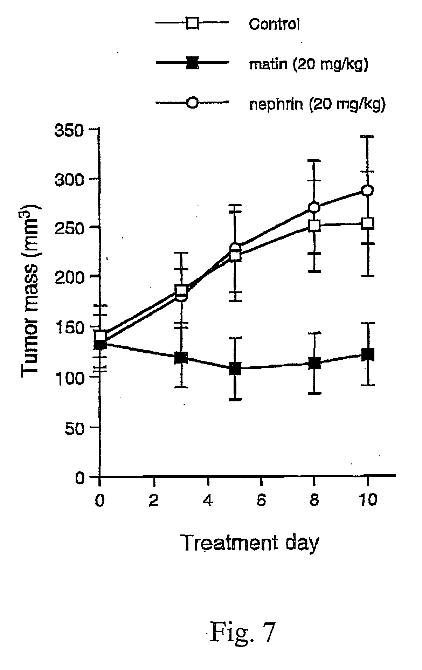

The results are shown in Fig. 7, which is a graph showing tumor size in mm³ (y-axis) against days of treatment (x-axis) for the PBS control (□), 20 mg/kg Matin (■) and 20 mg/kg nephrin (○). Matin, produced in *E. coli,* significantly inhibited the growth of PC-3 human prostate tumors (Fig. 7).

All references, patents, and patent applications are incorporated herein by reference in their entirety. While this invention has been particularly shown and described with references
to preferred embodiments thereof, it will be understood by those of ordinary skill in the art that various changes in form and details may be made therein without departing from the spirit and scope of the invention as defined by the appended claims.

## Claims

1. An antibody that binds specifically to a protein that consists of the amino acids 2132 to 2338 or of amino acids 2132 to 3084 of SEQ ID No:2; or to a fragment thereof comprising at least 25 continuous amino acids of said protein; or to a variant thereof having at least 70% sequence identity with said protein.

2. An antibody according to Claim 1 wherein said variant has at least 80% or at least 90% sequence identity with said protein.

3. An antibody that binds specifically to an isolated protein of the G1 domain of the α chain of laminin, or to a fragment analog, derivative or mutant thereof;
wherein said protein, fragment analog, derivative, or mutant has anti-angiogenic activity.

4. An antibody according to claim 3; wherein the G1 domain is the G1 domain of the a 1 chain of mouse laminin.

5. An antibody that specifically binds to:
a) the isolated protein of SEQ ID No: 2, or a fragment analog, derivative or mutant thereof that has anti-angiogenic activity;
b) a protein or peptide that has 80% or greater sequence identity with SEQ ID No: 2 and has anti-angiogenic activity; or
c) a protein or peptide that has 90% or greater sequence identity with SEQ ID No: 2 and has anti-angiogenic activity.

6. An antibody that binds specifically to a monomer or multimer of a protein, fragment, analog, derivative or mutant as described in any of claims 1 to 5

7. An antibody that binds specifically to a chimeric protein that comprises a protein, fragment, analog, derivative or mutant thereof as described in any of claims 1 to 6 (e.g. wherein the chimeric protein further comprises at least one protein molecule selected from the group consisting of: vascostatin or fragments thereof, arrestin or fragments thereof, canstatin or fragments thereof, tumstatin or fragments thereof, endostatin or fragments thereof, angiostatin or fragments thereof).

8. An antibody according to any of claims 1 to 7, for use in therapy, prognosis or diagnosis.

9. The use of an antibody according to any of claims 1 to 7 in the preparation of a medicament for up-regulating angiogenesis.

10. The use of an antibody according to any of claims 1 to 7 in the preparation of a medicament for wound healing or tissue repair (e.g. for treating post-infarct heart tissue).

11. The use of an antibody according to any of claims 1 to 7 in the preparation of a medicament for treating a developmental or reproductive disorder.

12. The use of an antibody according to any of claims 1 to 7 in the preparation of a diagnostic or prognostic kit or composition (e.g. for the diagnosis or prognosis of cancer).

13. A method comprising using an antibody according to any of claims 1 to 7 to detect or quantify an anti-angiogenic protein in a body fluid or tissue.

14. An antiserum directed to the Fab region of an antibody according to any of claims 1 to 7.

15. The use of an antiserum according to claim 14 in the preparation of a medicament for treating cancer.

16. A therapeutic, diagnostic or prognostic composition comprising an antibody according to any of claims 1 to 7 or an antiserum according to claim 14.
